# EUROPÄISCHE PATENTSCHRIFT

(11) **EP 0 661 291 B1**
(45) Veröffentlichungstag und Bekanntmachung des Hinweises auf die Patenterteilung: **07.03.2001**
(21) Anmeldenummer: 94117265.2
(22) Anmeldetag: 02.11.1994
(51) Int. Cl.: C07H 19/04, C07H 19/10, C07H 19/20, C07H 21/00

(54) **Festphasensynthese von Oligoribonucleotiden**
Solid phase synthesis of oligoribonucleotide
Synthèse des oligoribonucléotides sur Phase solide

(30) Priorität: 17.12.1993 DE 4343126
(43) Veröffentlichungstag der Anmeldung: 05.07.1995
(73) Patentinhaber: Aventis Pharma Deutschland GmbH, 65929 Frankfurt am Main (DE)
(72) Erfinder: Pfleiderer, Wolfgang, Prof., D-78464 Konstanz (DE); Schnell, Ralf, Dr., D-47798 Krefeld (DE); Matysiak, Stephan, Dl., D-78315 Radolfzell (DE)

(56) Entgegenhaltungen:
- TETRAHEDRON LETTERS., Bd.30, Nr.46, 1989, OXFORD GB Seiten 6361 - 6364 S.YAMAKAGE ET AL. 'The 1-(2-chloroethoxy)ethyl Group for the Protection of the 2'-Hydroxyl Group in the Synthesis of Oligoribonucleotides.'
- TETRAHEDRON, (INCL. TETRAHEDRON REPORTS), Bd.47, Nr.41, 1991, OXFORD GB Seiten 8717 - 8728 O.SAKTSUME ET AL. 'Solid Phase Synthesis of Oligoribonucleotides by the Phosphoramidite Approach using 2'-O-1-(2-chloroethoxy)ethyl Protection.'
- CHEMICAL ABSTRACTS, vol. 116, no. 5, 1992, Columbus, Ohio, US; abstract no. 41988k, H.TAKAKU ET AL. 'Preparation of Ribonucleoside Phosphoramidites for Solid-Phase Synthesis of Oligonucleotides.' Seite 840 ;Spalte 1 ; & JP-A-0 307 398 (YUKI GOSEI KOGYO CO., LTD....)
- NUCLEIC ACIDS RESEARCH., Bd.22, Nr.1, 1994, ARLINGTON, VIRGINIA US Seiten 94 - 99 E.ROZNERS ET AL. 'Evaluation of 2'-Hydroxyl Protection in RNA Synthesis using the H-Phosphonate Approach.'

## Beschreibung

Die chemische Polykondensation von Mononucleotiden ist eine wichtige Methode zur Herstellung von Desoxyribonucleinsäure (DNA) oder Ribonucleinsäure (RNA).

Ein grundlegendes Problem bei der chemischen Synthese von DNA oder RNA ist das Auffinden geeigneter Schutzgruppen der Amino- und Hydroxygruppen der Nucleosidbasen und der Zuckerreste. Diese Schutzgruppen müssen einerseits unter den Bedingungen der Polykondensationsreaktion, d.h. während der Reaktion, stabil sein und andererseits müssen sie genügend labil sein, um am Ende der Reaktion wieder entfernt werden zu können, ohne die Phosphodiester-Bindung wieder zu spalten [H. G. Khorana; Pure Appl. Chem. 17 (1968) 349].

Besonders problematisch ist die chemische Synthese von RNA, da der Ribose-Zuckerrest zwei Hydroxygruppen trägt, die beide geschützt werden müssen. Die Schutzgruppe der 5 '-Hydroxygruppe muß dabei vor jedem Polykondensationsschritt selektiv, d.h. ohne Abspaltung der 2'-Hydroxyschutzgruppe wieder abgespalten werden. Die Schutzgruppe der 2'-Hydroxygruppe hingegen darf erst am Ende der RNA-Synthese abgespalten werden, und zwar unter Bedingungen, die zu keiner Spaltung oder Isomerisierung der Phosphordiesterbindung führen [C.B. Reese, Nucleic Acids and Molecular Biology, Vol. 3, F. Eckstein & D. M. Lilley eds., Springer-Verlag Berlin-Heidelberg 1989, S.164-181].

Die effektive Oligoribonucleotidsynthese an festen Trägermaterialien ist daher in ihrer Effektivität noch weit entfernt von der gut funktionierenden Phosphoramidit-Methode zum Aufbau von Oligodesoxyribonucleotid-Ketten. Eine Übersicht geben die Arbeiten von C.B. Reese [Nucleic Acids and Molecular Biology, Vol. 3, F. Eckstein & D. M. Lilley eds., Springer-Verlag Berlin-Heidelberg 1989, S.164-181], S. L. Beaucage, R. P. Iyer [Tetrahedron 48 (1992) 2223-2311], E. Ohtsuka, S. Iwai [in Synthesis and Application of DNA and RNA, S. A. Narang, Ed., Academic Press, London 1987, S. 115-136] und O. Sakatsume et al. [Tetrahedron 47 (1991) 8717-8728].

In der Praxis finden vor allem die an den Aglykonen acylgeschützten 5'-O-Dimethoxytrityl-2'-O-tert.-butyldimethylsilyl-ribo-3'-O-(β-cyanoethyl, N-diisopropyl)phosphoramidite, die auf Arbeiten von N. Usman et al. [J.Am. Chem. Soc. 109 (1987) 7845], K.K. Ogilvie et al. [Proc. Natl. Acad. Sci. USA 85 (1988) 5764] zurückgehen, Verwendung. Diese sind auch käuflich erhältlich. Die damit durchgeführten Oligoribonucleotidsynthesen sind jedoch keineswegs zufriedenstellend, so daß nach neuen Lösungen gesucht werden muß [M. H. et al.: J. Org. Chem. 56 (1991) 4608, D. Gasparutto et al.: Nucleosides & Nucleotides 9 (1990) 1087, T. Wu et al.: J. Org. Chem. 55 (1990) 4717].

Eine ganze Reihe anderer Schutzgruppenkombinationen für die 2'- und 5'-Hydroxyfunktionen des Ribose-Zuckerrestes, die in den oben genannten Literaturstellen aufgeführt sind, brachte ebenfalls nicht den gewünschten Erfolg.

Neben der 2'-O-tert.-Butyldimethylsilyl-Schutzgruppe fanden bestimmte Acetalgruppierungen als Schutzgruppe für die 2'-Position Verwendung. Systematische Untersuchungen von Reese, zusammengefaßt z.B. in Beaucage, Tetrahedron 48 (1992) 223, haben kürzlich ergeben, daß sich Acetalgruppierungen wie der 1-(2-Chlorphenyl)-4-methoxypiperidin-4-yl (Cpmp) oder 1-(2-Fluorphenyl)-4-methoxypiperidin-4-yl (Fpmp) Rest an der 2'-OH Gruppe mit dem 5'-O-Dimethoxytrityl- und dem 5'-Pixylrest (9-(9-Phenyl)xanthenyl-ether) kombinieren lassen, da letztere Funktionen durch schwache Säuren schon vollständig abgespalten sind, bevor das Acetal überhaupt angegriffen wird. Auch diese an den Aglykonen acylgeschützten 5'-O-Dimethoxytrityl-2'-O-Fpmp-ribo-3'-O-(β-cyanoethyl, N-diisopropyl)phosphoramidite sind käuflich erhältlich.

Einer der großen Vorteile acetalischer Schutzgruppen gegenüber den Silylschutzgruppen ist, daß sie selektiv, ohne Nebenreaktion in die 2'-Position eingeführt werden können. Ein weiterer Vorteil ist die effizientere Kupplung der Acetale, die mit steigender Raumerfüllung der 2'-Schutzgruppe sinkt. So ist z.B. die Kupplung 2'-O-Tetrahydropyranyl (2'-O-Thp) geschützter Ribonucleotidmonomerer effizienter als die mit der 2'-OTBDMS-Schutzgruppe, die sterisch besonders anspruchsvoll ist.

Die Arbeit von O. Sakatsume et al. [Tetrahedron 47 (1991) 8717-8728] mit der Einführung der 2'-O-1-(2-Chlorethoxy)ethyl-Schutzgruppe (Cee) zeigt jedoch, daß eine weitere Feinabstimmung acetalischer Schutzgruppen mit den 5'-O-Dimethoxytrityl- und dem 5'-Pixylrest angeraten ist.

Überraschenderweise gelang es uns, Schutzgruppen für die 2'-Hydroxygruppe zu finden, die den bisher beschriebenen in Bezug auf die RNA-Synthese überlegen sind. Kriterien sind dabei vor allem die einfache und selektive Herstellung der Monomerbausteine, die Effizienz der Kupplung der Monomeren. Die Schutzgruppe sollte im Vergleich z.B. mit Ctmp gegenüber 1.5 % Dichloressigsäure, welches zur Entschützung der 5'-OH Gruppe während des Kupplungszyklus angewandt wird, etwas stabiler sein und andererseits noch genügend säurelabil, um am Ende der Synthese noch rasch abspaltbar zu sein.

Oligonucleotidsynthesen mit diesen neuartigen 2'-acetalgeschützten Oligoribonucleotid- Phosphitamiditen sind sehr erfolgreich verlaufen. Die Tatsache, daß bei Verwendung β-eliminierbarer Schutzgruppen, diese noch am festen Träger abgespalten werden können, gewährleistet eine einfache Reinigung und Isolierung des 2'-acetalgeschützten Oligoribonucleotids. Diese Verbindungen können auch als stabile Form des Oligoribonucleotids aufgefaßt werden, die, im Gegensatz zu ungeschützten Oligoribonucleotiden, unbedenklich längere Zeit aufbewahrt werden kann, um dann zu einem späteren Zeitpunkt bei Bedarf durch Säurebehandlung in das freie Oligomere überführt zu werden.

Oligonucleotide, die ein oder mehrere Ribonucleotideinheiten in ihrer geschützten Form enthalten, insbesondere solche, die diese am 3' und/oder am 5 '-Ende enthalten, zeigten ein stark verbessertes Resistenzverhalten gegenüber Nucleasen, wobei sich die Hybridisierungseigenschaften nur geringfügig änderten. Solche Oligonucleotide sind daher geeignet, als Antisense Oligonucleotide oder Ribozyme [A. Peyman, E. Uhlmann, Chem. Rev. 90 (1990) 543] Verwendung zu finden.

Gegenstand der Erfindung ist daher ein Verfahren zur Herstellung einer Verbindung der Formel I 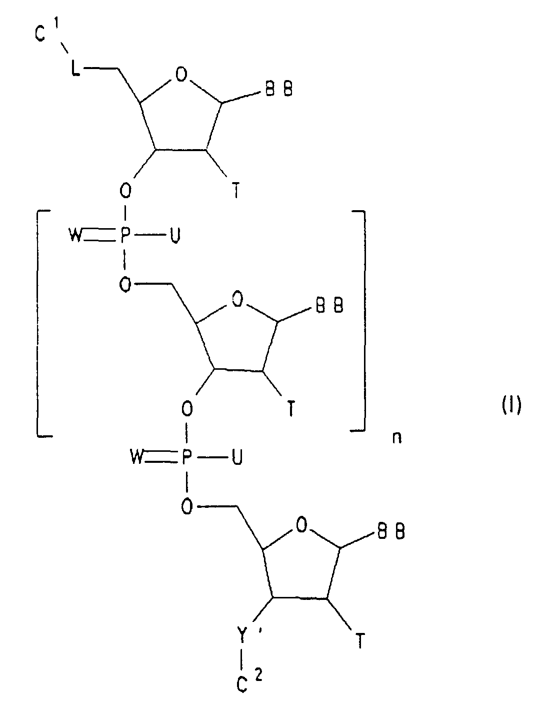 worin
- n: eine Zahl von 1-150, vorzugsweise 1-50, besonders bevorzugt 8-30, ganz besonders bevorzugt 8-20, ist;
- L: Oxy, Sulfandiyl oder Imino, bevorzugt Oxy, bedeutet;
- BB: unabhängig voneinander 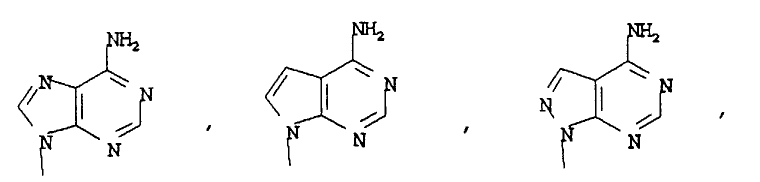 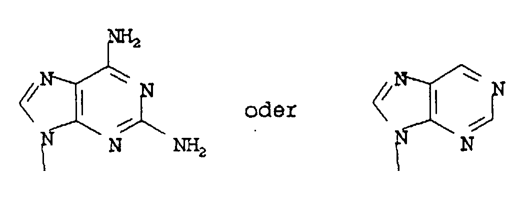 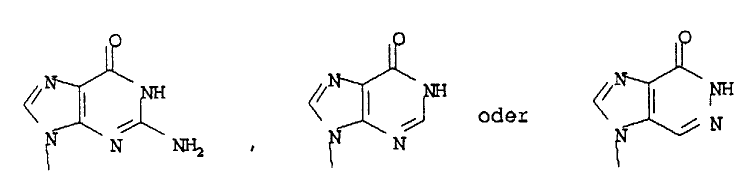 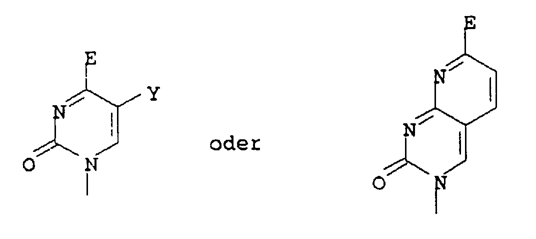 ist, und
- E: für OH oder NH₂ und
- Y: für Wasserstoff, C₁-C₄-Alkyl, Fluor, Chlor, Brom oder C₂-C₆-Alkenyl, C₂-C₆-Alkinyl, bevorzugt für Wasserstoff, CH₃ oder 1-Propinyl, besonders bevorzugt für Wasserstoff, stehen; oder
- BB: kann neben den natürlichen Nucleosid-Basen auch für andere modifizierte Nucleosid-Basen, wie 5-(Hydroxymethyl)uridin, 5-Aminouridin, Pseudouridin, Dihydrouridin, Inosin, 8-Aza-7-deazaadenosin, Tubercidin, Nebularin, Xanthosin, 2-Aminoadenosin-, Etheno-Adenosin, 7-DeazaGuanosin, O4-Methylthymidin, N6-Methyladenosin, O6-Methylguanosin oder Pyridopyrimidin-Nucleoside stehen;
- W: unabhängig voneinander Sauerstoff oder Schwefel, bevorzugt Sauerstoff, bedeutet;
- T: unabhängig voneinander für Wasserstoff, -OCH₃, -O-CH₂CH₃, -O-CH₂-CH = CH₂ oder OH, mindestens jedoch einmal für OH steht;
- Y': für Oxy, Sulfandiyl, Imino, (CH₂)ₖ oder N(CH₂)ₖ steht, wobei k eine ganze Zahl von 1 bis 18, vorzugsweise 1 bis 6, bedeutet, bevorzugt steht Y' für Oxy;
- U: für Hydroxy, Mercapto, SeH, C₁-C₁₈-Alkoxy, C₁-C₁₈-Alkyl, C₆-C₂₀-Aryl, C₆-C₁₄-Aryl-C₁-C₈-alkyl, NHR¹⁷, NR¹⁷R¹⁸ oder einen Rest der Formel (OCH₂CH₂)_{c}O(CH₂)_{c'}CH₂R²⁰ steht, bevorzugt steht U für Hydroxy, Mercapto, C₁-C₆-Alkoxy, C₁-C₆-Alkyl, NHR¹⁷, NR¹⁷R¹⁸, besonders bevorzugt für Hydroxy oder C₁-C₆-Alkyl; wobei
R¹⁷ C₁-C₁₈-Alkyl, C₆-C₂₀-Aryl, C₆-C₁₄-Aryl-C₁-C₈-alkyl, -(CH₂)_{d}-[NH(CH₂₋)_{d}]_{d'}-NR¹⁹R¹⁹; bevorzugt C₁-C₈-Alkyl oder Methoxyethyl, besonders bevorzugt C₁-C₄-Alkyl oder Methoxyethyl, worin
d eine ganze Zahl von 2 bis 6 und
d' eine ganze Zahl von 0 bis 6 sind, und
R¹⁸ C₁-C₁₈-Alkyl, C₆-C₂₀-Aryl, C₆-C₁₄-Aryl-C₁-C₈-alkyl steht oder im Falle von NR¹⁷R¹⁸ zusammen mit R¹⁷ und dem sie tragenden Stickstoffatom einen 5-6-gliedrigen heterocyclischen Ring bedeutet, der zusätzlich ein weiteres Heteroatom aus der Reihe O, S, N enthalten kann, wie z.B. der Morpholinyl- und der Imidazolidinyl-Ring;
R¹⁹ unabhängig voneinander Wasserstoff, oder C₁-C₆-Alkyl oder C₁-C₄-Alkoxy-C₁-C₆-alkyl ist;
c eine ganze Zahl von 1 bis 100, bevorzugt 3 bis 20 und besonders bevorzugt 3 bis 8;
c' eine ganze Zahl von 0 bis 18, bevorzugt 0 bis 15;
R²⁰ Wasserstoff oder eine funktionelle Gruppe wie Hydroxy, Amino, NHR¹⁷, COOH, CONH₂, COOR²¹, oder Fluor, Chlor oder Brom, bedeutet, mit R²¹ C₁-C₄-Alkyl, vorzugsweise Methyl, sind;
- C¹ und C²: gleich oder verschieden sind und für Wasserstoff, C₁-C₁₈-Alkyl, C₂-C₁₈-Alkenyl, C₂-C₁₈-Alkinyl, C₁-C₁₈-Alkylcarbonyl, C₂-C₁₈-Alkenylcarbonyl, C₂-C₁₈-Alkinylcarbonyl, C₆-C₂₀-Aryl, C₆-C₁₄-Aryl-C₁-C₈-alkyl, einen Rest der Formel II stehen, 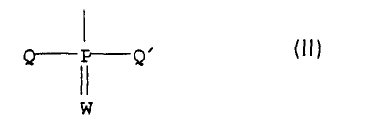 wobei
- W: wie oben definiert ist;
- Q und Q': unabhängig voneinander für Hydroxy, Mercapto, SeH, C₁-C₂₂-Alkoxy, -O-(CH₂)_{b}-NR¹⁵R¹⁶ stehen, mit
b 1 bis 6, und
R¹⁵ und R¹⁶ unabhängig voneinander Wasserstoff, C₁-C₁₈-Alkyl, vorzugsweise C₁-C₈-Alkyl, C₆-C₂₀-Aryl, C₆-C₁₄-Aryl-C₁-C₈-alkyl, C₆-C₁₄-Aryl-C₁-C₈-alkoxy, wobei Aryl auch Heteroaryl bedeutet und Aryl gegebenenfalls durch 1, 2, oder 3 gleiche oder verschiedene Reste aus der Reihe Carboxy, Amino, Nitro, C₁-C₄-Alkylamino, C₁-C₆-Alkoxy, Hydroxy, Fluor, Chlor, Brom und Cyano substituiert ist, C₁-C₁₈-Alkylmercapto, NHR¹⁷, NR¹⁷R¹⁸, wobei R¹⁷ und R¹⁸ wie oben definiert sind, oder zusammen mit dem sie tragenden Stickstoffatom einen 3-6 gliedrigen Ring bilden, oder
Q bzw. Q' steht bevorzugt für Hydroxy, Mercapto, OCH₂CH₃, O-i-C₃H₇, O-n-C₆H₁₃, O-n-C₁₈H₃₇, O-(CH₂)₃-(3-Pyridyl), O-(CH₂)₂-(4-Nitrophenyl), Farnesyl, Phytyl, Vitamin A, Vitamin E, Testosteron, Cholesterol, CH₃, O- (CH₂)₄-(9-Acridin); oder
- Q und Q': stehen für einen Rest der Formel III 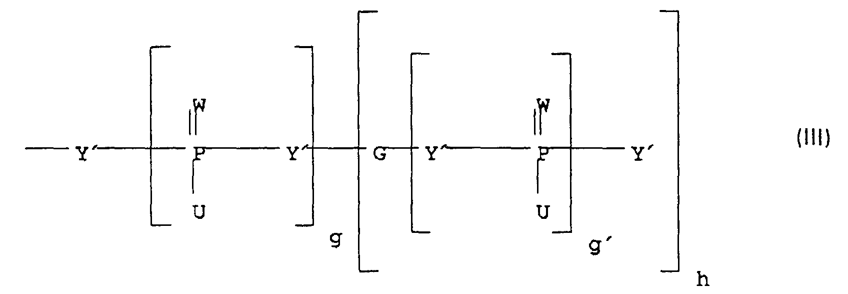 mit
g und g' = 0 oder 1,
h = 0 bis 10,
G C₂-C₁₂-Alkylen, insbesondere C₂-C₄-Alkylen, C₆-C₁₄-Aryl-di-C₁-C₈-alkylen, C₆-C₁₈-Arylen, gegebenenfalls durch Fluor, Chlor, Brom, Amino, Hydroxy, C₁-C₁₈-Alkyl, C₁-C₁₈-Alkoxy, C₁-C₁₈-Alkoxycarbonyl, C₆-C₁₄-Aryl, C₆-C₁₄-Aryl-C₁-C₁₈-alkyl, oder C₆-C₁₄-Aryl-C₁-C₈-alkoxy ein- bis dreifach substituiert sein kann, einer Gruppe der Formel (CH₂CH₂N')ᵢCH₂CH₂ oder (CH₂N')ᵢCH₂, worin i eine ganze Zahl von 1 bis 11, vorzugsweise 1 bis 5, ist und N' für Oxy, Sulfandiyl, Imino oder Methylen steht und
W, U und Y' sind wie oben definiert; oder
- Q und Q': bedeuten eine Gruppe, die die intrazelluläre Aufnahme begünstigt oder als Markierung einer DNA-Sonde dient oder bei der Hybridisierung des Oligonucleotidanalogons an die Target-Nucleinsäure diese unter Quervernetzung oder Spaltung angreift, wobei in Formel I die Nucleinsäuresequenz auch ein- oder mehrfach durch Linker der Formel III unterbrochen sein kann und Konjugate nach bekannten Verfahren auch über die Nucleinbasen oder über das Phosphodiester- oder Phosphothiodiester-Backbone gebildet werden können;
- C¹ und C²: stehen besonders bevorzugt für Wasserstoff und einen Rest der Formel II mit W = Oxy und Q = Q' = Hydroxy;
dadurch gekennzeichnet, daß man
a) eine Verbindung der Formel IV 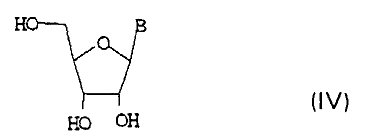 worin
   - B: steht für 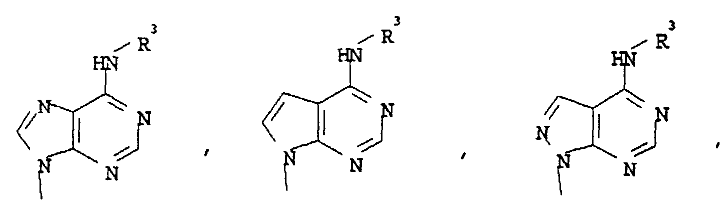 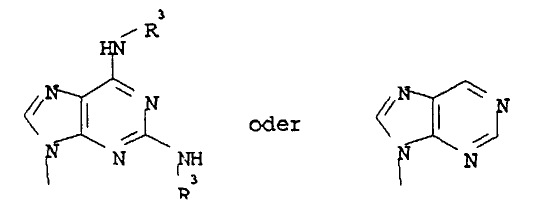 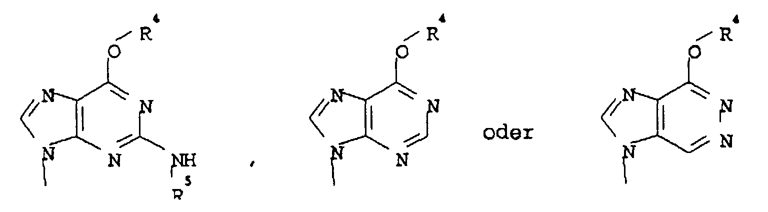 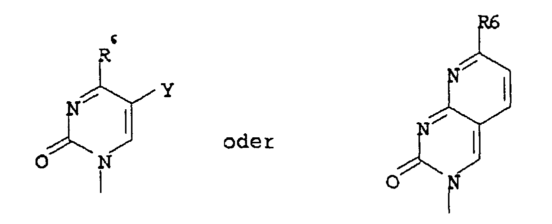 worin
   - R³: jeweils unabhängig voneinander eine Gruppe der Formel 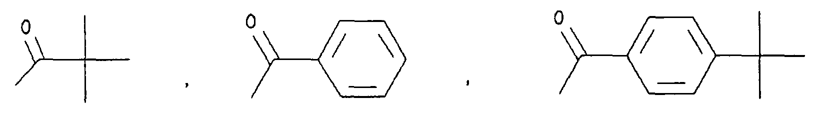 oder 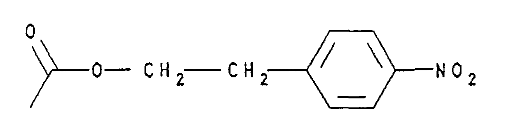 bedeutet;
   - R⁴: für Wasserstoff oder 2-(p-Nitrophenyl)ethyl steht;
   - R⁵: 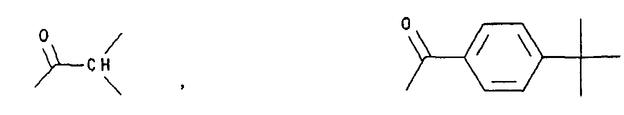 oder 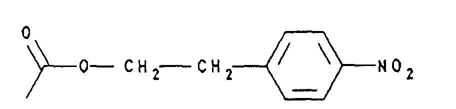
   - R⁶: OH, NH₂, 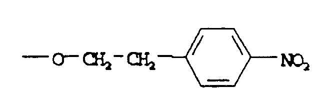 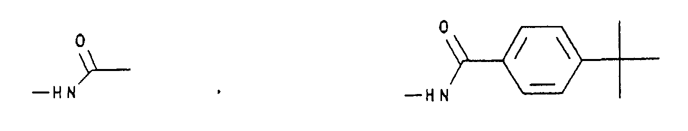 oder 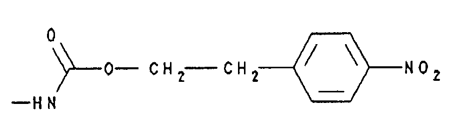 bedeuten;
   - Y: für Wasserstoff, C₁-C₄-Alkyl, Fluor, Chlor, Brom oder C₂-C₆-Alkenyl, C₂-C₆-Alkinyl, bevorzugt für Wasserstoff, CH₃ oder 1-Propinyl, besonders bevorzugt für Wasserstoff steht; oder
   - B: kann neben den natürlichen Nucleosid-Basen auch für andere modifizierte Nucleosid-Basen, deren Amino- bzw. Hydroxygruppen durch geeignete, bekannte Schutzgruppen, wie die para-Nitrophenylethyloxycarbonyl-Gruppe, die Benzoyl-Gruppe und die para-(t-Butyl)benzoylgruppe für die Hydroxygruppe und die Benzoyl-, para-(t-Butyl)benzoyl-, para-Nitrophenylethyloxycarbonyl-, Isobutyryl-, para(tert-Butyl)phenylacetyl-Gruppe für die Aminogruppe, geschützt werden stehen, wobei nachfolgend genannte Nucleoside mit modifizierten Basen (BB) käuflich sind und die Einführung der einzelnen Schutzgruppen beispielsweise nach C. Lehmann et al., Nucl. Acids Res. 17 (1989) 2379; C. B. Reese "The Chemical Synthesis of Oligo and Polyribonucleotides" in Nucleic Acids and Molecular Biology, Vol. 3, F. Eckstein & D. M. Lilley eds., Springer-Verlag Berlin-Heidelberg 1989, S.164-181; E. Sonveaux, Bioorganic Chemistry 14 (1986) 274; A. Peyman, E. Uhlmann, Chem. Rev. 90 (1990) 543; S. L. Beaucage, R. P. Iyer, Tetrahedron 49 (1993) 1925, 2223, 6123, erfolgen kann: 5-(Hydroxymethyl)uridin, 5-Aminouridin, Pseudouridin, Dihydrouridin, Inosin, 8-Aza-7-deazaadenosin, Tubercidin, Nebularin, Xanthosin, 2-Aminoadenosin-, Etheno-Adenosin, 7-Deaza-Guanosin, 04-Methylthymidin, N6-Methyladenosin, 06-Methylguanosin oder Pyridopyrimidin-Nucleoside,
   in 3'und 5'-Position nach bekannten Verfahren schützt, vorzugsweise durch Umsetzung mit 1,3-Dichlor-1,1,3,3-Tetraisopropyldisiloxan nach bekannten Verfahren [z.B. in Analogie zu Markiewicz, J. Chem. Res., Synop. 1979, 24; Nucleic Acids Res. Symp. Ser. 7 (1980) 115], beispielsweise in Pyridin bei 0°C, zu den 3',5'-O-(Tetraisopropyldisiloxan-1,3-diyl) geschützten Verbindungen;
b) anschließend die geschützte Verbindung mit dem Vinylether der Formel V umsetzt, 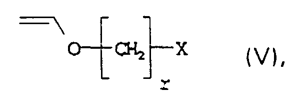 worin
   - r: 1 oder 2 bedeutet und
   - X: für C₆-C₁₂-Aryl steht, wobei Aryl ein- oder mehrfach, bevorzugt ein- bis dreifach, besonders bevorzugt ein- bis zweifach mit Hydroxy, Mercapto, Nitro, Fluor, Chlor, Brom, C₁-C₆-Alkyl, C₁-C₆-Alkoxy, C₁-C₆-Alkylmercapto, C₂-C₆-Alkenyl, C₃-C₆-Alkinyl, C(O)OH, C(O)NH₂, C(O)O-C₁-C₁₈-Alkyl, C(O)O-C₆-C₁₂-Aryl, C(O)-C₁-C₁₈-Alkyl, C(O)-C₆-C₁₂-Aryl, O-C(O)NH₂, O-C(O)O-C₁-C₁₈-Alkyl, O-C(O)O-C₆-C₁₂-Aryl, O-C(O)-C₁-C₁₈-Alkyl, O-C(O)-C₆-C₁₂-Aryl, O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch Amino, Hydroxy, Nitro, C₁-C₆-Alkyl, C₁-C₆-Alkoxy, C₁-C₆-Alkoxycarbonyl, Fluor, Chlor oder Brom substituiert sein kann;
   für r = 1 steht X weiterhin für Phenyl oder C₁-C₄-Alkoxy-phenyl;
   für r = 2 steht X weiterhin für CN, S-Phenyl, SO₂-Phenyl, N-Phthalimid oder NO₂;
   für r = 1 steht X bevorzugt für C₆-C₁₂-Aryl, wobei Aryl ein- bis zweifach, bevorzugt einfach mit Fluor, Chlor, Brom, C₁-C₄-Alkyl oder C₁-C₄-Alkoxy, und gegebenenfalls einfach mit O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch C₁-C₄-Alkyl, C₁-C₄-Alkoxy, Fluor, Chlor oder Brom substituiert sein kann;
   für r = 2 steht X bevorzugt für 4-Nitrophenyl;
   für r = 1 steht X besonders bevorzugt für 2,5-Dichlor-4-pivaloyloxyphenyl, 3-Chlor-4-[2-[4-nitrophenyl)ethoxycarbonyloxy]-phenyl, 2-Chlor-4-[2-[4-nitrophenyl)ethoxycarbonyloxy]-phenyl, 3-Fluor-4-[2-[4-nitrophenyl)ethoxycarbonyloxy]-phenyl, besonders bevorzugt für 3-Fluor-4-[2-[4-nitrophenyl)ethoxycarbonyloxy]-phenyl;
   in Analogie zu bekannten Verfahren umsetzt [z.B. O. Sakatsume et al.; Tetrahedron 47 (1991) 8717-8728 und dort zitierte Literatur], d.h. beispielhaft wird die 3',5'-geschützte Verbindung in einem geeigneten organischen Lösungsmittel, z.B. Toluol mit 1-20, bevorzugt 1-10, besonders bevorzugt 1-5 Equivalenten des Vinylethers der Formel V, gegebenenfalls nach Zusatz eines Katalysators, bevorzugt einer Säure, umgesetzt, und die anschließende Aufarbeitung, gegebenenfalls nach Neutralisation, ebenfalls nach bekannten Verfahren wie beispielsweise Extraktion, Kristallisation, Chromatographie durchführt;
c) die 5'- und 3'-Schutzgruppen, ebenfalls in Analogie zu bekannten Verfahren wieder abspaltet, wobei im Fall der 3'-5'-(O-Tetraisopropyldisiloxan-1,3-diyl) geschützten Verbindungen die Abspaltung der Schutzgruppe bespielsweise mit HNEt₃⁺F⁻ erfolgt;
d) die 5'-Schutzgruppe mit R² = Dimethoxytrityl, Monomethoxytrityl, Pixyl, Trityl, bevorzugt Dimethoxytrityl, nach bekannten Verfahren [z.B. M.J. Gait, Oligonucleotide Synthesis-a practical approach, IRL Press, 1984] einführt, beispielhaft die Dimethoxytritylgruppe durch Umsetzung mit Dimethoxytritylchlorid in Pyridin, wobei man eine Verbindung der Formel VI erhält 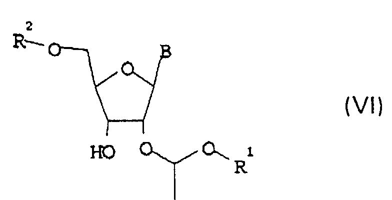 worin
   - R¹: für -[CH₂]ᵣ-X steht, mit
   - r: = 1 oder 2 und
   - X: für C₆-C₁₂-Aryl steht, wobei Aryl ein- oder mehrfach, bevorzugt ein- bis dreifach, besonders bevorzugt ein- bis zweifach mit Hydroxy, Mercapto, Nitro, Fluor, Chlor, Brom, C₁-C₆-Alkyl, C₁-C₆-Alkoxy, C₁-C₆-Alkylmercapto, C₂-C₆-Alkenyl, C₃-C₆-Alkinyl, C(O)OH, C(O)NH₂, C(O)O-C₁-C₁₈-Alkyl, C(O)O-C₆-C₁₂-Aryl, C(O)-C₁-C₁₈-Alkyl, C(O)-C₆-C₁₂-Aryl, O-C(O)NH₂, O-C(O)O-C₁-C₁₈-Alkyl, O-C(O)O-C₆-C₁₂-Aryl, O-C(O)-C₁-C₁₈-Alkyl, O-C(O)-C₆-C₁₂-Aryl, O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch Amino, Hydroxy, Nitro, C₁-C₆-Alkyl, C₁-C₆-Alkoxy, C₁-C₆-Alkoxycarbonyl, Fluor, Chlor oder Brom substituiert sein kann;
   für r = 1 steht X weiterhin für Phenyl oder C₁-C₄-Alkoxy-phenyl;
   für r = 2 steht X weiterhin für CN, S-Phenyl, SO₂-Phenyl, N-Phthalimid oder NO₂;
   für r = 1 steht X bevorzugt für C₆-C₁₂-Aryl, wobei Aryl ein- bis zweifach, bevorzugt einfach mit Fluor, Chlor, Brom, C₁-C₄-Alkyl oder C₁-C₄-Alkoxy, und gegebenenfalls einfach mit O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch C₁-C₄-Alkyl, C₁-C₄-Alkoxy, Fluor, Chlor oder Brom substituiert sein kann;
   für r = 2 steht X bevorzugt für 4-Nitrophenyl;
   für r = 1 steht X besonders bevorzugt für 2,5-Dichlor-4-pivaloyloxyphenyl, 3-Chlor-4-[2-[4-nitrophenyl)ethoxycarbonyloxy]-phenyl, 2-Chlor-4-[2-[4-nitrophenyl)ethoxycarbonyloxy]-phenyl, 3-Fluor-4-[2-[4-nitrophenyl)ethoxycarbonyloxy]-phenyl, besonders bevorzugt für 3-Fluor-4-[2-[4-nitrophenyl)ethoxycarbonyloxy]-phenyl;
   - R²: für Dimethoxytrityl, Monomethoxytrityl, Pixyl, Trityl, bevorzugt für Dimethoxytrityl, steht; und
   - B: wie oben definiert ist;
e) die Verbindung der Formel VI mit einer Verbindung der Formel VII umsetzt 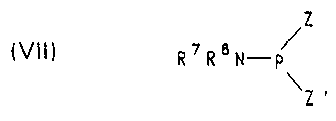 worin
   - Z': für OR⁹ oder C₁-C₁₈-Alkyl, C₁-C₁₈-Alkoxy, C₆-C₂₀-Aryl, C₆-C₁₄-Aryl-C₁-C₈-Alkyl, bevorzugt für OR⁹, C₁-C₆-Alkyl, C₁-C₆-Alkoxy, C₆-C₂₀-Aryl, C₆-C₁₄-Aryl-C₁-C₈-Alkyl, besonders bevorzugt für OR⁹ steht;
   - R⁷ und R⁸: gleich oder verschieden sind und C₁-C₈-Alkyl, vorzugsweise Isopropyl, oder C₅-C₁₂-Cycloalkyl, vorzugsweise bis C₈, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen, wie z.B. Morpholin, und Substituenten, wie OC(O)O-C₁-C₄-Alkyl-Ester, bedeuten; und
   - R⁹: für eine Gruppe der Formel 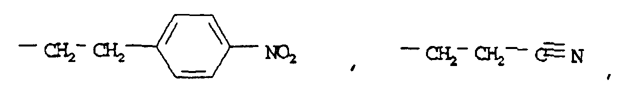 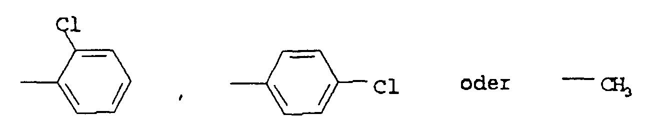
   oder eine Benzylgruppe, welche nicht oder ein- bis vierfach ringsubstituiert, vorzugsweise nicht substituiert ist, wobei der oder die Substituenten unabhängig voneinander Fluor, Chlor, Brom, eine C₁-C₄-Alkyl-, Nitro-, Methoxy-, oder Carboxylgruppe ist, steht;
   - Z: steht für Chlor oder Brom oder einen Rest der Formel NR⁷R⁸, wobei R⁷ und R⁸ wie oben definiert sind;
   in Gegenwart einer Base, vorzugsweise Pyridin oder einer Mischung von Tetrahydrofuran (THF), Dioxan, Dichlormethan (DCM), Chloroform, und/oder Acetonitril mit einem C₁-C₄-Trialkylamin, vorzugsweise Trimethyl-, Triethyl- oder Diisopropylethyl-Amin oder, wenn Z ein Rest der Formel NR⁷R⁸ ist, dann in Gegenwart einer Verbindung der Formel [HNR¹²R¹³R¹⁴]⁽⁺⁾ A⁽⁻⁾, wobei R¹², R¹³, R¹⁴ gleich oder verschieden voneinander sind und eine C₁-C₄-Alkylgruppe und A = Fluor, Chlor, Brom, insbesondere Chlor, bedeuten, oder Tetrazol, vorzugsweise in Gegenwart von Tetrazol, zu einer Verbindung der Formel VIII umsetzt 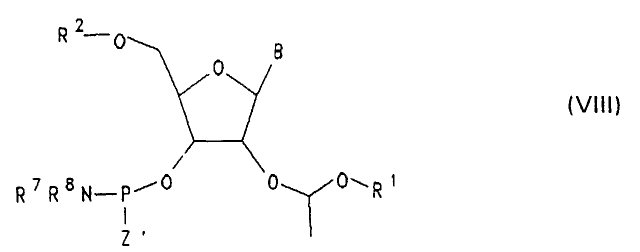 worin R¹, R², R⁷, R⁸, Z' und B wie oben definiert sind;
f) die nach d) erhaltene Verbindung VI nach bekannten Verfahren mit 1 bis 10 Equivalenten, bevorzugt mit 1 bis 2 Equivalenten eines Linkers, wie Bernsteinsäureanhydrid, in einem geeigneten organischen Lösungsmittel, beispielsweise Methylenchlorid, gegebenenfalls nach Zugabe eines Katalysators, beispielsweise 4-Dimethylaminopyridin, zu einer Verbindung der Formel IX 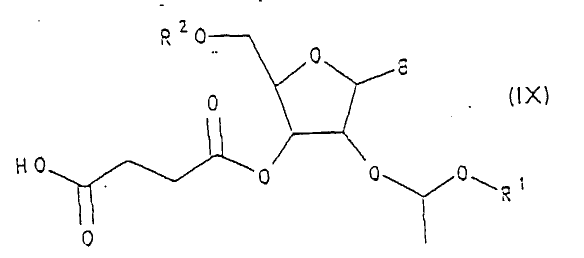 wobei R¹, R² und B wie oben definiert sind, umsetzt und anschließend nach bekannten Verfahren, wie beispielsweise Extraktion, Kristallisation, Chromatographie aufarbeitet, wobei der Bernsteinsäure-Rest in 3'-Position als Linker zu dem in der Synthese eingesetzten Polymerträger dient und alternativ zum Bernsteinsäurelinker auch andere Linker, wie z.B. in Sonveaux [Bioorg. Chem. 14 (1986) 274] beschrieben, verwendet werden können;
g) die Verbindung der Formel X 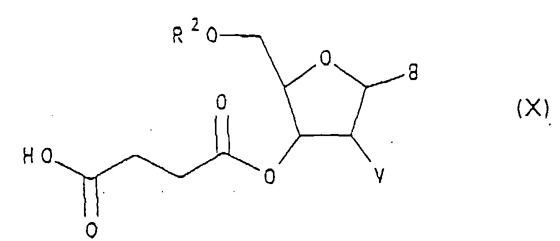 worin
   - R² und B: wie oben definiert sind;
   - V: für Wasserstoff, O-C₁-C₁₈-Alkyl, O-C₁-C₁₈-Alkenyl, O-C₁-C₁₈-Alkinyl oder -O-CH(CH₃)-OR¹, worin R¹ wie oben definiert ist, steht, bevorzugt für Wasserstoff, OCH₃, O-CH₂CH₃, O-CH₂-CH =CH₂ oder O-CH(CH₃)-OR¹, worin
   - R¹: wie oben definiert ist,
   nach bekannten Verfahren auf den festen Träger, wie Aminopropyl-CPG (CPG = Controlled Pore Glass), beispielsweise durch Umsetzung mit DCC und p-Nitrophenol in einem geeigneten Lösungsmittel koppelt [z.B. M.J. Gait, Oligonucleotide Synthesis - a practical approach, IRL Press, 1984];
h) die 5'-Schutzgruppe nach bekannten Verfahren, beispielsweise durch Behandlung mit 1 - 4 %iger Dichloressigsäure in Methylenchlorid oder Chloroform, abspaltet;
i) die erhaltene Verbindung mit einer Verbindung der Formel XI 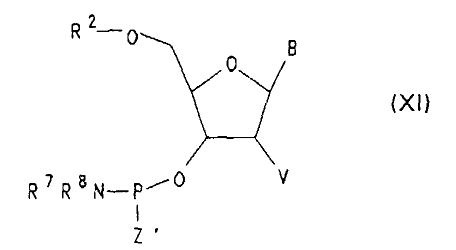 umsetzt, in der R², R⁷, R⁸, V, Z' und B wie oben definiert sind und
j) die erhaltene Verbindung nach bekannten Verfahren oxidiert, beispielsweise durch Umsetzung mit Jod in Gegenwart von wäßrigem Pyridin, Lutidin oder Collidin, gegebenenfalls auch in Gegenwart weiterer organischer Lösungsmittel wie beispielsweise Tetrahydrofuran, oder beispielsweise durch Umsetzung mit N,N,N',N'-Tetraethylthiuramdisulfid in Acetonitril, oder beispielsweise durch Umsetzung mit Jod in Gegenwart von Alkylamin oder Arylamin, wobei die verschiedenen, dem Fachmann bekannten Oxidationsverfahren, die der Herstellung von natürlichen und modifizierten Oligonucleotiden dienen, beispielsweise in Beaucage und Iyer [Tetrahedron 49 (1993) 6123] sowie Uhlmann und Peyman [Chem. Rev. 90 (1990) 543] zusammengefaßt sind, und die Oxidation bevorzugt durch Umsetzung mit Jod in Gegenwart von wäßrigem Pyridin, Lutidin oder Collidin, gegebenenfalls auch in Gegenwart weiterer organischer Lösungsmittel wie Tetrahydrofuran erfolgt;
k) die Reaktionsschritte h-j bis zur gewünschten Kettenlänge wiederholt;
l) das Oligonucleotid nach bekannten Verfahren vom Träger abspaltet, beispielhaft mit NH₃ bei 50 - 60°C, und die Schutzgruppen am Phosphat und Nucleobasen ebenfalls nach bekannten Verfahren abspaltet, wobei man die Verbindung der Formel XII erhält 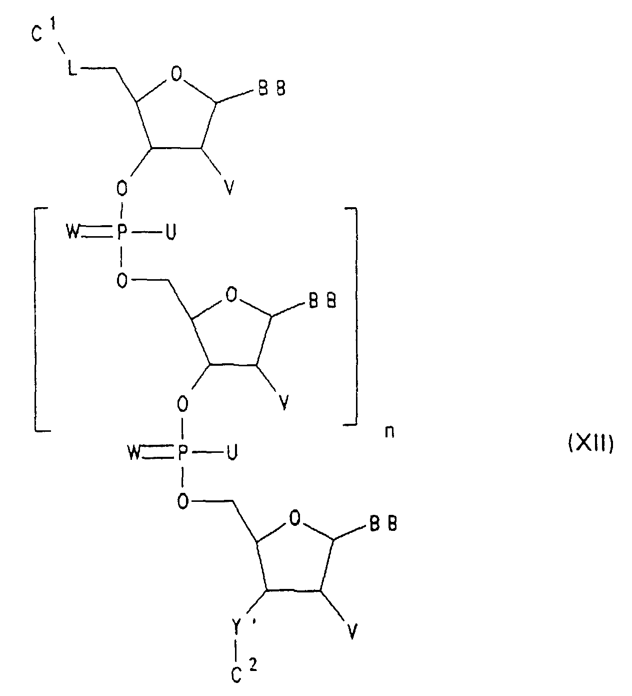 worin n, L, BB, W, V, Y', U, C¹ und C² wie oben definiert sind;
m) die Verbindung der Formel XII im sauren Bereich, vorzugsweise bei pH 1-3, besonders bevorzugt bei pH 1,5-2,5, ganz besonders bevorzugt bei pH 1,8-2,2, mit einer 1-30 %, bevorzugt 2-6 % Lösung von p-Toluolsulfonsäure in einem geeigneten organischen Lösungsmittel, beispielsweise Methylenchlorid/Methanol während 0,5 - 10h, bevorzugt 1-3h, besonders bevorzugt 3h inkubiert, anschließend z.B. mit NH₃/Methanol neutralisiert und nach dem Abdampfen des Lösungsmittels und nach Reinigung, z.B. durch Ausfällen aus Ethanol, Gelchromatographie, Gelelektrophorese sowie HPLC die Verbindung der Formel I erhält [s.a. E. Sonveaux, Bioorg. Chem. 14 (1986) 274].

Beispielhaft für Gruppen, die die intrazelluläre Aufnahme begünstigen, sind verschiedene lipophile Reste wie -O-(CH₂)ₓ-CH₃, worin x eine ganze Zahl von 6-18 bedeutet, -O-(CH₂)ₙ-CH = CH-(CH₂)ₘ-CH₃, worin n und m unabhängig voneinander eine ganze Zahl von 6 bis 12 bedeuten, -O-(CH₂CH₂O)₄-(CH₂)₉-CH₃, -O-(CH₂CH₂O)₈-(CH₂)₁₃-CH₃ und -O-(CH₂CH₂O)₇-(CH₂)₁₅-CH₃, aber auch Steroid-Reste wie Cholesteryl und Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor). Unter Markierungs-Gruppen sind fluoreszierende Gruppen beispielsweise von Dansyl-(= N-Dimethyl-1 -aminonaphthyl-5-sulfonyl-), Fluorescein- oder Coumarin-Derivaten oder chemilumineszierende Gruppen beispielsweise von Acridin-Drivaten zu verstehen sowie das über ELISA nachweisbare Digoxygenin-System, die über das Biotin/Avidin-System nachweisbare Biotin-Gruppe oder aber Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Typische Markierungsgruppen sind 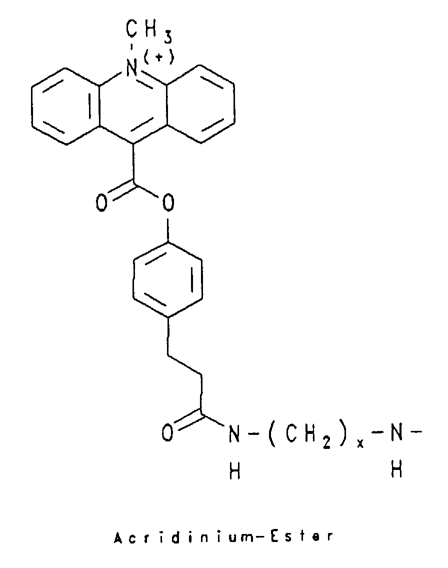 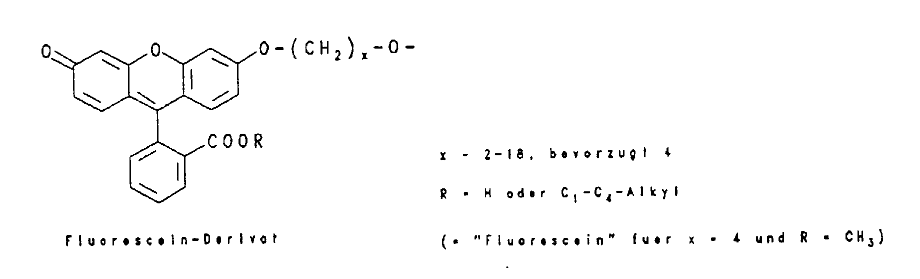 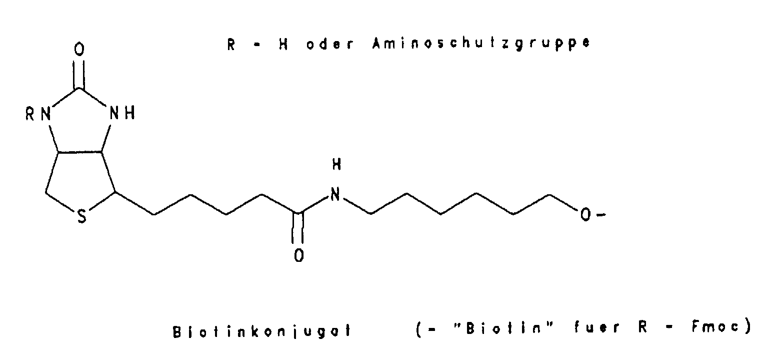 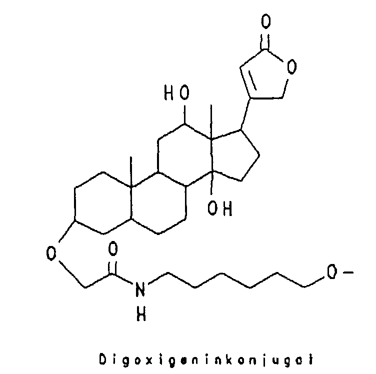 Oligonucleotidanaloga, die an Nucleinsäuren binden bzw. interkalieren und/oder spalten oder quervernetzen, enthalten z.B. Acridin-, Psoralen-, Phenanthrolin, Naphtochinon-, Daunomycin- oder Chlorethylaminoaryl-Konjugate. Typische interkalierende und quervernetzende Reste sind:

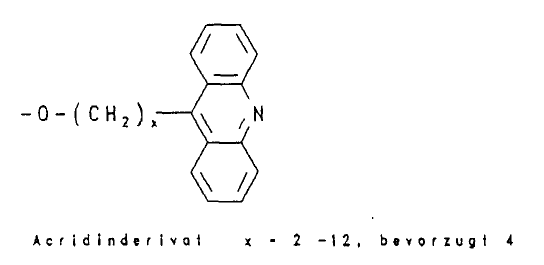

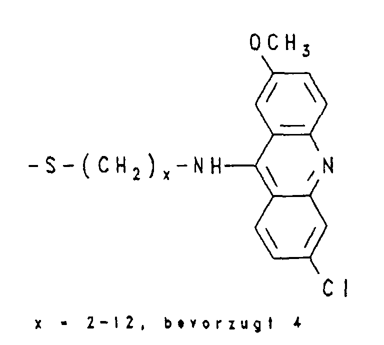

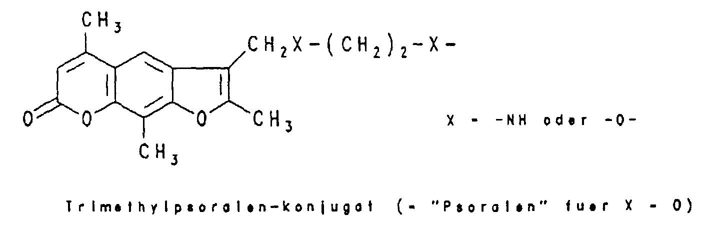

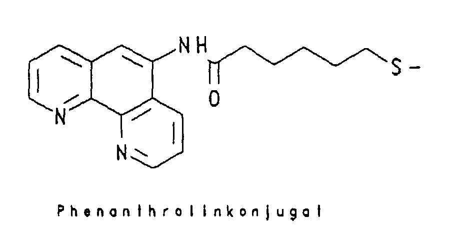

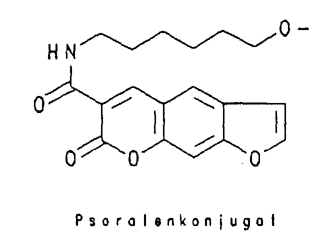

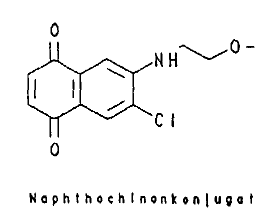

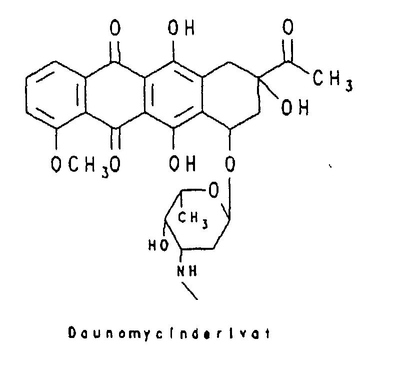

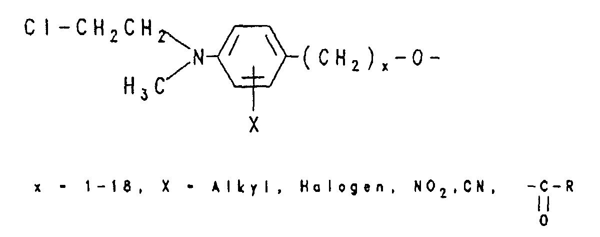

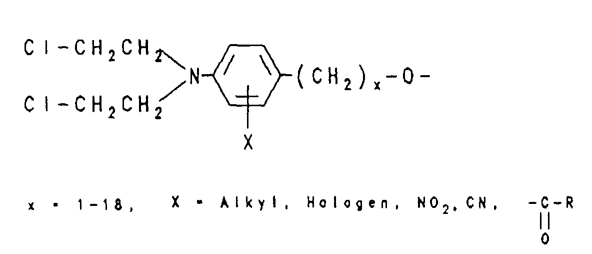

Gegenstand der Erfindung ist weiterhin die Verbindung der Formel VI 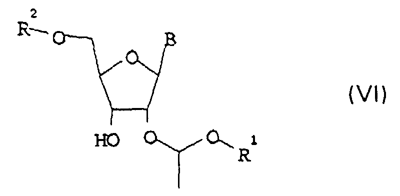 worin
- R¹: für -[CH₂]ᵣ-X steht, mit
r = 1 oder 2 und
- X: für C₆-C₁₂-Aryl steht, wobei Aryl ein- oder mehrfach, bevorzugt ein- bis dreifach, besonders bevorzugt ein- bis zweifach mit Hydroxy, Mercapto, Nitro, Fluor, Chlor, Brom, C₁-C₆-Alkyl, C₁-C₆-Alkoxy, C₁-C₆-Alkylmercapto, C₂-C₆-Alkenyl, C₃-C₆-Alkinyl, C(O)OH, C(O)NH₂, C(O)O-C₁-C₁₈-Alkyl, C(O)O-C₆-C₁₂-Aryl, C(O)C₁-C₁₈-Alkyl, C(O)-C₆-C₁₂-Aryl, O-C(O)NH₂, O-C(O)O-C₁-C₁₈-Alkyl, O-C(O)O-C₆-C₁₂-Aryl, O-C(O)-C₁-C₁₈-Alkyl, O-C(O)-C₆-C₁₂-Aryl, O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch Amino, Hydroxy, Nitro, C₁-C₆-Alkyl, C₁-C₆-Alkoxy, C₁-C₆-Alkoxycarbonyl, Fluor, Chlor oder Brom substituiert sein kann;
für r = 1 steht X weiterhin für Phenyl oder C₁-C₄-Alkoxy-phenyl;
für r = 2 steht X weiterhin für CN, S-Phenyl, SO₂-Phenyl, N-Phthalimid oder NO₂;
für r = 1 steht X bevorzugt für C₆-C₁₂-Aryl, wobei Aryl ein- bis dreifach, bevorzugt ein- bis zweifach mit Fluor, Chlor, Brom, C₁-C₄-Alkyl, C₁-C₄-Alkoxy, O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch C₁-C₄-Alkyl, C₁-C₄-Alkoxy, Fluor, Chlor oder Brom substituiert sein kann;
für r = 2 steht X bevorzugt für 4-Nitrophenyl;
für r = 1 steht X besonders bevorzugt für 2,5-Dichlor-4-pivaloyloxyphenyl, 3-Chlor-4-[2-[4-nitrophenyl)ethoxycarbonyloxy]-phenyl, 2-Chlor-4-[2-[4-nitrophenyl)ethoxycarbonyloxy]-phenyl, 3-Fluor-4-[2-[4-nitrophenyl)ethoxycarbonyloxy]-phenyl, besonders bevorzugt für 3-Fluor-4-[2-[4-nitrophenyl)ethoxycarbonyloxy]-phenyl;
- R²: für Dimethoxytrityl, Monomethoxytrityl, Pixyl, Trityl, bevorzugt für Dimethoxytrityl, steht;
- B: steht für 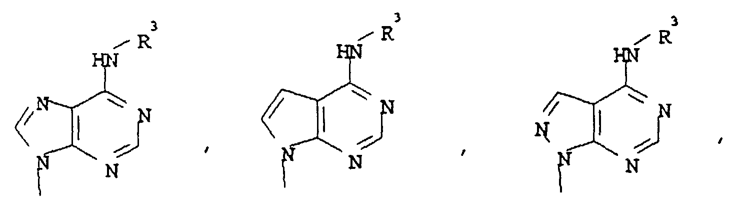 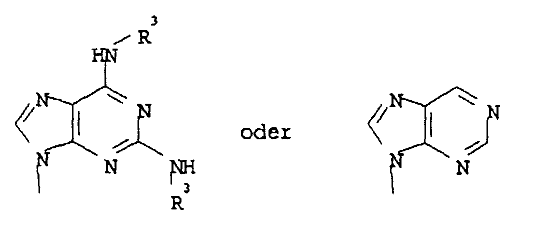 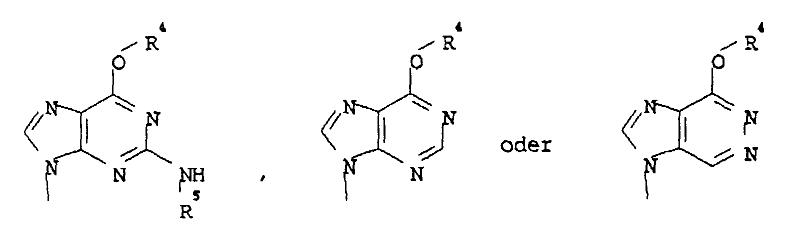 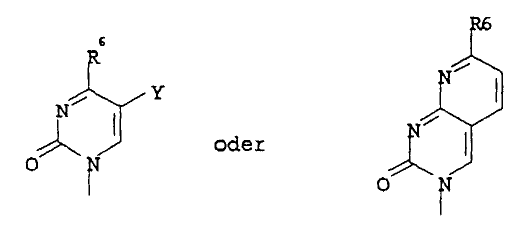 worin
- R³: jeweils unabhängig voneinander eine Gruppe der Formel 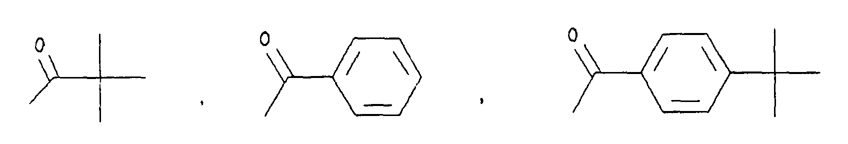 oder 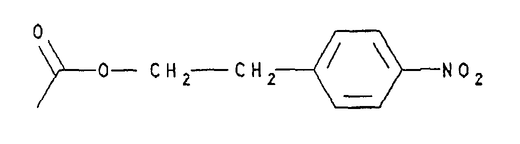 bedeutet;
- R⁴: für Wasserstoff oder 2-(p-Nitrophenyl)ethyl steht;
- R⁵: 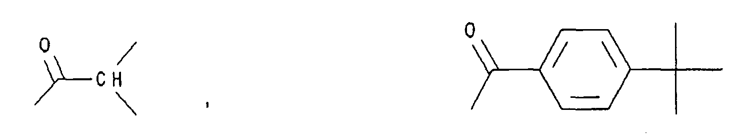 oder 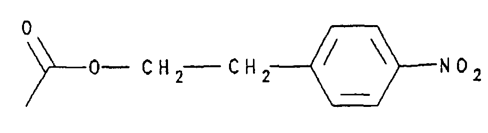
- R⁶: OH, NH₂, 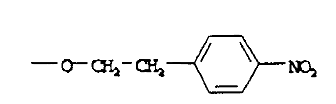 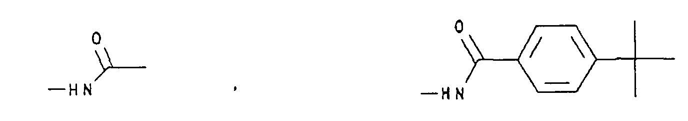 oder 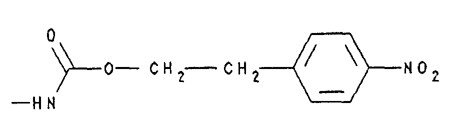 bedeuten;
- Y: für Wasserstoff, C₁-C₄-Alkyl, Fluor, Chlor, Brom oder C₂-C₆-Alkenyl, C₂-C₆-Alkinyl, bevorzugt für Wasserstoff, CH₃ oder 1-Propinyl, besonders bevorzugt für Wasserstoff, steht; oder
- B: kann neben den natürlichen Nucleosid-Basen auch für andere modifizierte Nucleosid-Basen, deren Amino- bzw. Hydroxygruppen durch geeignete, bekannte Schutzgruppen, wie die para-Nitrophenylethyloxycarbonyl-Gruppe, die Benzoyl-Gruppe und die para-(t-Butyl)benzoylgruppe für die Hydroxygruppe und die Benzoyl-, para-(t-Butyl)benzoyl-, para-Nitrophenylethyloxycarbonyl-, Isobutyryl-, para(tert-Butyl)phenylacetyl-Gruppe für die Aminogruppe, geschützt werden stehen.
- B: steht in allen Verbindungen bevorzugt für 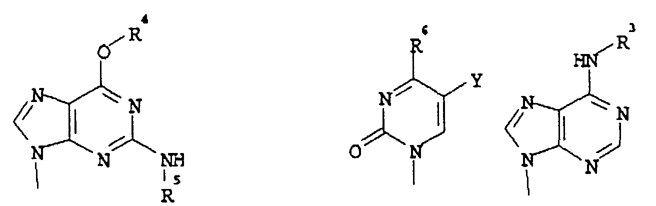
In den Verbindungen der Formel VI kann der Zucker neben der natürlichen β-Konfiguration auch die α-Konfiguration einnehmen, bevorzugt sind Verbindungen mit β-Zuckerkonfiguration.

Weiterhin betrifft die Erfindung Verbindungen der Formel VIII 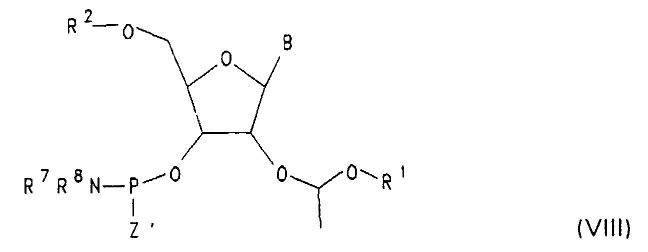 worin
- R¹: für -[CH₂]ᵣ-X steht, mit r = 1 oder 2 und
- X: für C₆-C₁₂-Aryl steht, wobei Aryl ein- oder mehrfach, bevorzugt ein- bis dreifach, besonders bevorzugt ein- bis zweifach mit Hydroxy, Mercapto, Nitro, Fluor, Chlor, Brom, C₁-C₆-Alkyl, C₁-C₆-Alkoxy, C₁-C₆-Alkylmercapto, C₂-C₆-Alkenyl, C₃-C₆-Alkinyl, C(O)OH, C(O)NH₂, C(O)O-C₁-C₁₈-Alkyl, C(O)O-C₆-C₁₂-Aryl, C(O)-C₁-C₁₈-Alkyl, C(O)-C₆-C₁₂-Aryl, O-C(O)NH₂, O-C(O)O-C₁-C₁₈-Alkyl, O-C(O)O-C₆-C₁₂-Aryl, O-C(O)-C₁-C₁₈-Alkyl, O-C(O)-C₆-C₁₂-Aryl, O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch Amino, Hydroxy, Nitro, C₁-C₆-Alkyl, C₁-C₆-Alkoxy, C₁-C₆-Alkoxycarbonyl, Fluor, Chlor oder Brom substituiert sein kann;
für r = 1 steht X weiterhin für Phenyl oder C₁-C₄-Alkoxy-phenyl;
für r = 2 steht X weiterhin für CN, S-Phenyl, SO₂-Phenyl, N-Phthalimid oder NO₂;
für r = 1 steht X bevorzugt für C₆-C₁₂-Aryl, wobei Aryl ein- bis dreifach, bevorzugt ein- bis zweifach mit Fluor, Chlor, Brom, C₁-C₄-Alkyl, C₁-C₄-Alkoxy, O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch C₁-C₄-Alkyl, C₁-C₄-Alkoxy, Fluor, Chlor oder Brom substituiert sein kann;
für r = 2 steht X bevorzugt für 4-Nitrophenyl;
für r = 1 steht X besonders bevorzugt für 2,5-Dichlor-4-pivaloyloxyphenyl, 3-Chlor-4-[2-[4-nitrophenyl)ethoxycarbonyloxy]-phenyl, 2-Chlor-4-[2-[4-nitrophenyl)ethoxycarbonyloxy]-phenyl, 3-Fluor-4-[2-[4-nitrophenyl)ethoxycarbonyloxy]-phenyl, besonders bevorzugt für 3-Fluor-4-[2-[4-nitrophenyl)ethoxycarbonyloxy]-phenyl;
- R²: für Dimethoxytrityl, Monomethoxytrityl, Pixyl, Trityl, bevorzugt für Dimethoxytrityl, steht;
- Z': für OR⁹ oder C₁-C₁₈-Alkyl, C₁-C₁₈-Alkoxy, C₆-C₂₀-Aryl, C₆-C₁₄-Aryl-C₁-C₈-Alkyl, bevorzugt für OR⁹, C₁-C₆-Alkyl, C₁-C₆-Alkoxy, C₆-C₂₀-Aryl, C₆-C₁₄-Aryl-C₁-C₈-Alkyl, besonders bevorzugt für OR⁹ steht;
- R⁷ und R⁸: gleich oder verschieden sind und C₁-C₈-Alkyl, vorzugsweise Isopropyl, oder C₅-C₁₂-Cycloalkyl, vorzugsweise bis C₈, Benzyl oder Phenyl, oder zusammen mit dem Stickstoffatom, an das sie gebunden sind, einen gesättigten oder ungesättigten heterocyclischen Ring der gegebenenfalls weiteren Heteroatomen, wie z.B. Morpholin, und Substituenten, wie OC(O)O-C₁-C₄-Alkyl-Ester, enthalten kann, bedeuten und
- R⁹: für eine Gruppe der Formel 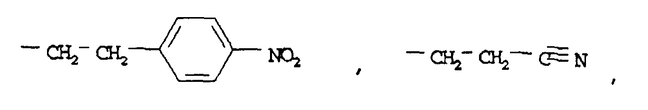 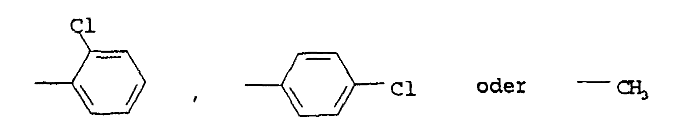 oder eine Benzylgruppe, welche nicht oder ein- bis vierfach ringsubstituiert, vorzugsweise nicht substituiert ist, wobei der oder die Substituenten unabhängig voneinander Fluor, Chlor, Brom, eine C₁-C₄-Alkyl-, Nitro-, Methoxy-, oder Carboxylgruppe ist, steht;
- B: steht für 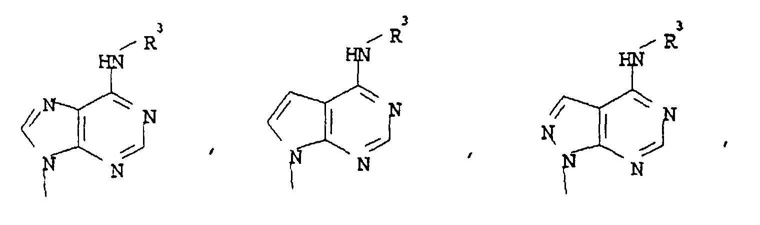 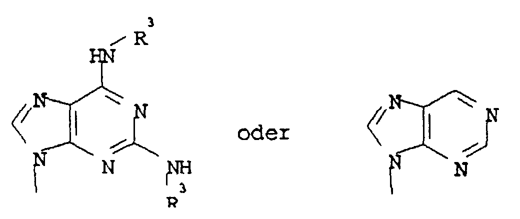 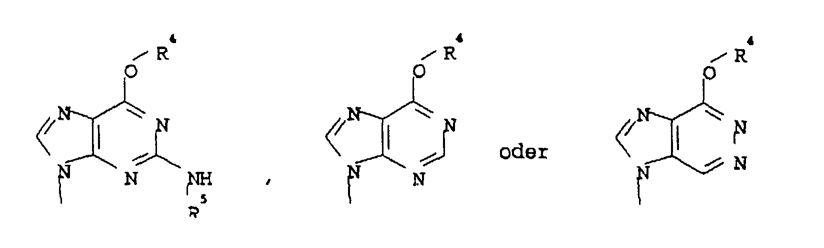 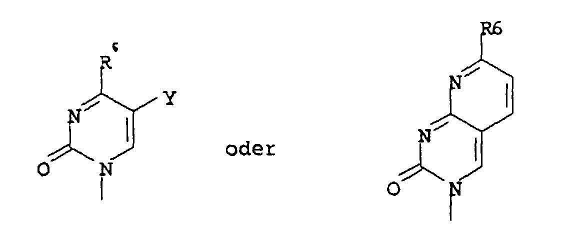 worin
- R³: jeweils unabhängig voneinander eine Gruppe der Formel 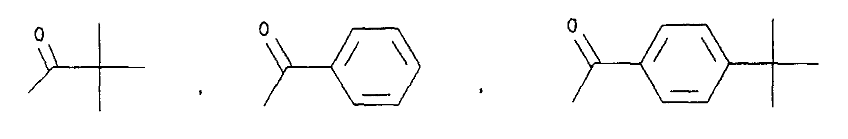 oder 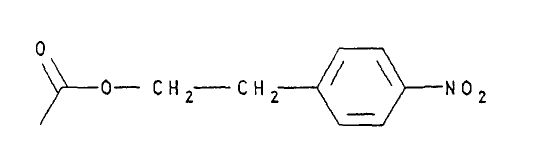 bedeutet;
- R⁴: für Wasserstoff oder 2-(p-Nitrophenyl)ethyl steht;
- R⁵: 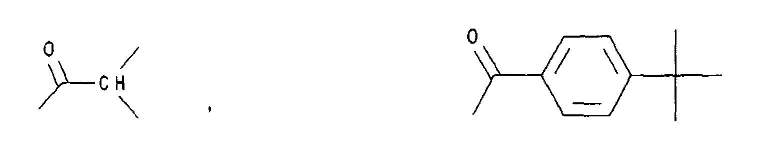 oder 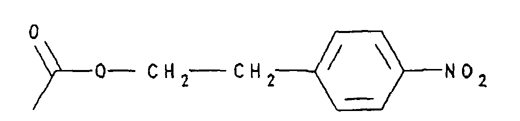
- R⁶: OH, NH₂, 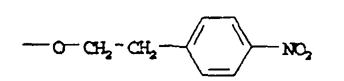 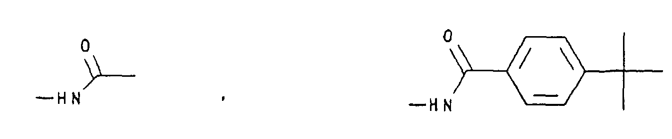 oder 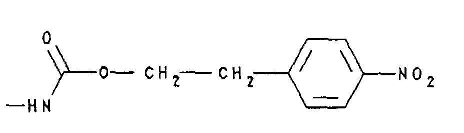 bedeuten;
- Y: für Wasserstoff, C₁-C₄-Alkyl, Fluor, Chlor, Brom oder C₂-C₆-Alkenyl, C₂-C₆-Alkinyl, bevorzugt für Wasserstoff, CH₃ oder 1-Propinyl, besonders bevorzugt für Wasserstoff steht; oder
- B: kann neben den natürlichen Nucleosid-Basen auch für andere modifizierte Nucleosid-Basen, deren Amino- bzw. Hydroxygruppen durch geeignete, bekannte Schutzgruppen, wie die para-Nitrophenylethyloxycarbonyl-Gruppe, die Benzoyl-Gruppe und die para-(t-Butyl)benzoylgruppe für die Hydroxygruppe und die Benzoyl-, para-(t-Butyl)benzoyl-, para-Nitrophenylethyloxycarbonyl-, Isobutyryl-, para(tert-Butyl)phenylacetyl-Gruppe für die Aminogruppe, geschützt werden stehen.

Ebenfalls Gegenstand der Erfindung ist eine Verbindung der Formel XII 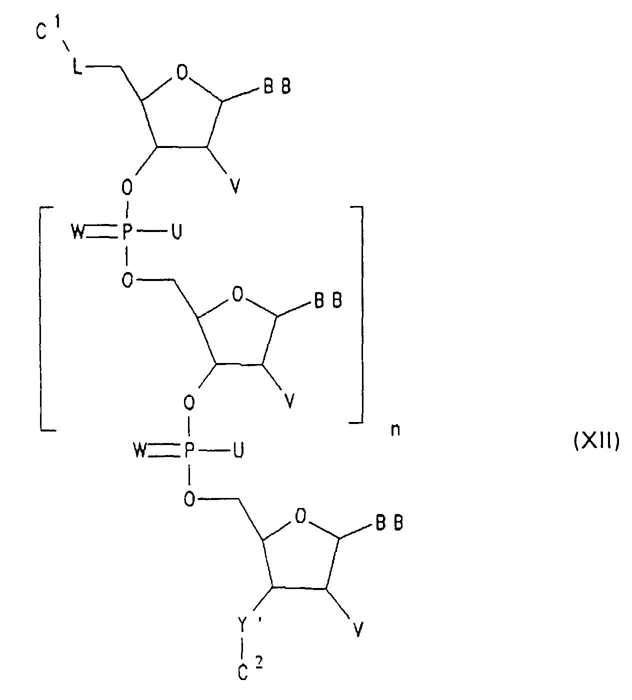 worin
- n: eine Zahl von 1-150, vorzugsweise 4-50, besonders bevorzugt 8-30, ganz besonders bevorzugt 8-20, ist;
- L: Oxy, Sulfandiyl oder Imino, bevorzugt Oxy, bedeutet;
- BB: unabhängig voneinander 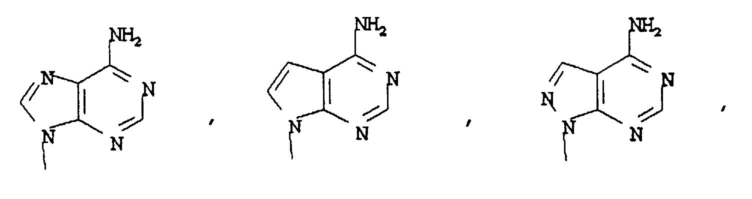 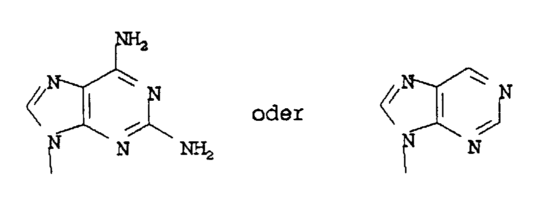 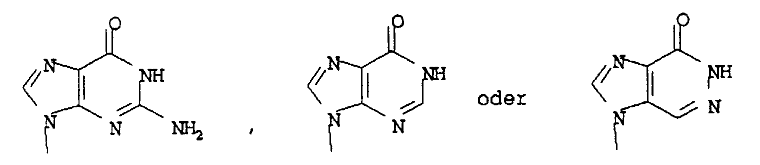 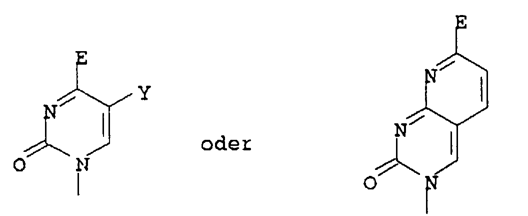 ist, und
- E: für OH oder NH₂ und
- Y: für Wasserstoff, C₁-C₄-Alkyl, Fluor, Chlor, Brom oder C₂-C₆-Alkenyl, C₂-C₆-Alkinyl, bevorzugt für Wasserstoff, CH₃ oder 1-Propinyl, besonders bevorzugt für Wasserstoff, stehen; oder
- BB: kann neben den natürlichen Nucleosid-Basen auch für andere modifizierte Nucleosid-Basen, wie 5-(Hydroxymethyl)uridin, 5-Aminouridin, Pseudouridin, Dihydrouridin, Inosin, 8-Aza-7-deazaadenosin, Tubercidin, Nebularin, Xanthosin, 2-Aminoadenosin-, Etheno-Adenosin, 7-DeazaGuanosin, O4-Methylthymidin, N6-Methyladenosin, O6-Methylguanosin oder Pyridopyrimidin-Nucleoside stehen;
- W: unabhängig voneinander Sauerstoff oder Schwefel, bevorzugt Sauerstoff, bedeutet;
- V: unabhängig voneinander für Wasserstoff, -O-C₁-C₁₈-Alkyl, -O-C₁-C₁₈-Alkenyl, -O-C₁-C₁₈-Alkinyl, oder -O-CH(CH₃)-OR¹, worin R¹ wie oben definiert ist, steht, mindestens jedoch einmal für -O-CH(CH₃)-OR¹; bevorzugt für Wasserstoff, -OCH₃, -O-CH₂CH₃, -O-CH₂, CH = CH₂, oder -O-CH(CH₃)-OR¹, mindestens jedoch einmal für -O-CH(CH₃)-OR¹;
- Y': für Oxy, Sulfandiyl, Imino, (CH₂)k oder N(CH₂)k steht, wobei k eine ganze Zahl von 1 bis 18, vorzugsweise 1 bis 6, bedeutet, bevorzugt steht Y' für Oxy;
- U: für Hydroxy, Mercapto, SeH, C₁-C₁₈-Alkoxy, C₁-C₁₈-Alkyl, C₆-C₂₀-Aryl, C₆-C₁₄-Aryl-C₁-C₈-alkyl, NHR¹⁷, NR¹⁷R¹⁸ oder einen Rest der Formel (OCH₂CH₂)_{c}O(CH₂)_{c'}CH₂R²⁰ steht, bevorzugt steht U für Hydroxy, Mercapto, C₁-C₆-Alkoxy, C₁-C₆-Alkyl, NHR¹⁷, NR¹⁷R¹⁸, besonders bevorzugt für Hydroxy oder C₁-C₆-Alkyl; wobei
R¹⁷ C₁-C₁₈-Alkyl, C₆-C₂₀-Aryl, C₆-C₁₄-Aryl-C₁-C₈-alkyl, -(CH₂)_{d}-[NH(CH₂₋)_{d}]_{d'}-NR¹⁹R¹⁹; bevorzugt C₁-C₈-Alkyl oder Methoxyethyl, besonders bevorzugt C₁-C₄-Alkyl oder Methoxyethyl, worin
d eine ganze Zahl von 2 bis 6 und
d' eine ganze Zahl von 0 bis 6 sind, und
R¹⁸ C₁-C₁₈-Alkyl, C₆-C₂₀-Aryl, C₆-C₁₄-Aryl-C₁-C₈-alkyl steht oder im Falle von NR¹⁷R¹⁸ zusammen mit R¹⁷ und dem sie tragenden Stickstoffatom einen 5-6-gliedrigen heterocyclischen Ring bedeutet, der zusätzlich ein weiteres Heteroatom aus der Reihe O, S, N enthalten kann, wie z.B. der Morpholinyl- und der Imidazolinyl-Ring;
R¹⁹ unabhängig voneinander Wasserstoff, oder C₁-C₆-Alkyl oder C₁-C₄-Alkoxy-C₁-C₆-alkyl ist;
c eine ganze Zahl von 1 bis 100, bevorzugt 3 bis 20 und besonders bevorzugt 3 bis 8;
c' eine ganze Zahl von 0 bis 18, bevorzugt 0 bis 15;
R²⁰ Wasserstoff oder eine funktionelle Gruppe wie Hydroxy, Amino, NHR¹⁷, COOH, CONH₂, COOR²¹, oder Fluor, Chlor oder Brom, bedeutet, mit R²¹ C₁-C₄-Alkyl, vorzugsweise Methyl, sind;
- C¹ und C²: gleich oder verschieden sind und für Wasserstoff, C₁-C₁₈-Alkyl, C₂-C₁₈-Alkenyl, C₂-C₁₈-Alkinyl, C₁-C₁₈-Alkylcarbonyl, C₂-C₁₈-Alkenylcarbonyl, C₂-C₁₈-Alkinylcarbonyl, C₆-C₂₀-Aryl, C₆-C₁₄-Aryl-C₁-C₈-alkyl, einen Rest der Formel II stehen, 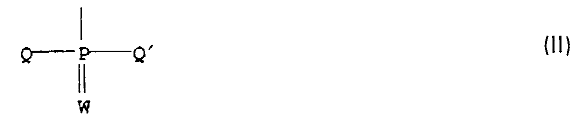 wobei
W wie oben definiert ist,
Q und Q' unabhängig voneinander für Hydroxy, Mercapto, SeH, C₁-C₂₂-Alkoxy, -O(CH₂)_{b}-NR¹⁵R¹⁶ stehen, mit
b 1 bis 6, und
R¹⁵ und R¹⁶ unabhängig voneinander Wasserstoff, C₁-C₁₈-Alkyl, vorzugsweise C₁-C₈-Alkyl, C₆-C₂₀-Aryl, C₆-C₁₄-Aryl-C₁-C₈-alkyl, C₆-C₁₄-Aryl-C₁-C₈-alkoxy, wobei Aryl auch Heteroaryl bedeutet und Aryl gegebenenfalls durch 1, 2, oder 3 gleiche oder verschiedene Reste aus der Reihe Carboxy, Amino, Nitro, C₁-C₄-Alkylamino, C₁-C₆-Alkoxy, Hydroxy, Fluor, Chlor, Brom und Cyano substituiert ist, C₁-C₁₈-Alkylmercapto, NHR¹⁷, NR¹⁷R¹⁸, wobei R¹⁷ und R¹⁸ wie oben definiert sind, oder zusammen mit dem sie tragenden Stickstoffatom einen 3-6 gliedrigen Ring bilden, oder
- Q bzw. Q': steht bevorzugt für Hydroxy, Mercapto, OCH₂CH₃, O-i-C₃H₇, O-n-C₆H₁₃, O-n-C₁₈H₃₇, O-(CH₂)₃-(3-Pyridyl), O-(CH₂)₂-(4-Nitrophenyl), Farnesyl, Phytyl, Vitamin A, Vitamin E, Testosteron, Cholesterol, CH₃, O- (CH₂)₄-(9-Acridin); oder
- Q und Q': stehen für einen Rest der Formel III 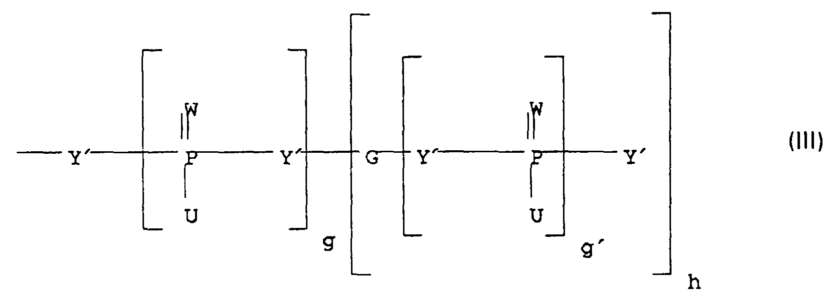 mit
g und g' = 0 oder 1,
h = 0 bis 10,
G C₂-C₁₂-Alkylen, insbesondere C₂-C₄-Alkylen, C₆-C₁₄-Aryl-di-C₁-C₈-alkylen, C₆-C₁₈-Arylen, gegebenenfalls durch Fluor, Chlor, Brom, Amino, Hydroxy, C₁-C₁₈-Alkyl, C₁-C₁₈-Alkoxy, C₁-C₁₈-Alkoxycarbonyl, C₆-C₁₄-Aryl, C₆-C₁₄-Aryl-C₁-C₁₈-alkyl, oder C₆-C₁₄-Aryl-C₁-C₈-alkoxy ein- bis dreifach substituiert sein kann, einer Gruppe der Formel (CH₂CH₂N')ᵢCH₂CH₂ oder (CH₂N')ᵢCH₂, worin i eine ganze Zahl von 1 bis 11, vorzugsweise 1 bis 5, ist und N' für Oxy, Sulfandiyl, Imino oder Methylen steht, und
W, U und Y' sind wie oben definiert; oder
- Q und Q': bedeuten eine Gruppe, die die intrazelluläre Aufnahme begünstigt oder als Markierung einer DNA-Sonde dient oder bei der Hybridisierung des Oligonucleotidanalogons an die Target-Nucleinsäure diese unter Quervernetzung oder Spaltung angreift, wobei in Formel XII die Nucleinsäuresequenz auch ein- oder mehrfach durch Linker der Formel III unterbrochen sein kann und Konjugate nach bekannten Verfahren auch über die Nucleinbasen oder über das Phosphodiester- oder Phosphothiodiester-Backbone gebildet werden können.

### Beispiele:

### 1.1 2'-O-(1-benzyloxy)ethyl-N⁴-NPEOC-3',5'-tetraisopropyldisiloxan-1,3-diylcytidin

In jeweils 10 ml absolutem Toluol werden 678 mg (1,0 mmol) 3',5'-geschütztes N⁴-NPEOC-Cytidin gelöst und am Rotationsverdampfer zweimal koevaporiert. Dann nimmt man den Schaum in 30 ml abs. Toluol auf, fügt 335 mg (2,5 mmol) des Benzylvinylethers zu und versetzt die farblose Lösung mit 2,0 ml (20 mg = 0,11 mmol) einer Stammlösung p-TsOH·H₂O in abs. THF (1,0 g/100 ml). Nachdem über Nacht bei Raumtemperatur gerührt wurde (DC-Kontrolle !) wird die klare Reaktionslösung mit EE auf 200 ml verdünnt und mit zweimal 100 ml ges. NaHCO₃-Lösung und 100 ml ges. NaCl-Lösung ausgeschüttelt.
Die wäßrigen Phasen werden mit 100 ml EE zurückextrahiert, die vereinigten organischen Phasen über Na₂SO₄ getrocknet, filtriert und einrotiert. Die weitere Reinigung erfolgt säulenchromatographisch über Kieselgel.

Die Produktfraktionen werden einrotiert und mehrmals mit MeOH/CH₂Cl₂ koevaporiert. Man erhält so 690 mg (0,84 mmol) farblosen Schaum, der aus beiden Diastereomeren (P1 + P2) besteht. Das entspricht einer Ausbeute von 84 % d.Th.;

### 1.2 2'-O-(1-benzyloxy)ethyl-N⁴-NPEOC-cytidin

520 mg (0,64 mmol) 2'-O-(1-benzyloxy)ethyl-N⁴-NPEOC-3',5'-tetraisopropyldisiloxan-1,3-diyl-cytidin (Beispiel 1.1) werden in 32 ml 0,2 N NH₄F-Lösung (6,4 mmol) aufgenommen und bei Raumtemperatur über Nacht gerührt. Die Lösung wird am Rotationsverdampfer fast bis zur Trockne eingeengt, die entstandene Suspension mit 5 ml CH₂Cl₂ aufgenommen und kurz mit Ultraschall behandelt, bevor man das Rohprodukt auf die Säule aufträgt. Die Säule ist mit 20 g Kieselgel beschickt und mit Toluol equilibriert.

Laufmittelgradienten (Toluol/EE/MeOH in ml): 100/0/0, 50/50/2,5, 50/50/5 (P1 + P2);
Fraktionen mit jeweils 20 ml;

Die Produktfraktionen werden einrotiert und mehrmals mit MeOH/CH₂Cl₂ koevaporiert. Man erhält so 320 mg (0,56 mmol) farblosen Schaum, der aus beiden Diasteromeren (P1 + P2) besteht. Das entspricht einer Ausbeute von 88 % d.Th..

### 1.3 2'-O-(1-benzyloxy)ethyl-5'-DMTR-N⁴-NPEOC-cytidin

980 mg (1,72 mmol) vorgetrocknetes 2'-O-(1-benzyloxy)ethyl-N⁴-NPEOC-cytidin (Beispiel 1.2) werden zweimal mit je 20 ml abs. Toluol am Rotationsverdampfer koevaporiert, dann in 10 ml abs. Pyridin aufgenommen, mit 50 ml abs. Toluol verdünnt und anschließend mit 700 mg DMTR-Cl (2,07 mmol) versetzt. Die Reaktionslösung läßt man über Nacht bei Raumtemperatur rühren und bricht die Reaktion dadurch ab, indem man 1 ml MeOH zusetzt.
Nach 5 Minuten wird die Lösung im Vakuum zum Öl einrotiert, mit 200 ml EE aufgenommen, mit zweimal 100 ml ges. NaHCO₃-Lösung und 100 ml ges. NaCl-Lösung ausgeschüttelt. Die wäßrige Phase wird mit 150 ml EE zurückextrahiert, die vereinigten org. Phasen mit Na₂SO₄ getrocknet, filtriert und zum Öl eingeengt. Die weitere Reinigung erfolgt säulenchromatographisch über Kieselgel.

Die Produktfraktionen werden einrotiert und mehrmals mit MeOH/CH₂Cl₂ koevaporiert. Man erhält so 1,45 g (1,661 mmol) chromatographisch reinen farblosen Schaum, der aus beiden Diastereomeren (P1 + P2) besteht. Das entspricht einer Ausbeute von 97 % d.Th.;

### 2. N⁴-NPEOC-5'-O-DMTR-2'-O-(1-benzyloxy)-ethyl-cytidin-3'-O-phosphorigsäure-β-cyano-ethylester-N,N-diisopropylamid

In einem 50 ml-Kolben werden 450 mg (0,5 mmol) des 2'-O-(1-benzyloxy)ethyl-5'-DMTR-N⁴-NPEOC-cytidin (Beispiel 1.3) eingewogen und zweimal mit je 20 ml abs. Toluol koevaporiert. Anschließend fügt man 7,5 ml (1,25 mmol) einer Stammlösung Phosphitylierungsreagenz (Phosphorigsäure-(β-cyanoethylester-N,N-diisopropylamid)chlorid) (0,15 mol/l abs. CH₂Cl₂) zu und engt erneut bis zum Öl ein. Anschließend verdünnt man mit 10 ml absolutem Acetonitril und rührt die Reaktionslösung nach Zugabe von 23 mg Tetrazol (0,33 mmol) bei Raumtemperatur für 6 h. Durch Gießen auf 150 ml NaHCO₃/EE (1/1)-Lösung wird die Reaktion abgebrochen. Man trennt die Phasen im Scheidetrichter, schüttelt erneut mit 50 ml EE aus und wäscht die vereinigten organischen Phasen mit 50 ml ges. NaCl-Lösung. Nach dem Trocknen über Na₂SO₄ und Filtrieren wird am Rotationsverdampfer zum Öl einrotiert.

Die weitere Reinigung erfolgt chromatographisch. Dazu wird ein Säule mit 2 cm Durchmesser mit 20 g Kieselgel beschickt und mit Toluol konditioniert.

Laufmittelgradienten (Toluol/EE in ml): 100/0; 200/70;
Fraktionen zu je 10 ml;

Die Produktfraktionen werden einrotiert und mit je 10 ml CH₂Cl₂/MeOH aufgeschäumt. Getrocknet wird bei Raumtemperatur im Hochvakuum. Man erhält so 459 mg (0,426 mmol) farblosem Schaum, was einer Ausbeute von 85 % d. Th. entspricht.

### 3. 2'-O-(1-benzyloxy)ethyl-5'-DMTR-N⁴-NPEOC-3'-O-succinyl-cytidin

In einem 50 ml-Kolben werden 330 mg (0,37 mmol) 2'-O-(1-benzyloxy)ethyl-5'-DMTR-N⁴-NPEOC-cytidin (Beispiel 1.3) eingewogen, 50 mg (0,490 mmol) Bernsteinsäureanhydrid und 60 mg 4-DMAP hinzugefügt und mit 10 ml absolutem Methylenchlorid aufgenommen. Die entstandene Lösung wird bei Raumtemperatur für 14 h gerührt. Der Reaktionsabbruch erfolgt durch Ausschütteln mit 50 ml Phosphatpuffer (pH = 6.0), nachdem die Reaktionslösung mit 100 ml EE verdünnt worden ist. Man wiederholt die Prozedur noch einmal, extrahiert die organische Phase mit Puffer trocknet über Na₂SO₄ filtriert und engt am Rotationsverdampfer zum Öl ein. Die weitere Reinigung erfolgt chromatographisch über eine Säule mit 2 cm Durchmesser, die mit 15 g Kieselgel beschickt und mit Toluol equilibriert wurde.

Laufmittelgradienten in ml (Toluol/EE): 100/0, 200/50, 150/50 (P) und 100/100 (P);
Fraktionen mit jeweils 10 ml;

Die Produktfraktionen werden einrotiert, mit CH₂Cl₂ aufgenommen und über eine Baumwolle gefüllte Pasteur-Pipette filtriert. Das Filtrat wird erneut eingeengt, mit MeOH und CH₂Cl₂ aufgeschäumt und im Hochvakuum getrocknet. Man erhält so 357 mg (0,361 mmol) farblosen Schaum. Das entspricht einer Ausbeute von 98 % d.Th..

### 4.1 2'-O-(1-benzyloxy)ethyl-N⁶-NPEOC-3',5'-tetraisopropyldisiloxan-1,3-diyladenosin

In jeweils 10 ml absolutem Toluol werden 1,40 g (2,0 mmol) 3',5'-Tetraisopropyldisiloxan-geschütztes N⁶-NPEOC-Adenosin gelöst und am Rotationsverdampfer zweimal koevaporiert. Dann nimmt man den Schaum in 30 ml abs. Toluol auf, fügt 375 mg (2,8 mmol) des Benzylvinylethers zu und versetzt die farblose Lösung mit 2,0 mol 20 mg = 0,11 mmol) einer Stammlösung p-TsOH·H₂O in abs. THF (1,0 g/100 ml). Nachdem über Nacht bei Raumtemperatur gerührt wurde (DC-Kontrolle), wird die klare Reaktionslösung mit EE auf 200 ml verdünnt und mit zweimal 100 ml ges. NaHCO₃-Lösung und 100 ml ges. NaCl-Lösung ausgeschüttelt. Die wäßrigen Phasen werden mit 100 ml EE zurückextrahiert, die vereinigten organischen Phasen über Na₂SO₄ filtriert und einrotiert.
Die weitere Reinigung erfolgt chromatographisch über eine Säule mit 3 cm Durchmesser, die mit 40 g Kieselgel beschickt ist und mit Toluol equilibriert wird.

Laufmittelgradienten (Toluol/EE in mol): 200/0, 160/40, 140/60, 100/100, 100/200 (P1 + P2);
Fraktionen mit jeweils 20 ml;

Die Produktfraktionen werden einrotiert und mehrmals mit MeOH/CH₂Cl₂ koevaporiert. Als Nebenprodukt erhält man so 720 mg (0,74 mmol = 37 % d.Th.), farblosen Schaum, der aus den beiden Diasteromeren (P1 + P2) des disubstituierten Produktes besteht.
Das gewünschte Produkt wird in 57 % d.Th. (980 mg = 1,13 mmol) als farbloser Schaum isoliert. Die Gesamtausbeute liegt bei 94 % d.Th..

### 4.2 2'-O-(1-benzyloxy)ethyl-N⁶-NPEOC-adenosin

2,08 g (2,40 mmol) 2'-O-(1-benzyloxy)ethyl-N⁶-NPEOC-3',5'-tetraisopropyldisiloxan-1,3-diyl-adenosin (Beispiel 4.1) werden in 120 ml einer 0,2 N NH₄F-Lösung (24 mmol) aufgenommen und bei Raumtemperatur über Nacht gerührt. Die Lösung wird am Rotationsverdampfer fast bis zur Trockne eingeengt, die enstandene Suspension mit 20 ml CH₂Cl₂ aufgenommen und kurz mit Ultraschall behandelt, bevor man das Rohprodukt auf die Säule aufträgt.

Die Säule ist mit 80 g Kieselgel beschickt und mit Toluol konditioniert.

Laufmittelgradienten (Toluol/EE/MeOH in ml): 200/0/0, 200/200,0, 200/200/20 (P1 + P2);
Fraktionen mit jeweils 30 ml;

Die Produktfraktionen werden einrotiert und mehrmals mit MeOH/CH₂Cl₂ koevaporiert. Man erhält so 1,38 g (2,321 mmol) farblosen Schaum, der aus beiden Diasteromeren (P1 + P2) besteht. Das entspricht einer Ausbeute von 97 % d.Th..

### 4.3 2'-O-(1-benzyloxy)ethyl-5'-DMTR-N⁶-NPEOC-adenosin

1,120 g (1,84 mmol) vorgetrocknetes 2'-O-(1-benzyloxy)ethyl-N⁶-NPEOC-adenosin (Beispiel 4.2) werden zweimal mit je 20 ml abs. Toluol am Rotationsverdampfer koevaporiert, dann in 20 ml abs. Pyridin aufgenommen, mit 50 ml abs. Toluol verdünnt und anschließend mit 770 mg DMTR-Cl (2,27 mmol) versetzt. Die Reaktionslösung läßt man über Nacht bei Raumtemperatur rühren und bricht die Reaktion dadurch ab, indem man 1 ml MeOH zusetzt. Nach 5 Minuten wird die Lösung im Vakuum zum Öl einrotiert, mit 200 ml EE aufgenommen, mit zweimal 100 ml ges. NaHCO₃-Lösung und 100 ml ges. NaCl-Lösung ausgeschüttelt. Die wäßrige Phase wird mit 150 ml EE zurückextrahiert, die vereinigten organischen Phasen mit Na₂SO₄ getrocknet, filtriert und zum Öl eingeengt. Die weitere Reinigung erfolgt chromatographisch über eine Säule mit 5 cm Durchmesser, die mit 80 g Kieselgel beschickt ist und mit Toluol equilibriert wird.

Laufmittelgradienten (Toluol/EE/MeOH in ml): 200/0, 200/200, 200/200/10, 200/200/20, 200/200/40;
Fraktionen mit jeweils 30 ml;

Die Produktfraktionen werden einrotiert und mehrmals mit MeOH/CH₂Cl₂ koevaporiert. Man erhält so 1,52 g (1,69 mmol) chromatographisch reinen farblosen Schaum, der aus beiden Diastereomeren (P1 + P2) besteht. Das entspricht einer Ausbeute von 90 % d.Th..

### 5. 2'-O-(benzyloxy)ethyl-5'-O-DMTR-N⁶-NPEOC-adenosin-3'-O-phosphorigsäure-β-cyanoethylester-N,N-diisopropylamid

In einem 50 ml-Kolben werden 450 mg (0,5 mmol) 2'-O-(1-benzyloxy)ethyl-5'-DMTR-N⁶-NPEOC-adenosin (Beispiel 4.3) eingewogen und zweimal mit je 20 ml abs. Toluol koevaporiert. Anschließend fügt man 7,5 ml (1,1 mmol) einer Stammlösung Phosphitylierungsreagenz (0,15 mol/l abs. CH₂Cl₂) zu und engt erneut bis zum Öl ein. Daraufhin verdünnt man das Öl in 10 ml abs. Acetonitril und rührt die Reaktionslösung nach Zugabe von 24 mg Tetrazol (0,38 mmol). Durch Gießen auf 150 ml NaHCO₃/EE (1/1)-Lösung wird die Reaktion abgebrochen. Man trennt die Phasen im Scheidetrichter, schüttelt erneut mit 50 ml EE aus und wäscht die vereinigten organischen Phasen mit 50 ml ges. NaCl-Lösung. Nach dem Trocknen über Na₂SO₄ und Filtrieren wird am Rotationsverdampfer zum Öl einrotiert. Die weitere Reinigung erfolgt chromatographisch. Dazu wird eine Säule mit 2 cm Durchmesser mit 20 g Kieselgel beschickt und mit Toluol konditioniert.

Laufmittelgradienten (Toluol/EE in ml): 100/0; 200/70;
Fraktionen zu je 10 ml;

Die Produktfraktionen werden einrotiert und mit je 10 ml CH₂Cl₂/MeOH aufgeschäumt. Getrocknet wird bei Raumtemperatur im Hochvakuum. Man erhält so 390 mg (0,340 mmol) farblosen Schaum. Die Ausbeute liegt damit bei 68 % d.Th..

### 6. N⁶-NPEOC-5'-DMTR-3'-succinoyl-2'-O-(1-benzyloxy)ethyl-adenosin

In einem 50 ml-Kolben werden 330 g 2'-O-(2-benzyloxy)ethyl-5'-DMTR-N⁶-NPEOC-adenosin (Beispiel 4.3) eingewogen, 50 mg (0,490 mmol) Bernsteinsäureanhydrid und 60 mg 4-DMAP hinzugefügt und mit 10 ml absolutem Methylenchlorid aufgenommen. Die entstandene Lösung wird bei Raumtemperatur für 14 h gerührt. Der Reaktionsabbruch erfolgt durch Ausschütteln mit 50 ml Phosphatpuffer (pH = 6,0), nachdem die Reaktionslösung mit 100 ml EE verdünnt worden ist. Man wiederholt die Prozedur noch einmal, extrahiert die organische Phase mit Puffer, trocknet über Na₂SO₄, filtriert und engt am Rotationsverdampfer zum Öl ein. Die weitere Reinigung erfolgt chromatographisch über eine Säule mit 2 cm Durchmesser, die mit 15 g Kieselgel beschickt ist und mit Toluol equilibriert wurde.

Laufmittelgradient in ml (Toluol/EE): 100/0, 100/50 (P1 + P2);
Fraktionen zu je 10 ml;

Die Produktfraktionen werden einrotiert, mit CH₂Cl₂ aufgenommen und über eine mit Baumwolle gefüllte Pasteur-Pipette filtriert. Das Filtrat wird erneut eingeengt, mit MeOH und CH₂Cl₂ aufgeschäumt und im Hochvakuum getrocknet. Man erhält so 290 mg (0,29 mmol) farblosem Schaum. Das entspricht einer Ausbeute von 79 % d.Th..

### 7.1 2'-O-(1-benzyloxy)ethyl-N²-NPEOC-O⁶-NPE-3',5'-tetraisopropyldisiloxan-1,3-diyl-guanosin

In jeweils 20 ml absolutem Toluol werden 3,07 g (3,54 mmol) 3',5'-Tetraisopropyldisiloxan-geschütztes N²-NPEOC-O⁶-NPE-Guanosin gelöst und am Rotationsverdampfer zweimal koevaporiert. Dann nimmt man den Schaum in 80 ml abs. Toluol auf, fügt 525 mg (3,91 mmol) des Benzylvinylethers zu und versetzt die farblose Lösung mit 5,0 ml (50 mg = 0,27 mmol) einer Stammlösung p-TsOH·H₂O in abs. THF (1,0 g/100 ml). Nachdem über Nacht bei Raumtemperatur gerührt wurde (DC-Kontrolle!), wird die klare Reaktionslöung mit EE auf 300 ml verdünnt und mit zweimal 150 ml ges. Na₂HCO₃-Lösung und 150 ml ges. NaCl-Lösung ausgeschüttelt. Die wäßrigen Phasen werden mit 200 ml EE zurückextrahiert, die vereinigten organischen Phasen über NaSO₄ getrocknet, filtriert und einrotiert. Die weitere Reinigung erfolgt chromatographisch über eine Säule mit 3 cm Durchmesser, die mit 90 g Kieselgel beschickt ist und mit Toluol equilibriert wird.

Laufmittelgradienten (Toluol/EE in ml): 200/0, 250/50, 250/100 (P1 + P2);
Fraktionen mit jeweils 30 ml;

Die Produktfraktionen werden einrotiert und mehrmals mit MeOH/CH₂Cl₂ koevaporiert. Man erhält so 3,13 g (3,22 mmol) farblosen Schaum, der aus beiden Diasteromeren (P1 + P2) besteht. Das entspricht einer Ausbeute von 91 % d.Th..

### 7.2 2'-O-(1-benzyloxy)ethyl-N²-NPEOC-O⁶-NPE-guanosin

2,94 g (3,02 mmol) N²-NPEOC-O⁶-NPE-2'-O-(1-benzyloxy)ethyl-3',5'-tetraisopropyl-disiloxan-1,3-diyl-guanosin (Beispiel 7.1) werden in 150 ml einer 0,2 N NH₄F-Lösung (30 mmol) aufgenommen und bei Raumtemperatur über Nacht gerührt. Die Lösung wird am Rotationsverdampfer fast bis zur Trockne eingeengt, die entstandene Suspension mit 20 ml CH₂Cl₂ aufgenommen und kurz mit Ultraschall behandelt, bevor man das Rohprodukt auf die Säule aufträgt. Die Säule ist mit 80 g Kieselgel beschickt und mit Toluol equilibriert.
Laufmittelgradienten (Toluol/EE/MeOH in ml): 200/0/0, 200/200/0, 200/200/20 (P1 + P2);
Fraktionen mit jeweils 30 ml;

Beim Einengen kristallisiert das Produkt aus und wird über einen Büchnertrichter abfiltriert und mit 50 ml MeOH gewaschen. Aus zwei Fraktionen erhält man insgesamt 2,10 g (2,764 mmol) amorphe Festsbustanz, die aus den beiden Diastereomeren (P1 + P2) besteht. Das entspricht einer Ausbeute von 92 % d.Th..

### 7.3 2'-O-(1-benzyloxy)ethyl-5'-DMTR-N²-NPEOC-O⁶-NPE-guanosin

1,590 g (2,093 mmol vorgetrocknetes 2'-O-(1-benzyloxy)ethyl-N²-NPEOC-O⁶-NPE-guanosin (Beispiel 7.2) werden zweimal mit je 30 ml abs. Toluol am Rotationsverdampfer koevaporiert, dann in 30 ml abs. Pyridin aufgenommen, mit 80 ml abs. Toluol verdünnt und anschließend mit 850 mg DMTR-Cl (2,51 mmol) versetzt. Die Reaktionslösung läßt man über Nacht bei Raumtemperatur rühren und bricht die Reaktion dadurch ab, indem man 1 ml MeOH zusetzt. Nach 5 Minuten wird die Lösung im Vakuum zum Öl einrotiert, mit 200 ml EE aufgenommen, mit zweimal 100 ml ges. NaHCO₃-Lösung und 100 ml ges. NaCl-Lösung ausgeschüttelt. Die wäßrige Phase wird mit 150 ml EE zurückextrahiert, die vereinigten organischen Phasen mit Na₂SO₄ getrocknet, filtriert und zum Öl eingeengt. Die weitere Reinigung erfolgt säulenchromatographisch über Kieselgel.

Die Produktfraktionen werden einrotiert und mehrmals mit MeOH/CH₂Cl₂ koevaporiert. Man erhält so 2,190 g (2,06 mmol) chromatographisch reinen farblosen Schaum, der aus beiden Diasteromeren (P1 + P2) besteht. Das entspricht einer Ausbeute von 99 % d.Th..

### 8. N²-NPEOC-O⁶-NPE-5'-O-DMTR-2'-(1-benzyloxy)ethyl-guanosin-3'-O-phosphorigsäure-β-cyano-ethylester-N,N-diisopropylamid

In einem 50 ml-Kolben werden 530 mg (0,5 mmol) des 2'-O-(1-benzyloxy)ethyl-5'-DMTR-N²-NPEOC-O⁶-NPE-guanosin (Beispiel 7.3) eingewogen und zweimal mit je 20 ml abs. Toluol koevaporiert. Anschließend fügt man 7,25 ml einer Stammlösung Phosphitylierungsreagenz (0,15 mol/abs. CH₂Cl₂) zu und engt bis zum Öl ein. Anschließend verdünnt man in 10 ml Acetonitril und rührt die Reaktionslösung nach Zugabe von 28 mg Tetrazol (0,38 mmol).
Durch Gießen auf 150 ml NaHCO₃/EE (1/1)-Lösung wird die Reaktion abgebrochen. Man trennt die Phasen im Scheidetrichter, schüttelt erneut mit 50 ml EE aus und wäscht die vereinigten organischen Phasen mit 50 ml ges. NaCl-Lösung. Nach dem Trocknen über Na₂SO₄ und Filtrieren wird am Rotationsverdampfer zum Öl einrotiert. Die weitere Reinigung erfolgt chromatographisch. Dazu wird eine Säule mit 2 cm Durchmesser mit 20 g Kieselgel beschickt und mit Toluol konditioniert.

Laufmittelgradienten (Toluol/EE in ml): 100/0; 200/70;
Fraktionen zu jeweils 10 ml;

Die Produktfraktionen werden einrotiert und mit je 10 ml CH₂Cl₂/MeOH aufgeschäumt. Getrocknet wird bei Raumtemperatur im Hochvakuum. Man erhält so 510 mg (0,404 mmol) farblosen Schaum, was einer Ausbeute von 81 % d.Th. entspricht.

### 9. 2'-O-(1-benzyloxy)ethyl-5'-DMTR-N²-NPEOC-O⁶-NPE-3'-O-succinoylguanosin

In einem 50 ml-Kolben werden 390 mg (0,367 mmol) 2'-O-(1-benzyloxy)ethyl-5'-DMTR-N²-NPEOC-O⁶-NPE-guanosin (Beispiel 7.3) eingewogen. 58 mg (0,490 mmol) Bernsteinsäureanhydrid und 72 mg 4-DMAP (0,589 mmol) hinzugefügt und mit 10 ml absolutem Methylenchlorid aufgenommen. Die entstandene Lösung wird bei Raumtemperatur über Nacht gerührt. Der Reaktionsabbruch erfolgt durch Ausschütteln mit 50 ml Phosphatpuffer (pH = 6,0), nachdem die Reaktionslösung mit 100 ml EE verdünnt worden ist. Man wiederholt die Prozedur noch einmal, extrahiert die organische Phase mit Puffer, trocknet bei Na₂SO₄, filtriert und engt am Rotationsverdampfer zum Öl ein. Die weitere Reinigung erfolgt chromatographisch über eine Säule mit 2 cm Durchmesser, die mit 15 g Kieselgel beschickt ist und mit Toluol equlibiriert wurde.

Laufmittelgradienten in ml (Toluol/EE): 100/0, 200/50, 150/50/ (P) und 100/100 (P);
Fraktionen zu je 10 ml;

Die Produktfraktionen werden einrotiert, mit CH₂Cl₂ aufgenommen und über eine mit Baumwolle gefüllte Pasteur-Pipette filtriert. Das Filtrat wird erneut eingeengt, mit MeOH und CH₂Cl₂ aufgeschäumt und im Hochvakuum getrocknet. Man erhält so 250 mg (0,22 mmol) farblosen Schaum. Das entspricht einer Ausbeute von 61 % d.Th..

### 10.1 2'-O-(1-benzyloxy)ethyl-3',5'-tetra-isopropyl-disiloxan-1,3-diyl-uridin

In jeweils 30 ml absolutem Toluol werden 4,86 g (10,9 mmol) 3',5'-geschütztes Uridin gelöst und am Rotationsverdampfer zweimal koevaporiert. Dann nimmt man den Schaum in 80 ml abs. Toluol auf, fügt 3,36 g (25,0 mmol) des Benzylvinylethers zu und versetzt die farblose Lösung mit 19,2 ml (192 mg = 1,0 mmol) einer Stammlösung p-TsOH·H₂O in abs. THF (1,0g /100 ml). Nachdem über Nacht bei Raumtemperatur gerührt wurde (DC-Kontrolle !), wird die klare Reaktionslösung mit EE auf 300 ml verdünnt und mit zweimal 100 ml ges. NaHCO₃-Lösung und 100 ml ges. NaCl-Lösung ausgeschüttelt. Die wäßrigen Phasen werden mit 100 ml EE zurückextrahiert, die vereinigten organischen Phasen über Na₂SO₄ getrocknet, filtriert und einrotiert. Die weitere Reinigung erfolgt chromatographisch über eine Säule mit 4 cm Durchmesser, die mit 75 g Kieselgel beschickt ist und mit Toluol equilibriert wird.

Laufmittelgradienten (Toluol/EE/MeOH in ml): 200/0, 250/50, 150/100 (P1 + P2);
Fraktionen mit jeweils 50 ml;

Die Produktfraktionen werden einrotiert und mehrmals mit MeOH/CH₂Cl₂ koevaporiert. Man erhält so 5,64 g (9,10 mmol) farblosen Schaum, der aus beiden Diastereomeren (P1 + P2) besteht. Das entspricht einer Ausbeute von 91 % d.Th..

### 10.2 2'-O-(1-benzyloxy)ethyl-uridin

3,10 g (5,00 mmol) 2'-O-(1-benzyloxy)ethyl-3',5'-tetraisopropyldisiloxan-1,3-diyl-uridin (Beispiel 10.1) werden in 250 ml 0,2 N NH₄F-Lösung (50 mmol) aufgenommen und bei Raumtemperatur über Nacht gerührt. Die Lösung wird am Rotationsverdampfer fast bis zur Trockne eingeengt, die entstandene Suspension mit 50 ml CH₂Cl₂ aufgenommen und kurz mit Ultraschall behandelt, bevor man das Rohprodukt auf die Säule (d = 5 cm) aufträgt. Die Säule ist mit 100 g Kieselgel beschickt und mit Toluol equlibriert.

Laufmittelgradienten (Toluol/EE/MeOH in ml): 300/0/0, 250/150/0, 150/150/15, 150/150/30 (P1 + P2);
Fraktionen mit jeweils 50 ml;

Die Produktfraktionen werden einrotiert und mehrmals mit MeOH/CH₂Cl₂ koevaporiert. Man erhält so 1,86 g (4,80 mmol) farblosen Schaum, der aus beiden Diastereomeren (P1 + P2) besteht. Das entspricht einer Ausbeute von 96 % d.Th..

### 10.3 2'-O-(1-benzyloxy)ethyl-5'-DMTR-uridin

780 mg (2,06 mmol) vorgetrocknetes 2'-O-(1-benzyloxy)ethyl-uridin (Beispiel 10.2) werden zweimal mit je 10 ml abs. Toluol am Rotationsverdampfer koevaporiert, dann in 10 ml abs. Pyridin aufgenommen und anschließend mit 780 mg (2,26 mmol) DMTR-Cl versetzt. Die Reaktionslösung läßt man über Nacht bei Raumtemperatur rühren und bricht die Reaktion dadurch ab, indem man 1 ml MeOH zusetzt. Nach 5 Minuten wird die Lösung im Vakuum zum Öl einrotiert, mit 150 ml EE aufgenommen, mit zweimal 50 ml ges. NaHCO₃-Lösung und 50 ml ges. NaCl-Lösung ausgeschüttelt. Die wäßrige Phase wird mit 50 ml EE zurückextrahiert, die vereinigten organischen Phasen mit Na₂SO₄ getrocknet, filtriert und zum Öl eingeengt. Die weitere Reinigung erfolgt chromatographisch über eine Säule mit 3 cm Durchmesser, die mit 30 g Kieselgel beschickt ist und mit Toluol equilibriert wird.

Laufmittelgradienten (Toluol/EE/MeOH in ml): 200/0, 100/100, 100/100/5, 100/100/100;
Fraktionen mit jeweils 30 ml;

Die Produktfraktionen werden einrotiert und mehrmals mit MeOH/CH₂Cl₂ koevaporiert. Man erhält so 1,29 g (1,90 mmol) chromatographisch reinen farblosen Schaum, der aus beiden Diastereomeren (P1 + P2) besteht. Das entspricht einer Ausbeute von 92 % d.Th..

### 11. 2'-O-(1-Benzyloxy)ethyl-5'-O-DMTR-N⁴-NPEOC-uridin-3'-O-(N,N-diisopropyl-2-cyanoethyl)-phosphitamid

In einem 50 ml-Kolben werden 1,00 g (1,47 mmol) des 2'-O-(1-benzyloxy)ethyl-5'-DMTR-uridin (Beispiel 10.3) eingewogen und zweimal mit je 20 ml abs. Toluol koevaporiert. Anschließend fügt man 20,0 ml (3 mmol) einer Stammlösung Phosphitylierungsreagenz (0,15 mol/l abs. CH₂Cl₂) zu und engt erneut bis zum Öl ein. Anschließend verdünnt man in 20 ml Acetonitril und rührt die Reaktionslösung nach Zugabe von 100 mg Tetrazol (1,49 mmol). Durch Gießen auf 250 ml NaHCO₃/EE (1/1)-Lösung wird die Reaktion abgebrochen. Man trennt die Phasen im Scheidetrichter, schüttelt erneut mit 70 ml EE aus und wäscht die vereinigten organischen Phasen mit 70 ml ges. NaCl-Lösung. Nach dem Trocknen über Na₂SO₄ und Filtrieren wird am Rotationsverdampfer zum Öl einrotiert. Die weitere Reinigung erfolgt chromatographisch. Dazu wird eine Säule mit 2 cm Durchmesser mit 15 g Kieselgel beschickt und mit Toluol konditioniert.

Laufmittelgradienten (Toluol/EE in ml): 100/0; 100/100;
Fraktionen zu je 10 ml;

Die Produktfraktionen werden einrotiert und mit je 10 ml CH₂Cl₂/MeOH aufgeschäumt. Getrocknet wird bei Raumtemperatur im Hochvakuum. Man erhält so 1,23 g (1,40 mmol) farblosen Schaum, was einer Ausbeute von 95 % d.Th. entspricht.

### 12. 2'-O-(1-benzyloxy)ethyl-5'-DMTR-3'-succinoyl-uridin

In einem 50 ml-Kolben werden 250 mg (0,368 mmol) 2'-O-(1-benzyloxy)ethyl-5'-DMTR-uridin (Beispiel 10.3) eingewogen, 50 mg (0,490 mmol) Bernsteinsäureanhydrid und 60 mg 4-DMAP hinzugefügt und mit 10 ml absolutem Methylenchlorid aufgenommen. Die entstandene Lösung wird bei Raumtemperatur für 14 h gerührt. Der Reaktionsabbruch erfolgt durch Ausschütteln mit 50 ml Phosphatpuffer (pH = 6,0); nachdem die Reaktionslösung mit 100 ml EE verdünnt worden ist. Man wiederholt die Prozedur noch einmal, extrahiert die organische Phase mit Puffer, trocknet über Na₂SO₄, filtriert und engt am Rotationsverdampfer zum Öl ein. Die weitere Reinigung erfolgt chromatographisch über eine Säule mit 2 cm Durchmesser, die mit 15 g Kieselgel beschickt ist und mit Toluol equilibriert wurde.

Laufmittelgradienten in ml (Toluol/EE): 100/0, 200/50, 150/50 (P) und 100/100 (P);
Fraktionen mit jeweils 10 ml;

Die Produktfraktionen werden einrotiert, mit CH₂Cl₂ aufgenommen und über eine mit Baumwolle gefüllte Pasteur-Pipette filtriert. Das Filtrat wird erneut eingeengt, mit MeOH und CH₂Cl₂ aufgeschäumt und im Hochvakuum getrocknet. Man erhält so 265 mg (0,340 mmol) farblosen Schaum. Das entspricht einer Ausbeute von 93 % d.Th..

### 13.1 2'-O-[1-(4-Nitrophenylethoxy)ethyl]-3',5'-O-(1,1,3,3-tetraisopropyldisiloxan-1,3-diyl)-uridin

2,0 g (4,10 mmol) 3',5'O-(1,1,3,3-Tetraisopropyldisiloxan-1,3-diyl)-uridin werden in 40 ml absolutem Dichlormethan gelöst, mit 3,07 mg (1,63 µmol) p-TsOH·H₂O, 1,18 g (6,15 mmol) 2-(4-Nitrophenyl)ethylvinylether versetzt und 30 Minuten bei Raumtemperatur gerührt. Nach beendeter Reaktion wird mit Dichlormethan verdünnt und 3 x mit je 90 ml gesättigter Natriumhydrogencarbonat-Lösung ausgeschüttelt. Die wäßrigen Phasen extrahiert man mit Dichlormethan zurück, sämtliche organischen Phasen werden vereinigt, über Natriumsulfat getrocknet, abfiltriert und einrotiert. Der Rückstand wird zur Reinigung auf eine Kieselgelsäule (3,5 x 21,5 cm) aufgetragen und mit Toluol > (300 ml), Toluol/Essigester 12:1 (480 ml), 11:1 (210 ml), 10:1 (220 ml), 9:1 (200 ml), 8:1 (180 ml), 7:1 (160 ml), 6:1 (140 ml), 5:1 (240 ml) und 4:1 (200 ml) chromatographiert. nach 1500 ml Laufmittelverbrauch erhält man die erste Produktfraktion. Alle Fraktionen die Produkt enthalten werden vereinigt und einrotiert. Den zurückbleibenden festen Rest kristallisiert man aus 2 ml Toluol/Essigester 1:1 um, die Kristalle werden abgesaugt, mit wenig Toluol/Essigester 1:1 nachgewaschen und bei 40°C im Hochvakuum getrocknet. Man erhält 2,3 g (3,38 mmol, 82 %) Verbindung als leicht gelb gefärbte Kristalle.
Schmelzpunkt: 145°C
DC (Kieselgel): R_{f} = 0,47 + 0,55

| Analyse: C₃₁H₄₉N₃O₁₀Si₂ (679,92) | | | | | | |
|---|---|---|---|---|---|---|
| ber. | C | 54,76 | H | 7,26 | N | 6,18 |
| gef. | | 54,60 | | 7,26 | | 6,09 |

UV (Methanol): λₘₐₓ[nm] (lg ε): 265 (4,28)
¹H-NMR (250 MHz, CDCl₃, TMS, ppm):
9,24 (m, 1H, H-N(3)); 8,12 + 8,09 (2d, o-H zu NO₂(NPEE)); 7.94-7,88 (m, 1H, H-C(6)); 7,43 + 7,36 (2d, 2H, m-H zu NO₂/NPEE)); 5,73-5,64 (m, 2H, H-C(1'), H-C(5)); 5,02-4,91 (m, 1H, CH(NPEE)); 4,26-3,60 (m, 7H, H-C(2'), α-CH₂(NPEE), H-C(3'), H-C(3'), H-C(4'), CH₂(5'));3,03-2,92 (m, 2H, β-CH₂(NPEE)); 1,39 + 1,33 (2d, 3H, CH₃(NPEE)); 1,07-0,90 (m, 28H, Isopropyl)

### 13.2 2'-O-[1-(4-Nitrophenylethoxy)ethyl]-uridin

2,0 mg (2,95 mmol) 2'-O-[1-(4-Nitrophenylethoxy)ethyl]-3',5'-O-(1,1,3,3-tetraisopropyldisiloxan-1,3-diyl)-uridin werden in 8,0 ml absolutem Tetrahydrofuran gelöst, mit 2,22 g (7,06 mmol) Tetrabutylammoniumfluorid · 3 H₂O versetzt, 25 Minuten bei Raumtemperatur gerührt und einrotiert. Man trägt den Rückstand zur Reinigung auf eine Kieselgelsäule (3,5 x 18,0 cm) auf, chromatographiert mit Dichlormethan (250 ml), Dichlormethan/Methanol 100:1 (250 ml), 98:1 (300 ml), 96:1 (300 ml), 94:1 (300 ml), 90:1 (250 ml), 85:1 (500 ml), 80:1 (400 ml) und erhält nach 1400 ml Laufmittelverbrauch die erste Produktfraktion. Sämtliche Fraktionen die Produkt enthalten werden vereinigt und einrotiert. Den festen Rückstand kristallisiert man aus 15 ml Essigester um, saugt den erhaltenen Niederschlag ab, trocknet ihn bei 40°C im Hochvakuum und isoliert 1,13 g (2,59 mmol, 88 %) leicht gelb gefärbte Kristalle von Verbindung.
Schmelzpunkt: 162°C
DC (Kieselgel): R_{f} = 0,42 + 0,46 Dichlormethan/Methanol 9:1

| Analyse: C₁₉H₂₃N₃O₉ (437,40) | | | | | | |
|---|---|---|---|---|---|---|
| ber. | C | 52,17 | H | 5,30 | N | 9,60 |
| gef. | | 51,71 | | 5,47 | | 9,20 |

UV (Methanol): λₘₐₓ [nm] (lg ε): 265 (4,23)
¹H-NMR (250 MHz, d₆-DMSO, ppm):
11,36 (s, 1H, H-N(6)); 8,12 (d, 2H, o-H zu NO₂(NPEE)); 7,92-7,88 (m, 1H, H-C(6)); 7,47 (d, 2H, m-H zu NO₂(NPEE)); 5,89-5,84 (m, 1H, H-C(1')); 5,65-5,62 (m, 1H, H-C(5)); 5,19-5,17 (m, 2H, HO-C(5')), HO-C(3')); 4,83-4,79 (m, 1H, CH(NPEE)); 4.16-3,50 (m, 7H, H-C(2')), H-C(3'), H-C(4'), α-CH₂(NPEE), CH₂(5')); 2,90-2,80 (m, 2H, β-CH₂(NPEE)); 1,22-1,1,13 (m, 3H, CH₃(NPEE)

### 13.3 2'-O-[1-(4-Nitrophenylethoxy)ethyl]-uridin

3,0 g (6,16 mmol) 3',5'-O-(1,1,3,3-Tetraisopropyldisiloxan-1,3-diyl)-uridin werden in 60 ml absolutem Dichlormethan gelöst, in einem Eis/Kochsalz-Bad auf 0°C abgekühlt, mit 4,6 mg (24,18 µmol) p-TsOH·H₂O, 1,78 g (9,24 mmol) 4-Nitrophenylethylvinylether versetzt, im Eisbad 2-3 Stunden gerührt und anschließend mit 0,5 N Natriummethanolat/Methanol-Lösung neutralisiert und einrotiert. Den Rückstand nimmt man in Dichlormethan auf, wäscht 3 x mit je 90 ml gesättigter Natriumhydrogencarbonat-Lösung, extrahiert die wäßrigen Phasen mit Dichlormethan zurück, vereinigt alle organischen Phasen, trocknet sie über Natriumsulfat, filtriert ab und rotiert ein.
Der Rückstand wird in 13,0 µl absolutem Tetrahydrofuran gelöst, mit 4,08 g (0,013 Mol) Tetrabutylammoniumfluorid · 3 H₂O versetzt, bei Raumtemperatur 1 1/4 Stunden gerührt und anschließend einrotiert. Den verbleibenden Rest trägt man zur Reinigung auf eine Flash-Kieselsäure (3,0 x 19,0) auf, eluiert mit Dichlormethan (250 ml), Dichlormethan/Methanol 100:1 (250 ml), 95:1 (285 ml), 90:1 (450 ml), 80:1 (480 ml) und erhält nach 1200 ml Laufmittelverbrauch die erste Produktfraktion. Alle produkthaltigen Fraktionen werden vereinigt, einrotiert und der zurückbleibende feste Rest wird aus 15 ml Essigester umkristallisiert. Man saugt die Kristalle ab und trocknet sie bei 40°C in Hochvakuum. Von dem gewünschten Produkt isoliert man 2,47 g (5,66 mmol, 92 %) als leicht gelb gefärbte Kristalle.
Schmelzpunkt: 162°C
DC (Kieselgel): R_{f} = 0,42 + 0,46 (Dichlormethan/Methanol 9:1)

| Analyse: C₁₉H₂₃N₃O₉ (437,40) | | | | | | |
|---|---|---|---|---|---|---|
| ber. | C | 52,17 | H | 5,30 | N | 9,60 |
| gef. | | 51,71 | | 5,47 | | 9,20 |

UV (Methanol): λₘₐₓ [nm] (Ig ε): 265 (4,23)
¹H-NMR (250 MHz, d₆-DMSO, ppm):
11,36 (s, 1H, H-N(6)); 8,12 (d, 2H, o-H zu NO₂(NPEE)); 7,92-7,88 (m, 1H, H-C(6)); 7,47 (d, 2H, m-H zu NO₂(NPEE)); 5,89-5,84 (m, 1H, H-C(1')); 5,65-5,62 (m, 1H, H-C(5)); 5,19-5,17 (m, 2H, HO-C(5')), HO-C(3')); 4,83-4,79 (m, 1H, CH(NPEE)); 4.16-3,50 (m, 7H, H-C(2')), H-C(3'), H-C(4'), α-CH₂(NPEE), CH₂(5')); 2,90-2,80 (m, 2H, β-CH₂(NPEE)); 1,22-1,1,13 (m, 3H, CH₃(NPEE)

### 13.4 5'-O-(4,4'-Dimethoxytriphenylmethyl)-2'-O-[1(4-nitrophenylethoxy)ethyl]-uridin

0,5 g (1,4 mmol) 2'-O- [1-(4-Nitrophenylethoxy)ethyl]-uridin werden 3 x mit je 15 ml absolutem Pyridin koevaporiert, der Rückstand wird anschließend in 60 ml absolutem Pyridin gelöst, mit 0, 46 g (,37 mmol) 4,4'-Dimethoxytriphenylmethylchlorid versetzt und bei Raumtemperatur 4 Stunden gerührt. Danach verdünnt man das Gemisch mit 10 ml Methanol, rührt weitere 30 Minuten bei Raumtemperatur, rotiert den Ansatz auf die Hälfte ein, nimmt mit Dichlormethan auf und wäscht 2 x mit je 80 ml Wasser. Die wäßrigen Phasen werden mit Dichlormethan rückextrahiert, sämtliche organischen Phasen werden über Natriumsulfat getrocknet, abfiltriert, einrotiert und der Rückstand mehrmals mit Toluol koevaporiert. Zur Reinigung trägt man das Rohprodukt auf eine Kieselgelsäule (4,0 x 25,0 cm) auf, chromatographiert mit Toluol (100 ml), Toluol/Essigester 2:1 (450 ml), 1:1 (600 ml) und erhält nach 550 ml Laufmittelverbrauch die erste Produktfraktion, die in der Folge alle aufgefangen, vereinigt und einrotiert werden. Man löst den Rückstand in 4 ml Dichlormethan, tropft in 500 ml Petrolether ein, saugt den erhaltenen Niederschlag ab und trocknet ihn bei 40°C im Hochvakumm über Paraffinschnitzeln. Es werden 0,67 g (0,91 mmol, 80 %) Verbindung als farbloser, amorpher Feststoff erhalten.
Schmelzpunkt: 136°C
DC (Kieselgel): R_{f} = 0,44 (Dichlormethan/Methanol 15:1)

| Analyse: C₄₀H₄₁N₃O₁₁ (739,78) | | | | | | |
|---|---|---|---|---|---|---|
| ber. | C | 64,94 | H | 5,58 | N | 5,68 |
| gef. | | 65,31 | | 6,09 | | 5,10 |

UV (Methanol): λₘₐₓ [nm] (Ig ε): 204 (4,78); 233 (4,33); 266 (4,22)
¹H-NMR (250 MHz, d₆-DMSO, ppm):
11,4 (s, 1H, NH); 8,11 (d, 2H, o-H zu NO₂(NPEE)); 7,74+ 7,71 (2d, 1H, H-C(6)); 7,48 (d, m-H zu NO₂(NPEE); 7,38-7,22 (m, 9H, o-H zu OCH₃, H(Phenyl)); 6,88 (d, 4H, m-H zu OCH₃); 5,85+5,81 (2d, 1H, H-C(1')); 5,32-5,21 (m, 2H, H-C(5), HO-C(3')); 4,91-4,87 (m, 1H), CH(NPEE)); 4,24-4.12 (m, 2H, H-C(2'), H-C(3')); 3,96 (m, 1H, H-C(4')); 3,72-3,58 (m, 8H, OCH₃, α-CH₂(NPEE)); 3,32-3,19 (m, 2H, CH₂(5')); 2,89 (t, 2H, β-CH₂(NPEE)); 1,26-1,18 (m, 3H, CH₃(NPEE))

### 14. 5'-O-(4,4'-Dimethoxytriphenylmethyl)-2'-O-[1-(4-nitrophenylethoxy)ethyl]-uridin-3'-O-(N,N-diisopropyl-2-cyanoethyl)-phosphitamid

Unter Schutzgasatmospäre werden 0,5 g (0, 67 mmol) 5'-O-4(4'-Dimethoxytriphenylmethyl)-2'-O-[1-(4-nitrophenylethoxy)ethyl]-uridin in 10 ml absolutem, säurefreiem Dichlormethan gelöst, mit 23,6 mg (0,33 mmol) 1H-Tetrazol, 0,407 g (1,35 mmol) Bis(diisopropylamino)(2-cyanoethoxy)phosphan versetzt und unter Stickstoffatmosphäre über Nacht bei Raumtemperatur gerührt. Anschließend verdünnt man das Reaktionsgemisch mit Dichlormethan, schüttelt 2 x mit je 30 ml Natriumchlorid/Natriumhydrogencarbonat-Lösung aus, extrahiert die wäßrigen Phasen mit Dichlormethan zurück, vereinigt alle organischen Phasen, die dann über Natriumsulfat getrocknet, abfiltriert und einrotiert werden.
Das Rohprodukt wird auf eine Kieselgelsäule (3,3 x 14,0 cm) aufgetragen, mit Toluol + 0,5 % Et₃N (50 ml), Toluol/Essigester 2:1 + 0,5 % Et₃N (300 ml), 1:1 + 0,5 % Et₃N (100 ml) eluiert und man erhält nach 200 ml Laufmittelverbrauch die erste Produktfraktion. Sämtliche Produktfraktionen werden vereinigt und einrotiert. Das resultierende Öl wird, bis zur Schaumbildung, mehrmals mit Dichlormethan koevaporiert. Nach Trocknen im Hochvakuum erhält man 314,0 mg (0,33 mmol, 50 %) als gelb gefärbtem Schaum.
DC (Kieselgel): R_{f} = 0,46 + 0,55 (Toluol/Essigester 1:1)

| Analyse: C₄₉H₅₈N₅O₁₂P (940,0) | | | | | | |
|---|---|---|---|---|---|---|
| ber. | C | 62,61 | H | 62,61 | N | 7,45 |
| gef. | | 62,42 | | 6,29 | | 7,49 |

UV (Methanol): λₘₐₓ [nm] (lg ε): 202 (4,89); 234 (4,32); 265 (4,26)
¹H-NMR (250 MHz, CDCl₃, TMS, ppm):
9,39 (s, 1H, NH); 8,1 (d, 2H, o-H zu NO₂(NPEE)); 8,09-7,93 (m, 1H, H-C(6)); 7,40-7,20 (m, 11H, m-H zu OCH₃, H(Phenyl), m-H zu NO₂(NPEE)); 6,08-5,99 (m, 1H, H-C(1')); 5,29-5,25 (m, 1H, H-C(5)); 5,21-4,98 (m, 1H, CH(NPEE); 4,58-4,38 (m, 2H, H-C(2'), H-C(3')); 4,27-4,20 (m, 1H, H-C(4')); 4,09-3,44 (m, 14H, α-CH₂(NPEE), P-O-CH₂, OCH₃, CH₂(5'), N-CH); 2,98-2,90 (m, 2H, β-CH₂(NPEE)); 2,64 + 2,43 (2t, 2H, CH₂-CN)); 1,40-1,14 (m, 15H, CH₃(NPEE), C(CH₃)₂)
³¹P-NMR (161,70 MHz, CDCl₃, H₃PO₄, ppm):
151,24; 150,85; 150,75; 150,14

### 15. 5'-O-(4,4'-Dimethoxytriphenylmethyl)-2'-O-[1-(4-nitrophenylethoxy)ethyl]-uridin-3'-O-(N,N-diisopropyl-[2-(4-nitrophenyl)ethyl)-phosphitamid

0,15 g (0,202 mmol) 5'-O-(4,4'-Dimethoxytriphenylmethyl)-2'-O-[1-(4-nitrophenylethoxy)ethyl]-uridin, 9,6 mg (0,137 mmol) 1H-Tetrazol werden in einen mit Stickstoffgas gefluteten Kolben eingewogen, in 4 ml absolutem, säurefreiem Dichlormethan gelöst, mit 0,21 g (0,547 mmol) Bis(diisopropylamino)-2-(4-nitrophenylethoxy)phosphan versetzt über Nacht bei Raumtemperatur unter Stickstoffatmosphäre gerührt. Anschließend rotiert man ein und trägt das Rohprodukt zur Reinigung auf eine Kieselgelsäule (3,0 x 10,0 cm) auf. Es wird mit Toluol (250 ml), Toluol/Essigester 3:1 (200 ml), 2:1 (600 ml) chromatographiert und man erhält nach 500 ml Laufmittelverbrauch die erste Produktfraktion. Sämtliche Fraktionen die Produkt enthalten werden vereinigt und einrotiert. Den Rückstand koevaporiert man, bis zur Schaumbildung, mehrmals mit Dichlormethan. Nach Trocknen im Hochvakuum erhält man 0,125 g (0,12 mmol, 60 %) gewünschtes Produkt in Form eines farblosen Schaums.
DC (Kieselgel): R_{f} = 0,52 (Toluol/Essigester 1:1)

| Analyse: C₅₄H₆₂N₅O₁₄P (1036,09) | | | | | | |
|---|---|---|---|---|---|---|
| ber. | C | 62,60 | H | 6,03 | N | 6,75 |
| gef. | | 62,55 | | 6,15 | | 6,48 |

UV (Methanol): λₘₐₓ [nm] (Ig ε): 202 (5,0); 235 (4,39); 267 (4,43)
¹H-NMR (250 MHz, CDCl₃, TMS, ppm):
8,72 (s, 1H, NH); 8,12-7,98 (m, 5H, H-C(6), o-H zu NO₂(NPEE und NPE)); 7,41-7,21 (m, 13H, H(Phenyl), m-H zu NO₂(NPEE und NPE), m-H zu OCH₃); 6,81 (d, 4H, o-H zu OCH₃); 6,15-6,0 (m, 1H, H-C(1')); 5,27-5,18 (m, 1H, H-C(5)); 5,01-4,89 (m, 1H, CH(NPEE)); 4,37-4,03 (m, 3H, H-C(2'), H-C(3'), H-C(4')); 4,0-3,21 (m, 14H, α-CH₂(NPEE), OCH₃, P-O-CH₂, CH₂(5'), N-CH); 3,01-2,85 (m, 4H, CH₂-NO₂), β-CH₂(NPEE)); 1,30-0,99 (m, 15H, CH₃(NPEE), C(CH₃)₂)
³¹P-NMR (161,70 MHz, CDCl₃, H₃PO₄, ppm):
150,04; 149,83; 149,39; 149,28

### 16.1 N⁴-[2-(4-Nitrophenyl)ethoxycarbonyl]-2'-O-[1-(4-nitrophenylethoxy)ethyl-3',5'-O-(1,1,3,3-tetraisopropyldisiloxan-1,3-diyl)-cytidin

0,5 g (0,73 mmol) N⁴-[2-(4-Nitrophenyl)ethoxycarbonyl]-3',5'-O-(1,1,3,3-tetraisopropyldisiloxan-1,3-diyl)-cytidin werden in 8 ml absolutem Dichlormethan gelöst, mit 0,58 mg (3,05 µmol) p-TsOH·H₂O versetzt und im Eisbad auf 0°C abgekühlt. Anschließend gibt man 0,211 g (1,095 mmol) 4-Nitrophenylvinylether zu und rührt die Reaktionslösung zunächst im Kältebad und läßt dann langsam auf Raumtemperatur erwärmen. Nach 6 Stunden wird die Reaktionslösung mit 1,0 N Natriummethanolat/Methanol-Lösung neutralisiert und einrotiert.
Das Rohprodukt trägt man zur Reinigung auf eine Kieselgelsäule (3,0 x 18,0 cm) auf, eluiert mit Toluol (400 ml), Toluol/Essigester 5:1 (240 ml), 4:1 (500 ml), 3:1 (840 ml), 2:1 (500 ml), und erhält nach 1300 ml Laufmittelverbrauch die erste Produktfraktion. Sämtliche Fraktionen die Produkt enthalten werden vereinigt, einrotiert und das resultierende Öl wird mehrmals mit Dichlormethan koevaporiert. Nach Trocknen des farblosen Schaumes bei 40°C in Hochvakuum bekommt man 0,53 g (0,607 mmol, 83 %) des gewünschten Produktes.
DC (Kieselgel): R_{f} = 0,27 + 0,44 (Toluol/Essigester 1:1)

| Analyse: C₄₀H₅₇N₅O₁₃Si₂ (872,09) | | | | | | |
|---|---|---|---|---|---|---|
| ber. | C | 55,09 | H | 6,58 | N | 8,03 |
| gef. | | 54,93 | | 6,65 | | 7,83 |

UV (Methanol): λₘₐₓ[nm] (Ig ε): 211 (4,53); 247 (4,33); 276 (4,36)
¹H-NMR (250 MHz, d₆-DMSO, ppm):
10,88 (s, 1H, NH); 8,16-8,02 (m, 5H, H-C(6), o-H zu NO₂(NPEE und NPEOC), 7,61-7,48 (m, 4H, m-H zu NO₂(NPEE und NPEOC)); 6,97 (d, 1H, H-C(5)); 5,67 + 5,69 (2s, 1H, H-C(1')); 5,05-4,92 (m, 1h, CH(NPEE)); 4,37-4,33 (m, 2H, α-CH₂(NPEOC)); 4,22-3,58 (m, 7H, H-C(2'), H-C(3'), H-C(4'); α-CH₂(NPEE), CH₂(5')); 3,09-2,90 (M, 4H, β-CH₂(NPEOC), β-CH₂(NPEE)); 1,34 + 1,26 (2d, 3H, CH₃(NPEE)); 1,03-0,77 (m, 28H, Isopropyl).

### 16.2 N⁴-[2-(4-Nitrophenyl)ethoxycarbonyl]-2'-O-[1-(4-nitrophenylethoxy)ethyl-3',5'-O-(1,1,3,3-tetraisopropyldisiloxan-1,3-diyl)-cytidin

1,0 g (1,47 mmol) 2'-O-[1-(4-Nitrophenylethoxy)ethyl-3',5'-O-(1,1,3,3,-tetraisopropyldisiloxan-1,3-diyl)-cytidin (aus Beispiel 19) wird in 20 ml absolutem Dimethylformamid gelöst, mit 0,59 g (1,91 mmol) 1-Methyl-3-p-nitrophenylethoxycarbonylimidazoliumchlorid versetzt und 3 Stunden bei Raumtemperatur gerührt. Anschließend rotiert man das Dimethylformamid im Hochvakuum ab, nimmt den Rückstand in Dichlormethan auf, schüttelt 2 x mit je 50 ml Wasser und 1 x mit 50 ml gesättigter Natriumhydrogencarbonat-Lösung aus. Sämtliche wäßrigen Phasen werden mit Dichlormethan rückextrahiert, die vereinigten organischen Phasen werden über Natriumsulfat getrocknet, abfiltriert und einrotiert.
Der Rückstand wird zur Reinigung auf eine Kieselgelsäule (2,0 x 18,0 cm) aufgetragen, mit Toluol (150 ml), Toluol/Essigester 4:1 (650 ml), 2:1 (150 ml), 1:1 (100ml) chromatographiert und man erhält nach 500 ml Laufmittelverbrauch die erste Produktfraktion. Alle produkthaltigen Fraktionen werden vereinigt, einrotiert, das verbleibende Öl wird mehrere Male mit Dichlormethan koevaporiert und man trocknet den resultierenden farblosen Schaum anschließend bei 40°C im Hochvakuum. Es kann 1,23 g (1,41 mmol, 95 %) Verbindung isoliert werden.
DC (Kieselgel): R_{f} = 0,27 + 0,44 (Toluol/Essigester 1:1)
C₄₀H₅₇N₅O₁₃Si₂ (872,09)
UV (Methanol): λₘₐₓ[nm] (Ig ε): 211 (4,53); 247 (4,33); 276 (4,36)
¹H-NMR (250 MHz, CDCl₃, TMS, ppm):
10,88 (s, 1H, NH); 8,16-8,02 (m, 5H, H-C(6), o-H zu NO₂(NPEE und NPEOC), 7,61-7,48 (m, 4H, m-H zu NO₂(NPEE und NPEOC)); 6,97 (d, 1H, H-C(5)); 5,67 + 5,69 (2s, 1H, H-C(1')); 5,05-4,92 (m, 1h, CH(NPEE)); 4,37-4,33 (m, 2H, α-CH₂(NPEOC)); 4,22-3,58 (m, 7H, H-C(2'), H-C(3'), H-C(4'); α-CH₂(NPEE), CH₂(5')); 3,09-2,90 (M, 4H, β-CH₂(NPEOC), β-CH₂(NPEE)); 1,34 + 1,26 (2d, 3H, CH₃(NPEE)); 1,03-0,77 (m, 28H, Isopropyl).

### 16.3 N⁴-[2-(4-Nitrophenyl)ethoxycarbonyl]-2'-O-[1-(4-nitrophenylethoxy)ethyl]-cytidin

2,0 g (2,95 mmol) N⁴-[2-(4-Nitrophenyl)ethoxycarbonyl]-3',5'-O-(1,1,3,3-tetraisopropyldisiloxan-1,3-diyl)-cytidin werden in 30 ml absolutem Dichlormethan gelöst, mit 2,36 mg (12,4 µmol) 4-Toluolsulfonsäure x H₂O versetzt und im Eisbad auf 0°C abgekühlt. Zu diesem Gemisch gibt man 0,85 g (4,42 mmol) 4-Nitrophenylethylvinylether, rührt die Reaktionsmischung zunächst im Kältebad und läßt anschließend langsam auf Raumtemperatur erwärmen. Nach 9 Stunden wird das Gemisch mit 1,0 N Natriummethanolat-/Methanol-Lösung neutralisiert und einrotiert. Das zurückbleibende Öl löst man in 7,0 ml absolutem Tetrahydrofuran, versetzt mit 1,95 g (6,19 mmol) Tetrabutylammoniumfluorid x 3 H₂O 0,5 ml Eisessig, rührt 4 Stunden bei Raumtemperatur und rotiert anschließend ein.
Der Rückstand wird zur Reinigung auf eine Kieselgelsäule (3,5 x 15,0 cm) aufgetragen, mit Dichlormethan (400 ml), Dichlormethan/Methanol 90:1 (270 ml), 50:1 (250 ml), 30:1 (500 ml) chromatographiert, wobei man nach 1100 ml Laufmittelverbrauch die erste Produktfraktionen erhalten kann. Sämtliche Fraktionen die Produkt enthalten werden vereinigt und einrotiert. Den Rückstand löst man in 5 ml Dichlormethan, tropft in 150 ml Diethylether ein, saugt den erhaltenen Niederschlag ab und trocknet ihn bei 40°C im Hochvakuum. Man erhält 1,25 g (1,98 mmol, 68 %) gewünschtes Produkt in Form eines farblosen, amorphen Feststoffs.
DC (Kieselgel): R_{f} = 0,38 + 0,48 (Dichlormethan/Methanol 95:5)

| Analyse: C₂₈H₃₁N₅O₁₂ (629,58) | | | | | | |
|---|---|---|---|---|---|---|
| ber. | C | 53,41 | H | 4,96 | N | 11,12 |
| gef. | | 53,46 | | 5,13 | | 10,89 |

UV (Methanol): λₘₐₓ[nm] (lg ε): 212 (4,49); 2,47 (4,32); 275 (4,34)
¹H-NMR (250 MHz, d₆-DMSO, ppm):
10,79 (s, 1H, NH); 8,41-8,35 (m, 1H, H-C(6)); 8,15-8,05 (m, 4H, o-H zu NO₂(NPEOC und NPE)); 7,60-7,42 (m, 4H, m-H zu NO₂(NPEOC und NPEE)); 6,95 (d, 1H, H-C(5)); 5,87-5,79 (2d, 1H, H-C(1')); 5,24-5,07 (m, 2H, HO-C(5'), HO-C(3')); 4,96 + (2q, 1H, CH(NPEE)); 4,75 (t, 2H, α-CH₂(NPEOC)); 4,10-3,67 4,90 (m, 7H, H-C(2'), H-C(3'), H-C(4'), CH₂(5'), α-CH₂(NPEE)); 3,07 (t, 2H, β-CH₂(NPEOC)); 1,25 + 1,19 (2d, 3H, CH₃(NPEE)

### 16.4 N⁴-[2-(4-Nitrophenyl)ethoxycarbonyl]-2'-O-[1-(4-Nitrophenylethoxy)ethyl]-cytidin

2,78 g (3,18 mmol) N⁴-[2-(4-Nitrophenyl)ethoxycarbonyl]-2'-O-[1-(4-nitrophenylethoxy)ethyl]-3',5'-O-(1,1,3,3-tetraisopropyldisiloxan-1,3-diyl)-cytidin löst man in 8,0 ml absolutem Tetrahydrofuran, versetzt mit 2,51 g (7,97 mmol) Tetrabutylammoniumfluorid x 3 H₂O, 0,6 ml Eisessig, rührt die Reaktionslösung 4 Stunden bei Raumtemperatur und rotiert anschließend ein. Das resultierende Öl wird zur Reinigung auf eine Kieselgelsäule (3,0 x 15,0 cm) aufgetragen, wobei man mit Dichlormethan (300 ml), Dichlormethan/Methanol 95:1 (380 ml), 90:1 (360 ml), 80:1 (240 ml), 70:1 (330 ml), 60:1 (900 ml), 55:1 (660 ml) eluiert und nach 1300 ml Laufmittelverbrauch die erste produkthaltige Fraktion erhält. Sämtliche Fraktionen welche Produkt aufweisen werden vereinigt, einrotiert, der resultierende Rückstand wird, bis zur Schaumbildung, mehrmals mit Dichlormethan koevaporiert. Nach Trocknen des farblosen Schaumes bei 40°C im Hochvakuum erhält man 1,95 g (3,09 mmol, 97 %) gewünschtes Produkt.
DC (Kieselgel): R_{f} = 0,38 + 0,48 (Dichlormethan/Methanol (95:5)
C₂₈H₃₁N₅O₁₂ (629,58)
Spektroskopische Daten sind identisch mit Beispiel 16.3.

### 16.5 N⁴-[2-(4-Nitrophenyl)ethoxycarbonyl]-2'-O-[1-(4-nitrophenylethoxy)ethyl]-cytidin

0,5 g (1,14 mmol) 2'-O-[1-(4-Nitrophenylethoxy)ethyl]-cytidin und 0,46 g (1,49 mmol) 1-Methyl-3-p-nitrophenylethoxycarbonylimidazoliumchlorid werden in 10 ml absolutem Dimethylformamid gelöst und 3 Stunden bei Raumtemperatur gerührt. Das Dimethylformamid rotiert man anschließend im Hochvakuum ab, nimmt den Rückstand in Dichlormethan auf, wäscht 2 x mit je 30 ml Wasser und 1 mal mit 30 ml gesättigter Natriumhydrogencarbonat-Lösung. Alle wäßrigen Phasen werden mit Dichlormethan rückextrahiert, die vereinigten organischen Phasen trocknet man über Natriumsulfat, filtriert ab und rotiert ein. Zur Reinigung trägt man den Rückstand auf eine Kieselgelsäule (3,0 x 17,0 cm) auf, eluiert mit Dichlormethan (200 ml), Dichlormethan/Methanol 98:1 (100 ml), 96:1 (95 ml), 94:1 (190 ml), 92:1 (90 ml), 90:1 (360 ml), 85:1 (340 ml), 80:1 (160 ml), 76:1 (75 ml), 70:1 (70 ml), 60:1 (60 ml), 50:1 (100 ml), 40.1 (80 ml), 30:1 (180 ml), 25:1 (250 ml) und erhält nach 1200 ml Laufmittelverbrauch die erste Produktfraktion. Sämtliche Fraktionen die Produkt enthalten werden vereinigt, einrotiert, der erhaltene Rückstand mehrmals, bis zur Schaumbildung, mit Dichlormethan koevaporiert. Nach Trocknen des farblosen Schaumes bei 40°C im Hochvakuum, erhält man 0,52 g (0,82 mmol, 73 %) von Verbindung.
DC (Kieselgel): R_{f} = 0,25 + 0,33 (Dichlormethan/Methanol 95:5)
C₂₈H₃₁N₅O₁₂ (629,58)
Spektroskopische Daten sind identisch mit Beispiel 16.3.

### 16.6 5'-O-(4,4'Dimethoxytriphenylmethyl)-N⁴-[2-(4-nitrophenyl)ethoxycarbonyl]-2'-O-[1-(4-nitrophenylethoxy)ethyl]-cytidin

1,95 g (3,09 mmol) N⁴-[2-(4-nitrophenyl)ethoxycarbonyl]-2'-O-[1-(4-nitrophenylethoxy)ethyl]-cytidin koevaporiert man 3 x mit je 10 ml absolutem Pyridin, löst den Rückstand in 60 ml absolutem Pyridin, versetzt mit 1,36 g (4,02 mmol) 4,4'-Dimethoxytriphenylmethylchlorid und rührt die Reaktionslösung bei Raumtemperatur über Nacht. Es wird anschließend 10 ml Methanol zugegeben, weitere 30 Minuten bei Raumtemperatur gerührt, einrotiert und der Rückstand mehrmals mit Toluol koevaporiert. Das Rohprodukt wird zur Reinigung auf eine Kieselgelsäule (3,0 x 12,0 cm) aufgetragen, mit Toluol (400 ml), Toluol/Essigester (3:1 (400 ml), 2:1 (1200 ml) eluiert und man erhält nach 850 ml Laufmittelverbrauch die erste Produktfraktion. Sämtliche Fraktionen die Produkt enthalten werden vereinigt und einrotiert. Den Rückstand löst man in 2 ml Dichlormethan, tropft in 250 ml Diethylether ein, saugt den ausgefallenen Niederschlag ab und trocknet ihn bei 40°C im Hochvakuum. Von Verbindung erhält man 2,61 g (2,80 mmol, 91 %) in Form eines farblosen, amorphen Feststoffs.
DC (Kieselgel): R_{f} = 0,48 (Toluol/Essigester/Methanol 5:4:1)

| Analyse: C₄₉H₅₁N₅O₁₅xH₂O (949,97) | | | | | | |
|---|---|---|---|---|---|---|
| ber. | C | 61,95 | H | 5,19 | N | 7,37 |
| gef. | | 61,42 | | 5,35 | | 7,09 |

UV (Methanol): λₘₐₓ [nm] (Ig e): 211 (4,72); 236 (4,55); 274 (4,40)
¹H-NMR (250 MHz, d₆-DMSO, ppm):
10,71 (s, 1H, NH); 8,28-8,22 (m, 1H, H-C(6)); 8,16-8,06 (m, 4H, o-H zu NO₂(NPEOC und NPEE)); 7,60-7,22 (m, 13H, m-H zu NO₂(NPEOC und NPEE), H(Phenyl), m-H zu OCH₃)); 6,90-6,87 (m, 4H zu OCH₃); 6,78-6,71 (m, 1H, H-C(5)); 5,75+5,74 (2s, 1H, H-C(1')); 5,41+5,19 (2d, 1H, HO-C(3')); 5,09+4,95 (2q, 1H, CH(NPEE)); 4,34 (t, 2H, α-CH₂(NPEOC)); 4,28-3,63 (m, 13H, H-C(2'), H-C(3'), H-C(4'), α-CH₂(NPEE), OCH₃, CH₂(5')); 3,07 (t, 2H, β-CH₂(NPEOC)); 2,92 (m, 2H, β-CH₂(NPEE)); 1,28+1,22 (2d, 3H, CH₃(NPEE))

### 17. 5'-O-(4,4'-Dimethoxytriphenylmethyl)-N⁴-[2-(4-nitrophenyl)ethoxycarbonyl]-2'-O-[1-(4-nitrophenylethoxy)ethyl)-cytidin-3'-O-(N,N-diisopropyl-2-cyanoethyl)-phosphitamid

0,4 g (0,43 mmol) 5'-0-(4,4'-Dimethoxytriphenyl)ethoxycarbonyl-N4-[2-(4-nitrophenyl)ethoxycarbonyl]-2'-O-[1-(4-nitrophenylethoxy)ethyl]-cytidin, 18,0 mg (0,25 mmol) 1H-Tetrazol wiegt man in einen mit Stickstoffgas gefluteten Kolben ein und löst die Reaktanden in 8 ml absolutem Acetonitril (Rückstandsanalyse). Anschließend werden 0,31 g (1,03 mmol) Bis(diisopropylamino)(2-cyanoethoxy)phosphan zugegeben und über Nacht, unter Stickstoffatmosphäre, bei Raumtemperatur gerührt. Danach verdünnt man die Reaktionslösung mit Essigester, schüttelt 3 x mit je 40 ml Natriumchlorid/Natriumhydrogencarbonat-Lösung aus, extrahiert die wäßrigen Phasen mit Essigester zurück, vereinigt sämtliche organischen Phasen, die anschließend über Natriumsulfat getrocknet, abfiltriert und einrotiert werden. Den öligen Rückstand trägt man zur Reinigung auf eine Kieselgelsäure (2,5 x 20,0 cm) auf, eluiert mit Toluol (100 ml), Toluol/Essigester 3:1 (200 ml), 2:1 (750 ml), 1:1 (200 ml) und erhält nach 450 ml Laufmittelverbrauch die erste Produktfraktion. Alle produkthaltigen Fraktionen werden vereinigt, einrotiert, der Rückstand mehrmals mit Essigester bis zur Schaumbildung koevaporiert und anschließend im Hochvakuum getrocknet. Man erhält 0,42 g (0,37 mmol, 86 %) Verbindung in Form eines farblosen Schaumes.
DC (Kieselgel): R_{f} = 0,73 + 0,76 (Toluol/Essigester/Methanol 5:4:1)

| Analyse: C₅₈H₆₆N₇O₁₅P (1132,18) | | | | | | |
|---|---|---|---|---|---|---|
| ber. | C | 61,53 | H | 5,87 | N | 8,65 |
| gef. | | 60,88 | | 6,24 | | 8,50 |

UV (Methanol): λₘₐₓ [nm] (Ig ε): 203 (4,91); [214 (4,66)]; 235 (4,48; 275 (4,33)
¹H-NMR (250 MHz, CDCl₃, TMS, ppm):
8,58-8,49 (m, 1H, H-C(6)); 8,20-8,06 (m, 5H-o-H zu NO₂(NPEE und NPEOC), NH); 7,42-7,21 (m, 13H, m-H zu NO₂(NPEE und NPEOC), H(Phenyl), m-H zu OCH₃); 6,87-6,58 (m, 5H, o-H zu OCH₃, H-C(5)); 6,10-5,91 (m, 1H-H-C(1')); 5,29-5,12 (m, 1H, CH(NPEE)); 4,49-4,39 (m, 2H, α-CH₂(NPEOC)); 4,32-4,10 (m, 3H, H-C(2'), H-C(3'), H-C(4')); 4,0-3,78 (m, 8H, α-CH₂(NPEE), OCH₃); 3,70-3,40 (m, 8H, P-O-CH₂, CH₂(5'), N-CH, β-CH₂(NPEOC)); 3,18-2,90 (m, 4H, CH₂-CN, β-CH₂(NPEE)); 1,33-0,92 (m, 15H, CH₃(NPEE), C(CH₃)₂)
³¹P-NMR (161,70 MHz, CDCl₃, H₃PO₄, ppm):
151,63; 150,34; 150,22; 149,54

### 18. 5'-O-(4,4'-Dimethoxytriphenylmethyl)-N⁴-[2-(4-nitrophenyl)ethoxycarbonyl]-2'-O-[1-(4-nitrophenylethoxy)ethyl]-cytidin-3'-O-(N,N-diisopropyl-[2-(4-nitrophenyl)ethyl)-phosphitamid

Man wiegt 0,4 g (0,43 mmol) 5'-O-(4,4'-Dimethoxytriphenylmethyl)-N⁴-[2-(4-nitrophenyl)ethoxycarbonyl]-2'-0-[1-(4-nitrophenylethoxy)ethyl]-cytidin, 20,31 mg (0,29 mmol) 1H-Tetrazol in einen mit Stickstoffgas gefluteten Kolben ein, löst in 9 ml absolutem Acetonitril (Rückstandsanalyse), gibt 0,46 g (1,5 mmol) Bis(diisopropylamino)-2-(4-nitrophenylethoxy)phosphan zu und rührt die Reaktionslösung unter Schutzgasatmosphäre über Nacht bei Raumtemperatur. Danach verdünnt man mit Essigester, wäscht 3 x mit je 40 ml Natriumchlorid/Natriumhydrogencarbonat-Lösung, extrahiert die wäßrigen Phasen mit Essigester zurück, vereinigt alle organischen Phasen, trocknet sie anschließend über Natriumsulfat, filtriert ab und rotiert ein. Das resultierende Öl wird zur Reinigung auf eine Kieselgelsäule (2,5 x 19,0 cm) aufgetragen, mit Toluol (100 ml), Toluol/Essigester 4:1 (250 ml), 3.1 (1200 ml) eluiert, wobei man nach 400 ml Laufmittelverbrauch die erste Produktfraktion auffangen kann. Sämtliche Fraktionen die Produkt enthalten werden vereinigt, einrotiert und der Rückstand mit Essigester bis zur Schaumbildung koevaporiert. Nach Trocknen im Hochvakuum erhält man 0,48 g (0,39 mmol, 91 %) in Form eines farblosen Schaumes.
DC (Kiesegel): R_{f} = 0,19 + 0,32 + 0,38 (Toluol/Essigester/Methanol 5:4:1)

| Analyse: C₆₃H₇₀N₇O₁₂P (1228,27) | | | | | | |
|---|---|---|---|---|---|---|
| ber. | C | 61,60 | H | 5,74 | N | 7,98 |
| gef. | | 61,31 | | 5,93 | | 7,75 |

UV (Methanol): λₘₐₓ [nm] (lg ε): 204 (5,0); [211 (4,85)]; 236 (4,57); 274 (4,57)
¹H-NMR (250 MHz, CDCl₃, TMS, ppm):
8,52-8,41 (m, 1H, H-C(6)); 8,20-7,97 (m, 7H, o-H zu NO₂(NPEOC, NPEE und NPE), NH); 7,43-7,19 (m, 15H, m-H zu NO₂(NPEOC, NPEE und NPE), H(Phenyl), m-H zu OCH₃); 6,84-6,55 (m, 5H, o-H zu OCH₃, H-C(5)); 6,02-5,90 (m, 1H, H-C(1')); 5,21-5,12 (m, 1H, CH(NPEE)); 4,44-4,42 (m, 2H, α-CH₂(NPEOC)); 4,25-4,13 (m, 3H, H-C(2'), H-C(3'), H-C(4')); 3,92-3,61 (m, 10H, P-O-CH₂, α-CH₂(NPEE), OCH₃); 3,51-3,39 (m, 4H, CH₂(5'), N-CH); 3,15-2,81 (m, 6H, β-CH₂(NPEE)); 1,34-1,02 (m, 15H, CH₃(NPEE), C(CH₃)₂)
³¹P-NMR (161,70 MHz, CDCl₃, H₃PO₄, ppm):
150,28; 149,21; 148,97; 148,35

### 19.1 2'-O-[1-(4-Nitrophenylethoxy)ethyl]-3',5'-O-(1,1,3,3-tetraisopropyldisiloxan-1,3-diyl)-cytidin

2,0 g (3,79 mmol) N⁴-Acetyl-3',5'-O-(1,1,3,3-tetraisopropyldisiloxan-1,3-diyl)-cytidin werden in 40 ml absolutem Dichlormethan gelöst, mit 0,31 g (1,64 mmol) p-TsOH·H₂O, 1,09 g (5,68 mmol) 4-Nitrophenylvinylether versetzt, bei Raumtemperatur 2 1/2 Stunden gerührt und anschließend mit 9,5 N Natriummethanolat/Methanol-Lösung neutralisiert und einrotiert. Man nimmt den Rückstand in Dichlormethan auf, schüttelt 3 x mit je 70 ml gesättigter Natriumhydrogencarbonat-Lösung aus, extrahiert alle wäßrigen Phasen mit Dichlormethan zurück und vereinigt anschließend alle organischen Phasen, welche dann über Natriumsulfat getrocknet, abfiltriert und einrotiert werden. Zur Reinigung trägt man das Rohprodukt auf eine Kieselgelsäule (3,5 x 21,0) auf. Es wird mit Toluol (200 ml), Toluol/Essigester 1:1 (200 ml), 1:2 (600 ml) 1:3 (600 ml), 1:4 (250 ml), 1:5 (480 ml), 1:8 (180 ml) eluiert und man erhält die erste Fraktion, welche Produkt enthält, nach 1500 ml Laufmittelverbrauch, Sämtliche Produktfraktionen werden vereinigt und einrotiert. Das verbleibende Öl koevaporiert man mehrmals mit Dichlormethan und der resultierende farblose Schaum wird anschließend bei 40°C im Hochvakuum getrocknet. Von der Verbindung kann 1,64 g (2,41 mmol, 64 %) erhalten werden.
DC (Kieselgel): R_{f} = 0,51 (Toluol/Essigester/Methanol 5:4:1)

| Analyse: C₃₁H₅₀N₄O₉Si₂ (678,93) | | | | | | |
|---|---|---|---|---|---|---|
| ber. | C | 54,84 | H | 7,42 | N | 8,25 |
| gef. | | 54,76 | | 7,35 | | 8,05 |

UV (Methanol): λₘₐₓ[nm] (Ig ε): 271 (4,27)
¹H-NMR (250 MHz, d₆-DMSO, ppm):
8,19-8,08 (m, 2H, o-H zu NO₂(NPEE)); 7,71-7,57 (m, 1H, H-C(6)); 7,54-7,48 (m, 2H, m-H zu NO₂(NPEE); 7,22 (s, 2H, NH₂); 5,06 + 5,04 (2q, 1H, CH(NPEE)); 4,19-3,60 (m, 7H, H-C(2'), H-C(3'), α-CH₂(NPEE), H-C(4'), CH₂(5')); 2,93-2,9 (m, 2H, β-CH₂(NPEE)); 1,32 + 1,25 (2d, 3H, CH₃(NPEE)); 1,02-0,78 (m, 28 H, Isopropyl)

### 19.2 2'-O-[1-(4-Nitrophenylethoxy)ethyl]-cytidin

Man löst 2,0 g (3,79 mmol) N⁴-Acetyl-3',5'-O-(1,1,3,3-tetraisopropyldisiloxan-1,3-diyl)-cytidin in 40 ml absolutem Dichlormethan, versetzt die Reaktionslösung mit 0,31 g (1,64 mmol) p-TsOH·H₂O 1,09 g (5,68 mmol) 4-Nitrophenylethylvinylether, rührt das Gemisch 2 1/2 Stunden bei Raumtemperatur, neutralisiert mit 0,5 N Natriumethanolat/Methanol-Lösung und rotiert anschließend ein. Der Rückstand wird in Dichlormethan aufgenommen und 3 x mit je 70 ml gesättigter Natriumhydrogencarbonat-Lösung ausgeschüttelt. Alle wäßrigen Phasen extrahiert man mit Dichlormethan zurück, vereinigt sämtliche organischen Phasen, trocknet sie über Natriumsulfat, filtriert ab und rotiert ein. Der verbleibende Rückstand wird in 3,0 ml absolutem Tetrahdyrofuran aufgenommen, mit 2,87 g (9,09 mmol) Tetrabutylammoniumfluorid x 3 H₂O versetzt, bei Raumtemperatur 20 Minuten gerührt und anschließend einrotiert.
Zur Reinigung trägt man das erhaltene Öl auf eine Kieselgelsäule (3,7 x 17,0 cm) auf, eluiert mit Dichlormethan (200 ml), Dichlormethan/Methanol 90:1 (270 ml), 80:1 (160 ml), 70:1 (140 ml), 50:1 (100 ml), 40:1 (80 ml), 20:1 (150 ml), 10:1 (440 ml), 9:1 (100 ml), und erhält nach 1000 ml Laufmittelverbrauch die erste produkthaltige Fraktion. Sämtliche Produktfraktionen werden vereinigt, einrotiert und der Rückstand wird aus 100 ml Dichlormethan umkristallisiert. Den ausgefallenen Feststoff saugt man ab und trocknet ihn anschließend bei 40°C im Hochvakuum. Man erhält 1,03 g (2,36 mmol, 62 %) farbloses, kristallines Produkt, das trotz Umkristallisation immer noch mit Tetrabutylammoniumfluorid verunreinigt ist.
Schmelzpunkt: 145°C
DC (Kieselgel): R_{f} = 0,34 + 0,4 (Chloroform/Methanol 1:1)
UV (Methanol): λₘₐₓ[nm] (Ig ε): 271 (4,16)
¹H-NMR (250 MHz, d₆-DMSO, ppm):
8,10 (2d, 2H, o-H zu NO₂(NPEE)), 7,88 + 7,85 (2d, 1H, H-C(6)); 7,49 (2d, 2H, m-H zu NO₂(NPEE)); 7,28 + 7,20 (2s, 2H, NH₂); 5,89 + 5,82 (2d, 1H, H-C(1')); 5,72 + 5,70 (2d, 1H, H-C(5)); 5,20-4,99 (m, 2H, HO-C(5'), HO-C(3')); 4,35 (m, 1H, CH(NPEE)); 4,08-3,48 (m, 7H, H-C(2'), H-C(3'), H-C(4'), CH₂(5'), α-CH₂(NPEE)); 2,95-2,78 (m, 2H, β-CH₂(NPEE); 1,18 + 1,14 (2d, 3H, CH₃(NPEE));

### 19.3 N⁴-Acetyl-2'-O-[1-(4-nitrophenylethoxy)ethyl]-cytidin

3,3 g (6,25 mmol) N⁴-Acetyl-3',5'-O-(1,1,3,3-tetradiisopropyldisiloxan-1,3-diyl)-cytidin werden in 60 ml absolutem Dichlormethan gelöst, im Eis/Kochsalz-Bad auf 0°C abgekühlt, mit 0,51 g (2,70 mmol) p-TsOH·H₂O, 1,80 g (9,37 mmol) 4-Nitrophenylethylvinylether versetzt, 2 Stunden im Kältebad gerührt und anschließend in einem Scheidetrichter 3 x mit je 70 ml gesättigter Natriumhydrogencarbonat-Lösung ausgeschüttelt. Die wäßrigen Phasen extrahiert man mit Dichlormethan zurück, sämtliche organischen Phasen werden vereinigt, über Natriumsulfat getrocknet, abfiltriert und einrotiert. Der Rückstand wird in 14,0 ml absolutem Tetrahydrofuran gelöst, mit 4,3 g (13,63 mmol) Tetrabutylammoniumfluorid x 3 H₂O versetzt, 30 Minuten bei Raumtemperatur gerührt und anschließend einrotiert.
Zur Reinigung trägt man das verbleibende Öl auf eine Kieselgelsäule (4,0 x 13,0 cm) auf, chromatographiert mit Dichlormethan (600 ml), Dichlormethan/Methanol 95:1 (570 ml), 90:1 (810 ml), 85:1 (570 ml), 80:1 (480 ml), 70:1 (560 ml), 60:1 (480 ml), 55:1 (280 ml), 50:1 (500 ml), 45:1 (640 ml), 40:1 (980 ml) und erhält nach 4000 ml Laufmittelverbrauch die erste Produktfraktion. Sämtliche produkthaltigen Fraktionen werden vereinigt und einrotiert. Man löst den Rückstand in 3 ml Dichlormethan, tropft in 300 ml Diethylether ein, saugt den erhaltenen Niederschlag ab und trocknet bei 40°C im Hochvakuum. Vom Produkt werden 2,62 g (5,47 mmol, 88 %) in Form eines farblosen, amorphen Feststoffs erhalten.
Schmelzpunkt: 157°C
DC (Kieselgel): R_{f} = 0,56 + 0,63 (Dichlormethan/Methanol 9:1)

| Analyse: C₂₁H₂₆N₄O₉ (478,46) | | | | | | |
|---|---|---|---|---|---|---|
| ber. | C | 52,71 | H | 5,47 | N | 11,70 |
| gef. | | 52,70 | | 5,59 | | 11,09 |

UV (Methanol): λₘₐₓ [nm] (Ig ε): 212 (4,35); 250 (4,26); 279 (4,08)
¹H-NMR (250 MHz, d₆-DMSO, ppm):
10,89 (s, 1H, NH); 8,41 (t, 1H, H-C(6)); 8,12-8,02 (m, 2H, o-H zu NO₂(NPEE)); 7,47 (t, 2H, m-H zu NO₂(NPEE)); 7,16 (d, 1H, H-C(5)); 5,88 + 5,79 (2d, 1H, H-C(1')); 5,18 (m, HO-C(5')); 5,08 (m, 1H, HO-C(3')); 4,99 + 4,91 (2q, 1H, CH(NPEE)); 4,10-3,57 (m, 7H, H-C(2'), H-C(3'), H-C(4'), CH₂(5'), α-CH₂(NPEE)); 2,92-2,83 (m, 2H, β-CH₂(NPEE)); 2,08 (s, 3H, CH₃(Acetyl)); 1,25 + 1,20 (2d, 3H, CH₃(NPEE));

### 19.4 N⁴-Acetyl-5'-O-(4,4'-dimethoxytriphenylmethyl)-2'-O-[1-(4-nitrophenylethoxy)ethyl]-cytidin

1,0 g (2,09 mmol) N⁴-Acetyl-2'-O-[1-(4-nitrophenylethoxy)ethyl]-cytidin werden 3 x mit je 10 ml absolutem Pyridin koevaporiert, der Rückstand wird in 40 ml absolutem Pyridin aufgenommen, mit 0,77 g (2,3 mmol), 4,4'-Dimethoxytriphenylmethylchlorid versetzt und bei Raumtemperatur über Nacht gerührt. Das Reaktionsgemisch wird dann mit 10 ml Methanol verdünnt, weitere 30 Minuten bei Raumtemperatur gerührt, auf die Hälfte eingeengt, mit Dichlormethan aufgenommen und 2 x mit je 50 ml Wasser ausgeschüttelt. Die wäßrigen Phasen extrahiert man mit Dichlormethan zurück, alle organischen Phasen werden vereinigt, über Natriumsulfat getrocknet, abfiltriert, einrotiert und der Rückstand mehrere Male mit Toluol koevaporiert.
Das verbleibende Öl wird zur Reinigung auf eine Kieselgelsäule (3,2 x 23,0 cm) aufgetragen, mit Dichlormethan (100 ml), 100:1 (200 ml), 98:1 (200 ml), 95:1 (480 ml), 90:1 (270 ml), 85:1 (250 ml), 80:1 (240 ml), 70:1 (140 ml) chromatographiert und man erhält nach 1200 ml Laufmittelverbrauch die erste Produkfraktion. Sämtliche produkthaltigen Fraktionen werden vereinigt und einrotiert. Das Produkt löst man in 2 ml Dichlormethan, tropft man in 250 ml Diethylether ein, saugt den erhaltenen Niederschlag ab und trocknet ihn bei 40°C im Hochvakuum. Von Verbindung werden 1,29 g (1,65 mmol, 80 %) als farbloser, amorpher Feststoff erhalten.
Schmelzpunkt: 160°C
DC (Kieselgel): R_{f} = 0,48 (Dichlormethan/Methanol 15:1)

| Analyse: C₄₂H₄₄N₄O₁₁ (780,83) | | | | | | |
|---|---|---|---|---|---|---|
| ber. | C | 64,60 | H | 5,67 | N | 7,17 |
| gef. | | 64,85 | | 6,00 | | 6,87 |

UV (Methanol) λₘₐₓ [nm] (Ig ε): 204 (4,84); 236 (4,48); 274 (4,17), [296 (4,06)]
¹H-NMR (250 MHz, d₆-DMSO, ppm):
10,90 (s, 1H, NH); 8,34-8,24 (m, 1H, H-C(6)); 8,09'(d, 2H-o-H zu NO₂(NPEE)); 7,52-7,23 (m, 11H, m-H zu NO₂(NPEE), H(Phenyl), m-H zu OCH₃); 6,98-6,95 (m, 1H, H-C(5)); 6,90-6,87 (m- 4H, o-H zu OCH₃); 5,88 + 5,76 (2s, 1H, H-C(1')); 5,25-5.15 (m, 1H, HO-C(3')); 5,08 + 4,95 (2q, 1H, CH(NPEE)); 4,27-3,97 (m, 3H, H-C(2'), H-C(3'), H-C(4')); 3,89-3,68 (m, 10H, CH₂(5'), α-CH₂(NPEE), OCH₃); 2,92 (m, 2H, β-CH₂(NPEE)); 2,08 (s, 3H, CH₃(Acetyl)); 1,29-1,22 (m, 3H, CH₃(NPEE))

### 20.1 N⁶-[2-(4-Nitrophenyl)ethoxycarbonyl]-2'-O-[1-(4-nitrophenylethoxy)ethyl]3',5'-O-(1,1,3,3-tetraisopropyldisiloxan-1,3-diyl)-adenosin

0,5 g (0,711 mmol) N⁶-[2-(4-Nitrophenyl)ethoxycarbonyl]-3',5'-O-(1,1,3,3-tetraisopropyldisiloxan-1,3-diyl)-adenosin werden in 9 ml absolutem Dichlormethan gelöst, mit 9,56 mg (2,94 µmol) p-TsOH·H₂O versetzt und im Eisbad auf 0°C abgekühlt. Anschließend gibt man 0,20 g (1,06 mmol) 4-Nitrophenylethylvinylether zu, rührt die Reaktionslösung zunächst im Eisbad, läßt anschließend auf Raumtemperatur erwärmen und rührt über Nacht. Es wird 3 x mit je 50 ml gesättigter Natriumhydrogencarbonat-Lösung ausgeschüttelt. Die wäßrigen Phasen extrahiert man mit Dichlormethan zurück, alle organischen Phasen werden vereinigt, über Natriumsulfat getrocknet, abfiltriert und einrotiert.
Zur Reinigung trägt man das Rohprodukt auf eine Kieselgelsäule (3,5 x 14,0 cm) auf, chromatographiert mit Toluol (500 ml), Toluol/Essigester 5:1 (300 ml), 4:1 (500 ml), 3:1 (800 ml), 2:1 (500 ml) erhält nach 1400 ml Laufmittelverbrauch die erste Produktfraktion, die in der Folge aufgefangen und einrotiert werden. Der Rückstand wird, bis zur Schaumbildung, mehrmals mit Dichlormethan koevaporiert. Vom gewünschten Produkt erhält man nach Trocknen des farblosen Schaumes bei 40°C im Hochvakuum 0,412 g (0,46 mmol, 65 %).
DC (Kieselgel): R_{f} = 0,39 (Toluol/Essigester 1:1)

| Analyse: C₄₁H₅₇N₇O₁₂Si₂ (896,12) | | | | | | |
|---|---|---|---|---|---|---|
| ber. | C | 54,95 | H | 6.41 | N | 10,94 |
| gef. | | 54,71 | | 6,48 | | 10,62 |

UV (Methanol): λₘₐₓ [nm] (Ig ε): 203 (4,93); 267 (4,58); [276 (4,51)]
¹H-NMR (250 MHz, CDCl₃, TMS, ppm):
8,69 + 8.61 (2s, 1H, H-C(8)); 8,30 + 8,22 (2s, 1H, H-C(2)); 8,21 (s, 1H, NH); 8,15 (d, 2H, o-H zu NO₂(NPEOC)); 8,05 + 7,99 (2d, 2H, o-H zu NO₂(NPEE); 7,43-7,29 (m, 4H, m-H zu NO₂(NPEOC und NPEE)); 6,02 + 6,0 (2s, 1H, H-C(1')); 5,06 + 5,01 (2q, 1H, CH(NPEE)); 4,57-3,68 (m, 9H, H-C(2'), α-CH₂(NPEOC), H-C(3'), α-CH₂(NPEE), H-C(4'), CH₂(5')); 3,13 (t, 2H, β-CH₂(NPEOC)); 2,98-2,90 (m, 2H, β-CH₂(NPEE)); 1,45 + 1,38 (2d, 3H, CH₃(NPEE)); 1,07-0,84 (m, 28H, Isopropyl)

### 20.2 N⁶-[2-(4-Nitrophenyl)ethoxycarbonyl]-2'-O-[(1-(4-nitrophenylethoxy)ethyl]-adenosin

0,3 g (0,334 mmol) N⁶-[2-(4-Nitrophenyl)ethoxycarbonyl]-3',5'-O-(1,1,3,3-tetraisopropyldisiloxan-1,3-diyl)-2'-O-(1-p-nitrophenylethoxy)ethyl-adenosin werden in 1,0 ml absolutem Tetrahydrofuran gelöst, mit 0,26 g (0,836 mmol) Tetrabutylammoniumfluorid x 3 H₂O, 0,09 ml (1,67 mmol) Eisessig versetzt, 4 Stunden bei Raumtemperatur gerührt und anschließend einrotiert. Zur Reinigung trägt man den Rückstand auf eine Kieselgelsäule (3,0 x 12,0 cm) auf, eluiert mit Dichlormethan (400 ml), Dichlormethan/Methanol 95:1 (670 ml), 90:1 (360 ml), 85:1 (340 ml), 80:1 (240 ml), 75:1 (750 ml), 70:1 (700 ml), 65:1 (650 ml), 60:1 (610 ml) und erhält nach 2200 ml Laufmittelverbrauch die erste Produktfraktion. Sämtliche produkthaltigen Fraktionen werden vereinigt, einrotiert und der Rückstand wird, bis zur Schaumbildung, mehrere Male mit Dichlormethan koevaporiert. Nach Trocknen des farblosen Schaumes bei 40°C im Hochvakuum kann man 0,201 g(0,307 mmol, 92 %) Verbindung isolieren.
DC (Kieselgel): R_{f} = 0,5 + 0,57 (Dichlormethan/Methanol 95:5)

| Analyse: C₂₉H₃₁N₇O₁₁ x 1/2 H₂O (662,61) | | | | | | |
|---|---|---|---|---|---|---|
| ber. | C | 52,56 | H | 4,86 | N | 14,79 |
| gef. | | 52,41 | | 4,98 | | 14,52 |

UV (Methanol): λₘₐₓ[nm] (lg ε): 212 (4,55); 267 (4,54); [273 (4,40)]
¹H-NMR (250 MHz, d₆-DMSO, ppm):
10,65 + 10,63 (2s, 1H, NH); 8,72 + 8,69 (2s, 1H, H-C(8)); 8,61 + 8,59 (2s, 1H, H-C(2)); 8,13 (d, 2H, o-H zu NO₂(NPEOC)); 8,03 (d, 2H, o-H zu NO₂(NPEE)); 7,61 (d, 2H, m-H zu NO₂(NPEOC)); 7,24 + 7,23 (2d, 2H, m-H zu NO₂(NPEE)); 6,11 + 6,08 (2s, 1H, H-C(1')); 5,24 (m, 2H, HO-C(5'), HO-C(3')); 4,81-4,74 (m, 2H, CH(NPEE), H-C(2')); 4,38 (t, 2H, α-CH₂(NPEOC)); 4,38-4,26 (m, 1H, H-C(3')); 3,99 (m, 1H, H-C(4')); 3,72-3,42 (m, 4H, CH₂(5'), α-CH₂(NPEE)); 3,10 (t, 2H, β-CH₂(NPEOC)); 2,63-2,49 (m, 2H, β-CH₂(NPE)); 1,18 + 1,10 (2d, 3H, CH₃(NPEE))

### 20.3 N⁶-[2-(4-Nitrophenylethoxycarbonyl]-2'-O-[(1-(4-nitrophenyl)ethyl]-adenosin

0,5 g (0,711 mmol) N⁶-2-(4-Nitrophenyl)ethoxycarbonyl]-3',5'-O-(1,1,3,3-tetraisopropyldisiloxan-1,3-diyl)-adenosin werden in 9 ml absolutem Dichlormethan gelöst, mit 0,56 mg (2,94 µmol) p-TsOH·H₂O versetzt und im Eisbad auf 0°C abgekühlt. Zu dieser Lösung gibt man 0,20 g (1,06 mmol) 4-Nitrophenylethylvinylether, rührt zunächst im Eisbad, läßt das Reaktionsgemisch langsam auf Raumtemperatur erwärmen und rührt anschließend über Nacht. Es wird 3 x mit je 50 ml gesättigter Natriumhydrogencarbonat-Lösung ausgeschüttelt, alle wäßrigen Phasen extrahiert man mit Dichlormethan zurück, die vereinigten organischen Phasen werden über Natriumsulfat getrocknet und einrotiert. Der Rückstand wird in 1,8 ml absolutem Tetrahydrofuran gelöst, mit 0,56 g (1,778 mmol) Tetrabutylammoniumfluorid · 3 H₂O, 0,2 ml (3,55 mmol) Eisessig versetzt, bei Raumtemperatur 4 Stunden gerührt und anschließend einrotiert. Zur Reinigung trägt man das resultierende Öl auf eine Kieselgelsäule (3,0 x 10,9 cm) auf, eluiert mit Dichlormethan (500 ml), Dichlormethan/Methanol 95:1 (485 ml), 80:1 (410 ml), 70:1 (710 ml), 65:1 (650 ml), erhält nach 1450 ml Laufmittelverbrauch die erste Fraktion die Produkt enthält, vereinigt alle folgenden Produktfraktionen und rotiert ein. Der Rückstand wird mehrmals, bis zur Schaumbildung, mit Dichlormethan koevaporiert und anschließend bei 40°C im Hochvakuum getrocknet. Man erhält 0,31 g (0,474 mmol, 67 %) gewünschtes Produkt in Form eines farblosen Schaumes.
DC (Kieselgel): R_{f} = 0,5 + 0,57 (Dichlormethan/Methanol 95:5)
C₂₉H₃₁N₇O₁₁ x 1/2 H₂O (662,61)
Spektroskopische Daten sind identisch mit Beispiel 20.2.

### 20.4 5'-O-(4,4'-Dimethoxytriphenylmethyl)-N⁶-[2-(4-nitrophenyl)ethoxy-carbonyl]-2'-O-[1-(4-nitrophenylethoxy)ethyl]-adenosin

0,5 g (0,76 mmol) N⁶-[-2-(4-Nitrophenyl)ethoxycarbonyl]-2'-O-[1-(4-nitrophenylethoxy)ethyl]-adenosin koevaporiert man 3 x mit je 10 ml absolutem Pyridin, löst den Rückstand in 30 ml absolutem Pyridin, versetzt mit 0,36 g (1,07 mmol) 4,4'-Dimethoxytriphenylmethylchlorid und rührt bei Raumtemperatur über Nacht. Anschließend wird mit Essigester verdünnt und 3 x mit je 30 ml gesättigter Natriumhyrogencarbonat-Lösung ausgeschüttelt. Die wäßrigen Phasen extrahiert man mit Essigester zurück, alle organischen Phasen werden vereinigt, über Natriumsulfat getrocknet, abfiltriert, einrotiert und der Rückstand mehrmals mit Toluol koevaporiert.
Das Rohprodukt wird zur Reinigung auf eine Kieselgelsäule (3,0 x 16,0 cm) aufgetragen, mit Toluol (200 ml), Toluol/Essigester 3:1 (400 ml), 2:1 (300 ml), 1:1 (400 ml), 1:2 (600 ml) eluiert und man erhält nach 1100 ml Laufmittelverbrauch die erste Produktfraktion. Sämtliche produkthaltigen Fraktionen werden vereinigt und einrotiert. Den resultierenden Rest löst man in 3 ml Essigester, tropft in 75 ml n-Hexan ein, saugt den erhaltenen Niederschlag ab und trocknet ihn bei 40°C im Hochvakuum über Paraffinschnitzeln. Von der Verbindung erhält man 0,7 g (0,73 mmol, 96 %) in Form eines farblosen, amorphen Feststoffs.
DC (Kieselgel): R_{f} = 0,44 (Toluol/Essigester/Methanol 5:4:1)

| Analyse: C₅₀H₄₉N₇O₁₃ (955,98) | | | | | | |
|---|---|---|---|---|---|---|
| ber. | C | 62,82 | H | 5,16 | N | 10,25 |
| gef. | | 62,28 | | 5,26 | | 10,23 |

UV (Methanol): λₘₐₓ [nm] (Ig ε): 202 (5,01); 236 (4,42); 267 (4,59); [275 (4,52)]
¹H-NMR (250 MHz, d₆-DMSO, ppm):
10,55 (s, 1H, NH); 8,55-8,49 (m, 2H, H-C(8), H-C(2)); 8,13 (d, 2H, o-H zu NO₂(NPEOC)); 8,03-7,97 (m, 2H, o-H zu NO₂(NPEE); 7,60 (d, 2H, m-H zu NO₂(NPEOC)); 7,37-7,20 (m, 11H, m-H zu NO₂(NPEE), H(Phenyl), m-H zu OCH₃); 6,48-6,78 (m, 4H, o-H zu OCH₃); 6,13-6,11 (m, 1H, H-C(1')); 5,28-5,26 (m, 1H, HO-C(3')); 4,94-4,87 (m, 1H, CH(NPEE)); 4,85-4,78 (m, 1H, H-C(2')); 4,41-4,36 (m, 3H, α-CH₂(NPEOC), H-C(3')); 4,12-4,10 (m, 1H, H-C(4')); 3,70 (s, 6H, OCH₃); 3,62-3,39 (m, 2H, CH₂(5')); 3,10 (t, 2H, β-CH₂(NPEOC)); 2,64-2,52 (m, 2H, β-CH₂(NPEE)); 1,20+1,13 (2d, 3H, CH₃(NPEE))

### 21. 5'-O-(4,4'-Dimethoxytriphenylmethyl)-N⁶-[2-(4-nitrophenyl)ethoxycarbonyl]-2'-O-[1-(4-nitrophenylethoxy)ethyl]-adenosin-3'-O-(N,N-diisopropyl-2-cyanoethyl)-phosphitamid

0,4 g (0,46 mmol) 5'O-(4,4'-Dimethoxytriphenylmethyl)-N⁶-[2-(4-nitrophenyl)ethoxycarbonyl]-2'-O-[1-(4-nitrophenylethoxy)ethyl]-adenosin werden zusammen mit 21,8 mg (0,31 mmol) 1H-Tetrazol in einen mit Stickstoffgas gefluteten Kolben eingewogen, mit 8 ml absolutem Acetonitril (Rückstandsanalyse) in Lösung gebracht und anschließend mit 0,37 g (1,24 mmol) Bis(diisopropylamino)(2-cyanoethoxy)phosphan versetzt. Die Reaktionslösung wird bei Raumtemperatur über Nacht unter Schutzgasatmosphäre gerührt, mit Essigester verdünnt und 3 x mit je 40 ml Natriumchlorid/Natriumhydrogencarbonat-Lösung gewaschen. Mit Essigester extrahiert man alle wäßrigen Phasen zurück, die vereinigten organischen Phasen werden über Natriumsulfat getrocknet, abfiltriert und einrotiert.
Zur Reinigung trägt man den Rückstand auf eine Kieselgelsäule (2,5 x 18,0 cm) auf, chromatographiert mit Toluol/Essigester 3:1 (400 ml), 2:1 (600 ml), erhält nach 550 ml Laufmittelverbrauch die erste Produktfraktion, welche nachfolgend alle vereinigt und einrotiert werden. Nach Koevaporieren des Rückstandes mit Essigester und anschließendem Trocknen des farblosen Schaumes im Hochvakuum, erhält man 0,45 g (0,39 mmol, 85 %) Verbindung.
DC (Kieselgel): R_{f} = 0,14+0,23 (Toluol/Essigester 1:1)

| Analyse: C₅₉H₆₆N₉O₁₄P (1156,20) | | | | | | |
|---|---|---|---|---|---|---|
| ber. | C | 61,29 | H | 5,75 | N | 10,90 |
| gef. | | 60,61 | | 6,01 | | 10,76 |

UV (Methanol): λₘₐₓ[nm] (lg ε): 204 (4,98); 236 (4,47); 266 (4,56); [276 (4,48)]
¹H-NMR (250 MHz, CDCl₃, TMS, ppm):
8,66-8,63 (m, 1H, H-C(8)); 8,21-7,98 (m, 6H, H-C(2), o-H zu NO₂(NPEOC und NPEE), NH); 7,46-7,11 (m, 13H, m-H zu NO₂(NPEE und NPEOC), H-Phenyl, m-H zu OCH₃)); 6,83-6,76 (m, 4H, o-H zu OCH₃)); 6,21-6,11 (m, 1H, H-C(1')); 5,23-5,08 (m, 1H, H-C(2')); 4,98-4,73 (m, 1H, CH(NPEE)); 4,61-4,50 (m, 3H, α-CH₂(NPEOC), H-C(3')); 4,41-4,28 (m, 1H, H-C(4')); 3,93-3,16 (m, 8H, OCH₃, α-CH₂(NPEE)); 3,76-3,42 (m, 4H, P-O-CH₂, CH₂(5')); 3,45-3,27 (m, 2H, n-CH); 3,16 (t, 2H, β-CH₂(NPEOC)); 2,72-2,57 (m, 4H, β-CH₂-CN), CH₂-CN); 1,35-1,15 (m, 15H, CH₃(NPEE), C(CH₃)₂)
³¹P-NMR (161,70 MHz, CDCl₃, H₃PO₄, ppm):
151,56; 150,98; 150,79; 150,53

### 22. 5'-O-(4,4'-Dimethoxytriphenylmethyl)-N⁶-[2-(4-nitrophenyl)ethoxycarbonyl]-2'-O-[1-(4-nitrophenylethoxy)ethyl]-adenosin-3'-O-(N,N-diisopropyl-[2-(4-nitrophenyl)ethyl)-phosphitamid

0,4 g (0,41 mmol) 5'-0-(4,4'-Dimethoxytriphenylmethyl)-N⁶-[2-(4-nitrophenyl)ethoxycarbonyl]-2'-O-[1-(4-nitrophenylethoxy)ethyl]-adenosin werden zusammen mit 19,78 mg (0,282 mmol) 1H-Tetrazol in einen mit Stickstoffgas gefluteten Kolben eingewogen, mit 9 ml absolutem Acetonitril (Rückstandsanalyse) gelöst und anschließend mit 0,45 g (1,13 mmol) Bis(diisopropylamino)-2-(4-nitrophenylethoxy)phosphan versetzt. Die Reaktionslösung wird unter Schutzgasatmosphäre über Nacht bei Raumtemperatur gerührt, mit Essigester verdünnt und 3 x mit je 40 ml Natriumchlorid/Natriumhydrogencarbonat-Lösung gewaschen. Alle wäßrigen Phasen werden mit Essigester rückextrahiert, die vereinigten organischen Phasen werden über Natriumsulfat getrocknet, abfiltriert und einrotiert. Das Rohprodukt wird zur Reinigung auf eine Kieselgelsäule (82,5 x 18,0 cm) aufgetragen, mit Toluol (100 ml), Toluol/Essigester 5:1 (300 ml), 4:1 (300 ml), 3:1 (400 ml), 2:1 (300 ml) eluiert, wobei man nach 700 ml Laufmittelverbrauch die erste Produktfraktion auffangen kann. Sämtliche produkthaltigen Fraktionen werden vereinigt, einrotiert, der erhaltene Rückstand wird mehrmals mit Essigester koevaporiert und der resultierende farblose Schaum wird im Hochvakuum getrocknet. Es können 0,431 (0,33 mmol, 80 %) der gewünschten Verbindung isoliert werden.
DC (Kiesegel): R_{f} = 0,26 + 0,30 (Toluol/Essigester 1:1)

| Analyse: C₆₄H₇₀N₉O₁₆P (1252,29) | | | | | | |
|---|---|---|---|---|---|---|
| ber. | C | 61,38 | H | 5,68 | N | 10,06 |
| gef. | | 60,81 | | 5,82 | | 9,54 |

UV (Methanol): λₘₐₓ [nm] (Ig ε): 207 (5,23); [214 (5,09)]; 269 (4,70)
¹H-NMR (250 MHz, CDCl₃, TMS, ppm):
8,66-8,60 (m, 1H, H-C(8)); 8,19-7,96 (m, (H, H-C(2), o-H zu NO₂(NPEOC, NPE und NPEE), NH); 7,44-7,07 (m, 15H, m-H zu NO₂(NPEE und NPEOC), H(Phenyl), m-H zu OCH₃); 6,76 + 6,75 (2d, 4H, o-H zu OCH₃); 6,25-6,08 (m, 1H, H-C(1')); 5,21-4,68 (m, 2H, H-C(2'), CH(NPEE)); 4,54-4,42 (m, 3H, H-C(3'), α-CH₂(NPEOC)); 4,38-4,28 (m, 1H, H-C(4')); 4,0-3,70 (m, 8H, α-CH₂(NPEE), OCH₃); 3,60-3,40 (m, 4H, P-O-CH₂(5')); 3,35-3,22 (m, 2H, N-CH); 3,14 (t, 2H, CH₂(NPEOC); 3,05-2,52 (m, 4H, β-CH₂(NPEE), β-CH₂(NPE); 1,29-0,97 (m, 15H, CH₃(NPEE), C(CH₃)2)
³¹P-NMR (161,70 MHz, CDCl₃, H₃PO₄, ppm):
150,58; 149,89; 149,32; 149,25

### 23.1 N⁶-Benzoyl-2'-O-[1-(4-nitrophenylethoxy]-3',5'-O-(1,1,3,3-tetraisopropyldisiloxan-1,3-diyl)-adenosin

1,0 g (1,63 mmol) N⁶-Benzoyl-3',5'-O-(1,1,3,3-tetraisopropyldisiloxan-1,3-diyl)-adenosin werden in 16 ml absolutem Dichlormethan gelöst, mit 32,6 mg (0,17 mmol) p-TsOH·H₂O 0,46 g (2,4 mmol) 4-Nitrophenylethylvinylether versetzt, bei Raumtemperatur 1 1/2 Stunden gerührt, mit 0,5 N Natriummethanolat/Methanol-Lösung neutralisiert und einrotiert. Der Rückstand wird in Dichlormethan aufgenommen und 3 x mit je 50 ml gesättigter Natriumhydrogencarbonat-Lösung extrahiert. Sämtliche wäßrigen Phasen extrahiert man mit Dichlormethan zurück, die vereinigten organischen Phasen werden über Natriumsulfat getrocknet, abfiltriert und einrotiert. Das Rohprodukt wird zur Reinigung auf eine Kieselgelsäule 83,5 x 16,0 cm) aufgetragen, wobei mit Toluol (200 ml), Toluol/Essigester 4:1 (400 ml), 3:1 (600 ml), 2:1 (500 ml) chromatographiert wird und man nach 500 ml Laufmittelverbrauch die erste Produktfraktion auffangen kann. Sämtliche Fraktionen, welche Produkt enthalten, werden vereinigt, einrotiert und der Rückstand, bis zur Schaumbildung, mehrere Male mit Dichlormethan koevaporiert. Nach Trocknen des leicht gelb gefärbten Schaumes bei 40°C im Hochvakuum, erhält man 1,0 g (1,24 mmol, 76 %) Verbindung.
DC (Kieselgel): R_{f} = 0,40 (Toluol/Essigester 1:1)

| Analyse: C₃₉H₅₄N₆O₉Si₂ (807,07) | | | | | | |
|---|---|---|---|---|---|---|
| ber. | C | 58,04 | H | 6,74 | N | 10,41 |
| gef. | | 58,05 | | 6,78 | | 10,26 |

UV (Methanol): λ [nm] (Ig ε): 276 (4,41)
¹H-NMR (250 MHz, CDCl₃, TMS, ppm):
9.01 (s, 1H, NH); 8,71 + 8,63 (2s, 1H, H-C(8)); 8,27 + 8,20 (2s, 1H, H-C(2)); 8,10-7,94 (m, 4H, o-H Zu NO₂(NPEE), o-H(Benzoyl); 7,57-7,07 (m, 5H, m-H zu NO₂(NPEE), m- und p-H(Benzoyl)); 6,01-5,99 (2s, 1H, H-C(1')); 5,06-4,97 (m, 1H, CH(NPEE)); 4,60-4,49 (m, 1H, H-C(2')); 4,40-4,32 (m, 1H, H-C(3')); 4,23-3,64 (m, 5H, H-C(4'), CH₂(5'), α-CH₂(NPEE)); 2,97-2,86 (m, 2H, β-CH₂(NPEE)); 1,41 + 1,34 (2d, 3H, CH₃(NPEE)); 1,02-0,88 (m, 28H, Isopropyl)

### 23.2 N⁶-Benxoyl-2'-O-[1-(4-nitrophenylethoxy)ethyl]-adenosin

2,0 g (3,25 mmol) N⁶-Benxoyl-3',5'-O-(1,1,3,3-tetraisopropyldisiloxan-1,3-diyl)-adenosin werden in 40 ml absolutem Dichlormethan gelöst, mit 5,6 mg (29,4 µmol) p-TsOH·H₂O versetzt und im Eisbad auf 0°C abgekühlt. Zu dieser Lösung gibt man 2,0 g (10,6 mmol) 4-Nitrophenylethylvinylether, rührt zunächst im Eisbad, läßt das Reaktionsgemisch langsam auf Raumtemperatur erwärmen, neutralisiert das Reaktionsgemisch nach 1 1/2 Stunden mit 0,5 N Natriummethanolat/Methanol-Lösung und rotiert ein. Der Rückstand wird in Dichlormethan aufgenommen, 3 x mit je 60 ml gesättigter Natriumhydrogencarbonat-Lösung ausgeschüttelt. Alle wäßrigen Phasen extrahiert man mit Dichlormethan zurück, die vereinigten organischen Phasen werden über Natriumsulfat getrocknet und einrotiert. Der Rückstand wird in 8,0 ml absolutem Tetrahydrofuran gelöst, mit 2,46 g (7,82 mmol) Tetrabutylammoniumfluorid · 3 H₂O versetzt, bei Raumtemperatur 30 Minuten gerührt und anschließend einrotiert.
Zur Reinigung trägt man das resultierende Öl auf eine Kieselgelsäule (83,0 x 17,0 cm) auf, eluiert mit Dichlormethan (200 ml), Dichormethan/Methanol 50:1 (200 ml), 45:1 (370 ml), 40:1 (140 ml), 35:1 (180 ml), erhält nach 550 ml Laufmittelverbrauch die erste Fraktion die Produkt enthält, vereinigt alle folgenden Produktfrationen und rotiert ein. Den Rückstand löst man in 2 ml Dichlormethan gelöst, tropft in 250 ml Diethylether, saugt den entstandenen farblosen Niederschlag ab und trocknet ihn anschließend bei 40°C im Hochvakuum. Man erhält 1,26 g (2,23 mmol, 68 %) gewünschtes Produkt in Form eines farblosen, amorphen Feststoffs.
Schmelzpunkt: 170°C
DC (Kieselgel): R_{f} = 0,54 + 0,60 (Dichlormethan/Methanol 9:1)

| Analyse: C₂₇H₂₈N₆O₈ (653,61) | | | | | | |
|---|---|---|---|---|---|---|
| ber. | C | 57,44 | H | 4,99 | N | 14,88 |
| gef. | | 57,06 | | 5,26 | | 14,62 |

UV (Methanol): λₘₐₓ[nm] (lg ε): 277 (4,44);
¹H-NMR (250 MHz, d₆-DMSO, ppm):
11,23 (s, 1H, NH); 8,75 + 8,74 (2s, 1H, H-C(8)); 8,72 + 8,71 (2s, 1H, H-C(2)); 8,08-8,01 (m, 4H, o-H zu NO₂(NPEE), o-H(Benzoyl)); 7,64-7,51 (m, 3H, m- und p-H(Benzoyl)); 7,31-7,28 (2d, 2H, m-H zu NO₂(NPEE)); 6,15 (d, 1H, H-C(1')); 6,15 (d, 1H, H-C(1')); 5,37-5,13 (m, 2H, HO-C(5'), HO-C(3')); 4,93-4,72 (m, 2H, CH(NPEE), H-C(2')); 4,35-4,17 (m, 1H, H-(3')); 4,0 (m, 1H, H-C(4')); 3,79-3,40 (m, 4H, CH₂(5'), α-CH₂(NPEE)); 2,68-2,60 (m, 2H, β-CH₂(NPEE)); 1,18 + 1,134 (2d, 3H, CH₃(NPEE))

### 23.3 N⁶-Benzoyl-5'-O-(4,4'-dimethoxytriphenylmethyl)-2'-O-[1-4'-nitrophenylethoxy)ethyl]-adenosin

0,9 g (1,59 mmol) N⁶-Benzoyl-2'-O-[1-(4-nitrophenylethoxy)ethyl]-adenosin koevaporiert man 3 x mit je 10 ml absolutem Pyridin, löst den Rückstand in 40 ml absolutem Pyridin, versetzt mit 0,59 g (1,75 mmol) 4,4'-Dimethoxytriphenylmethylchlorid und rührt die Reaktionslösung bei Raumtemperatur über Nacht. Anschließend gibt man 10 ml Methanol zu, rührt weitere 30 Minuten bei Raumtemperatur, engt auf die Hälfte ein, nimmt mit Dichlormethan auf und schüttelt 2 x mit je 50 ml Wasser aus. Die wäßrige Phase extrahiert man mit Dichlormethan zurück, alle organischen Phasen werden vereinigt, über Natriumsulfat getrocknet, abfiltriert, einrotiert und der Rückstand mehrmals mit Toluol koevaporiert.
Zur Reinigung trägt man das Rohprodukt auf eine Kieselgelsäule (3,2 x 17,0 cm) auf, eluiert mit Toluol (100 ml), Toluol/Essigester 2:1 (600 ml), 1:1 (300 ml), 1:2 (900 ml), 1:3 (400 ml), erhält nach 1100 ml Laufmittelverbrauch die erste produkthaltige Fraktion, die in der Folge alle vereinigt und einrotiert werden. Den Rückstand löst man in 2 ml Dichlormethan, tropft in 200 ml Diethylether ein, saugt den erhaltenen Niederschlag ab und trocknet ihn bei 40°C im Hochvakuum. Man erhält 1,1 g (1,27 mmol, 80 %) des gewünschten Produkts als farblosen, amorphen Feststoff.
Schmelzpunkt: 120°C
DC (Kieselgel): R_{f} = 0,63 (Dichlormethan/Methanol 15:1)

| Analyse: C₄₈H₄₀N₆O₁₀ (866,93) | | | | | | |
|---|---|---|---|---|---|---|
| ber. | C | 66,50 | H | 5,34 | N | 9,69 |
| gef. | | 65,82 | | 5,21 | | 9,77 |

UV (Methanol): λₘₐₓ [nm] (Ig ε): 203 (4,91); 233 (4,49); 276 (4,45)
¹H-NMR (250 MHz, d₆-DMSO, ppm):
11,25 (s, 1H, NH); 8,65-8,61 (m, 2H, H-C(2)); 8,07-8,02 (m, 4H, o-H zu NO₂(NPEE), o-H(Benzoyl); 7,64-7,51 (m, 3H, m- und p-H(Benzoyl)); 7,36-7,15 (m, 11H, m-H zu OCH₃, H(Phenyl), m-H zu NO₂(NPEE)); 6,84-6,81 (m, 4H, o-H zu OCH₃)); 6,17-6,16 (m, 1H, H-C(1')); 5,35-5,33 (m, 1H, HO-C(3')); 5,05-4,91 (m, 1H, CH(NPEE)); 4,87-4,83 (m, 1H, H-C(2')); 4,48-4,31 (m, 1H, H-C(3')); 4.13 (m, 1H, H-C(4')); 3,70-3,48 (m, 10H, OCH₃, CH₂(5'), α-CH₂(NPEE)); 2,70-2,61 (m, 2H, β-CH₂(NPEE)); 1,25 + 1,13 (2d, 3H, CH₃(NPEE))

### 24. N⁶-Benzoyl-5'-O-(4,4'-dimethoxytriphenyImethyl)-2'-O-[1-(4-nitrophenylethoxy)ethyl]-adenosin-3'-O-(N,N-diisopropyl-2-cyanoethyl)-phosphitamid

In einen mit Stickstoffgas gefluteten Kolben wiegt man 1,0 g (1,5 mmol) N⁶-Benzoyl-5'-O-(4,4'-dimethoxytriphenyImethyl)-2'-O-[1-(4-nitrophenylethoxy)ethyl-adenosin, 54,64 mg (0,78 mmol) 1H-Tetrazol ein, löst die Reaktanden in 20 ml absolutem, säurefreiem Dichormethan, versetzt mit 0,83 g (3,11 mmol) Bis(diisopropylamino)(2-cyanoethoxy)phosphan und rührt die Reaktionslösung unter Schutzgasatmosphäre über Nacht. Man verdünnt anschließend mit Dichlormethan, schüttelt 3 x mit je 50 ml Natriumchlorid/Natriumhydrogencarbonat-Lösung aus, extrahiert die wäßrigen Phasen mit Dichlormethan zurück, vereinigt sämtliche organischen Phasen, trocknet sie über Natriumsulfat, filtriert ab und rotiert ein.
Zur Reinigung trägt man das Rohprodukt auf eine Kieselgelsäule (3,0 x 15,0 cm) auf, eluiert mit Toluol (200 ml), Toluol/Essigester 3:1 (400 ml), 2:1 (600 ml), erhält nach 550 ml Laufmittelverbrauch die erste Produktfraktion, welche in der Folge vereinigt und einrotiert werden. Bis zur Schaumbildung koevaporiert man den resultierende Rückstand mehrmals mit Dichlormethan. Nach Trocknen des farblosen Schaumes im Hochvakuum isoliert man 1,07 g (1,0 mmol, 87 %) des gewünschten Produkts.
DC (Kieselgel): R_{f} = 0,54 + 0,66 (Toluol/Essigester 1:3)

| Analyse: C₅₇H₆₃N₈O₁₁P (1067,15) | | | | | | |
|---|---|---|---|---|---|---|
| ber. | C | 64,15 | H | 5,95 | N | 10,50 |
| gef. | | 64,52 | | 6,27 | | 9,96 |

UV (Methanol): λₘₐₓ [nm] (Ig ε): 204 (4,93; 233 (4,52); 276 (4,46)
¹H-NMR (250 MHz, CDCl₃, TMS, ppm):
9,10 (s, 1H, NH): 8,70-8,68 (m, 1H, H-C(8)); 8,28-8,21 (m, 1H, H-C(2)); 8,04-7,99 8m, 4H, o-H zu NO₂(NPEE), o-H(Benzoyl); 7,62-7,16 (m, 14H, H(Phenyl), m- und p-H(Benzoyl), m-H zu NO₂(NPEE), m-H zu OCH₃)); 6,83-6,67 (m, 4H, o-H zu OCH₃)); 6,23-6,11 (m, 1H, H-C(1')); 5,39-5,11 (m, 1H, H-C(2')); 4,98-4,72 (m, 1H, CH(NPE)); 4,3-4,53 (m, 1H, H-C(4')); 3,93-3,82 (m, 2H, α-CH₂(NPEE)); 3,77+3,76 (2s, 6H, OCH₃); 3,68-3,21 (m, 6H, P-O-CH₂, CH₂(5'), N-CH); 2,70-2,62 (m, 4H, CH₂-CN, β-CH₂(NPEE)); 1,38-1,02 (m, 15H, CH₃(NPEE), C(CH₃)₂)
³¹P-NMR (161,70 MHz, CDCl₃, H₃PO₄, ppm):
151,60; 151,05; 150,87; 150,54

### 25.1 N²-[2-(4-Nitrophenyl)ethoxycarbonyl)-O⁶-[2-(4-nitrophenyl)ethyl]-2'-O-[1-(4-nitrophenylethoxy)ethyl]-3',5'-O-(1,1,3,3-tetraisopropyldisiloxan-1,3-diyl)-guanosin

0,5 g (0,575 mmol) N²-[2-(4-Nitrophenyl)ethoxycarbonyl]-O⁶-[2-(4-nitrophenyl)ethyl-3',5'-O-(1,1,3,3-tetraisopropyldisiloxan-1,3-diyl)-guanosin werden in 7 ml absolutem Dichlormethan gelöst, mit 0,6 mg (7,1 µmol) p-TsOH·H₂O versetzt und im Eisbad auf 0°C abgekühlt. Anschließend gibt man 0,22 g (1,15 mmol) 4-Nitrophenylethylvinylether zu, rührt zunächst im Eisbad, nach 2 Stunden läßt man die Reaktionslösung langsam erwärmen und rührt anschließend bei Raumtemperatur über Nacht. Es wird mit Dichlormethan verdünnt, 3 x mit je 40 ml gesättigter Natriumhydrogencarbonat-Lösung ausgeschüttelt, die wäßrigen Phasen man mit Dichlormethan zurückextrahiert und alle organischen Phasen vereinigt, über Natriumsulfat getrocknet und einrotiert.
Den öligen Rückstand trägt man zur Reinigung auf eine Kieselgelsäule (3,0 x 11,0 cm) auf, chromatograhiert mit Toluol (500 ml), Toluol/Essigester 20:1 (630 ml), 15:1 (320 ml), 10:1 (440 ml), 5:1 (360 ml), 4:1 (350 ml), erhält nach 1900 ml Laufmittelverbrauch die erste Produktfraktion, vereinigt alle produkthaltigen Fraktionen und rotiert ein. Einen farblosen Schaum erhält man durch mehrmaliges Koevaporieren des öligen Rückstandes mit Dichlormethan. Nach Trocknen des Schaumes bei 40°C im Hochvakuum, isoliert man 0,55 g (0,518 mmol, 90 %) gewünschtes Produkt.
DC (Kieselgel): R_{f} = 0,53 (Toluol/Essigester 1:1)

| Analyse: C₄₉H₆₄N₈O₁₅Si₂ (1061,27) | | | | | | |
|---|---|---|---|---|---|---|
| ber. | C | 55,45 | H | 6,07 | N | 10,55 |
| gef. | | 55,91 | | 6,39 | | 9,83 |

¹H-NMR (250 MHz, d₆-DMSO, ppm):
10,31 +10,30 (2s, 1H, NH); 8,20-8,10 (m, 5H, H-C(8), o-H zu NO₂(NPE und NPEOC)); 7,93+7,74 (2d, 2H, o-H zu NO₂(NPEE)); 7,64-7,56 (m, 4H, m-H zu NO₂(NPE und NPEOC); 7,37+7,23 (2d, 2H, m-H zu NO₂(NPEE)); 5,91+5,88 (2d, 1H, H-C(1')); 5,04+4,93 (2q, 1H, CH(NPEE); 4,76-4,41 (m, 4H, α-CH₂(NPEOC), H-C(2'), H-C(3')); 4,34 (m, 2H, α-CH₂(NPE)); 4,18-3,53 (m, 5H, H-C(4'), α-CH₂(NPEE), CH₂(5')); 3,28 (t, 2H, β-CH₂(NPEOC)); 3,07 (m, 2H, β-CH2(NPE)); 2,82+2,72 (2t, 2H, β-CH₂(NPEE)); 1,29+1,25 (2d, 3H, CH₃(NPEE)); 0,99-0,83 (m, 28 H, Isopropyl)

### 25.2 N²-[2-(4-Nitrophenyl)ethoxycarbonyl]-O⁶-[2-(4-nitrophenyl)ethyl]-2'-O-[1-(4-nitrophenylethoxy)ethyl]-guanosin

0,4 g (0,46 mmol) N²-[2-(4-Nitrophenyl)ethoxycarbonyl]-O⁶-[2-(4-nitrophenyl)ethyl]-2'-O-[1-(4-nitrophenylethoxy)ethyl]-3',5'-O-(1,1,3,3-tetraisopropyldisiloxan-1,3-diyl)-guanosin werden in 2,0 ml absolutem Tetrahydrofuran gelöst, mit 0,35 g (1,10 mmol) Tetrabutylammoniumfluorid -3 H₂O, 0,12 ml (2,21 mmol) Eisessig versetzt, bei Raumtemperatur 5 Stunden gerührt und anschließend einrotiert.
Zur Reinigung trägt man das Rohprodukt auf eine Kieselgelsäule (2,5 x 14,0 cm) auf, eluiert mit Dichlormethan (300 ml), Dichlormethan/Methanol 100:1 (400 ml), 90:1 (540 ml), 80:1 (400 ml), 70:1 (350 ml), erhält nach 800 ml Laufmittelverbrauch die erste Fraktion die Produkt enthält, vereinigt alle produkthaltigen Fraktionen und rotiert ein. Den erhaltenen Rückstand koevaporiert man, bis zur Schaumbildung, mehrmals mit Dichlormethan, trocknet den farblosen Schaum bei 40°C im Hochvakuum und isoliert daraufhin 0,288 g (0,351 mmol, 95 %) Verbindung.
DC (Kieselgel): R_{f} = 0,25 + 0,3 (Dichlormethan/Methanol 95:5)

| Analyse: C₃₇H₃₈N₈O₁₄ (818,76) | | | | | | |
|---|---|---|---|---|---|---|
| ber. | C | 54,27 | H | 4,67 | N | 13,68 |
| gef. | | 54,25 | | 5,16 | | 13,26 |

UV (Methanol): λₘₐₓ[nm] (Ig ε): 202 (4,60); 215 (4,60); 268 (4,60)
¹H-NMR (250 MHz, d₆-DMSO, ppm):
10,53 + 10,34 (2s, 1H, NH); 8,48 + 8,43 (2s, 1H, H-C(8)); 8,15-8,10 (m, 4H, o-H zu NO₂(NPE und NPEOC)); 8,0-7,92 (2d, 2H, o-H zu NO₂(NPEE)); 7,62-7,57 (m, 4H, m-H zu NO₂(NPE und NPEOC)); 7,23 + 7,19 (2d, 2H, m-H zu NO₂(NPEE)); 6,0 + 5,97 (2d, 1H, H-C(1')); 5,23 + 5,18 (2d, 1H, HO-C(3')); 5,07-5,00 (m, 1H, HO-C(5')); 4,78-4,60 (m, 4H, α-CH₂(NPEOC), H-C(2'), CH(NPEE)); 4,36 (t, 2H, α-CH₂(NPE)); 4,28-4,18 (m, 1H, H-C(4')); 3,72-3,49 (m, 4H, CH₂(5'), α-CH₂(NPEE)); 3,36-3,26 (m, 2H, β-CH₂(NPEOC)); 3,09 (t, 2H, β-CH₂(NPE)); 2,66-2,52 (m, 2H, β-CH₂(NPEE)); 1,17 + 1,11 (2d, 3H, CH₃(NPEE))

### 25.3 N²-[2-(4-Nitrophenyl)ethoxycarbonyl]-O⁶-[2-(4-nitrophenyl)ethyl]-2'-O-[1-(4-nitrophenylethoxy)ethyl]-guanosin

2,0 g (2,3 mmol) N²-[2-(4-Nitrophenyl)ethoxycarbonyl]-O⁶-[2-(4-nitrophenyl)ethyl]-3',5'-O-(1,1,3,3-tetraisopropyldisiloxan-1,3-diyl)-guanosin werden in 25 ml absolutem Dichlormethan gelöst, mit 2,8 mg (14,22 µmol) p-TsOH·H₂O versetzt und im Eisbad auf 0°C abgekühlt. Zu dieser Lösung gibt man 0,99 g (4,6 mmol) 4-Nitrophenylethylvinylether, rührt zunächst im Eisbad, läßt nach 2 Stunden langsam auf Raumtemperatur erwärmen und rührt dann über Nacht. Die Reaktionslösung wird mit Dichlormethan verdünnt und 3 x mit je 50 ml gesättigter Natriumhydrogencarbonat-Lösung gewaschen. Alle wäßrigen Phasen extrahiert man mit Dichlormethan zurück, die vereinigten organischen Phasen werden über Natriumsulfat getrocknet, abfiltriert und einrotiert. Das resultierende Öl löst man in 6,0 ml absolutem Tetrahydrofuran, versetzt mit 1,67 g (5,3 mmol) Tetrabutylammoniumfluorid · 3 H₂O, 0,6 ml (0,01 mmol) Eisessig, rührt 5 Stunden bei Raumtemperatur und rotiert anschließend ein.
Das Rohprodukt wird zur Reinigung auf eine Kieselgelsäule (3,0 x 13,0 cm) aufgetragen, mit Dichlormethan (400 ml), Dichlormethan/Methanol 100:1 (500 ml), 90:1 (360 ml), 80:1 (800 ml), 70:1 (350 ml) eluiert und man erhält nach 1100 ml Laufmittelverbrauch die erste Produktfraktion. Alle folgenden Fraktionen, die Produkt enthalten werden vereinigt, einrotiert und der Rückstand wird, bis zur Schaumbildung, mehrere Male mit Dichlormethan koevaporiert. Nach Trocknen des farblosen Schaumes bei 40°C im Hochvakuum, erhält man vom gewünschten Produkt 1,75 g (1,64 mmol, 93 %).
DC (Kieselgel): R_{f} = 0,25 + 0,3 (Dichlormethan/Methanol 95:5)
C₃₇H₃₈N₈O₁₄ (818,76)
Spektroskopische Daten sind identisch mit Beispiel 25.2.

### 25.4 5'-O-(4,4'-Dimethoxytriphenylmethyl)-N²-[2-(4-nitrophenyl)ethoxycarbonyl]-O⁶-[2-(4-nitrophenyl)ethyl]-2'-O-[1-(4-nitrophenylethoxy)ethyl]-guanosin

1,8 g (2,19 mmol) N²-[2-(4-Nitrophenyl)ethoxycarbonyl]-O⁶-[2-(4-nitrophenyl)ethyl]-2'-O-[1-(4-nitrophenylethoxy)ethyl]-guanosin werden 3 x mit je 10 ml absolutem Pyridin koevaporiert, der Rückstand wird in 35 ml absolutem Pyridin aufgenommen, mit 0,96 g (2,85 mmol) 4,4'-Dimethoxytriphenylmethylchlorid versetzt und bei Raumtemperatur über Nacht gerührt. Anschließend verdünnt man das Reaktionsgemisch mit Essigester, wäscht 3 x mit je 50 ml gesättigter Natriumhydrogencarbonat-Lösung, extrahiert die wäßrigen Phasen mit Essigester zurück, vereinigt sämtliche organischen Phasen, trocknet sie über Natriumsulfat, filtriert ab, rotiert ein und koevaporiert den Rückstand mehrmals mit Toluol.
Das Rohprodukt trägt man zur Reinigung auf eine Kieselgelsäule (3,0 x 14,0 cm) auf, chromatographiert mit Toluol (200 ml), Toluol/Essigester 5:1 (300 ml), 4:1 (750 ml), 3:1 (800 ml), 2:1 (1200 ml), erhält nach 1300 ml Laufmittelverbrauch die erste Produktfraktion, die im folgenden alle vereinigt und einrotiert werden. Den Rückstand löst man in 5 ml Dichlormethan, tropft in 200 ml n-Hexan ein, saugt den erhaltenen Niederschlag ab, trocknet ihn bei 40°C im Hochvakuum über Paraffinschnitzeln und isoliert anschließend 2,36 g (2,1 mmol, 96 %) Verbindung als farblosen, amorphen Feststoff.
DC (Kieselgel): R_{f} = 0,6 (Toluol/Essigester, Methanol 5:4:1)

| Analyse: C₅₈H₅₆N₈O₁₆ (1121,13) | | | | | | |
|---|---|---|---|---|---|---|
| ber. | C | 62,13 | H | 5,03 | N | 9,99 |
| gef. | | 61,61 | | 5,03 | | 10,18 |

UV (Methanol): λₘₐₓ [nm] (Ig ε): 201 (5,01); [214 (4,77)]; 236 (4,49); 268 (4,65)
¹H-NMR (250 MHz, d₆-DMSO, ppm):
10,33 (2s, 1H, NH); 8,31 (s, 1H, H-C(8)); 8,15-8,11 (m, 4H, o-H zu NO₂(NPEOC und NPE)); 7,98+7,92 (2d, 2H zu NO₂(NPEE)); 7,64-7,55 (m, 4H, m-H zu NO₂(NPEOC und NPE)); 7,33-7,15 (m, 1H, m-H zu NO₂(NPEE), H(Phenyl), m-H zu OCH₃); 6,80-6,70 (m, 4H, o-H zu OCH₃); 6,02 (m, 1H, H-C(1')); 5,20 (d, 1H, HO-C(3')); 4,84-4,70 (m, 4H, H-C(2'), CH(NPEE), α-CH₂(NPEOC)); 4,84-4,30 (m, 3H, H-C(3'), α-CH₂(NPE)); 4,01 (m, 1H, H-C(4')); 3,688+3,685+3,680+ 3,673 (4s, 6H, OCH₃); 3,66-3,26 (m, 4H, CH₂(5'), α-CH₂(NPEE)); 3,34-3,19 (m, 2H, β-CH₂(NPEOC)); 3,07 (t, 2H, β-CH₂(NPE)); 2,71-2,50 (m, 2H, β-CH₂(NPEE)); 1,19+1,14 (2d, 3H, CH₃(NPEE))

### 26. 5'-O-(4,4'-Dimethoxytriphenylmethyl)-N²-[2-(4-nitrophenyl)ethoxycarbonyl-O⁶-[2-(4-nitrophenyl)ethyl]-2'-O-[1-(4-nitrophenylethoxy)ethyl]-guanosin-3'-O-(N,N-diisopropyl-2-cyanoethyl)-phosphitamid

In einem mit Stickstoffgas gefluteten Kolben wiegt man 0,4 g (0,35 mmol) 5'-O-(4,4'-Dimethoxytriphenylmethyl)-N²-[2-(4-nitrophenyl)ethoxycarbonyl]-O⁶-[2-(4-nitrophenyl)ethyl]-2'-O-[1-(4-nitrophenylethoxy)ethyl]-guanosin, 15,0 mg (0,21 mmol) H-Tetrazol ein, löst die Reaktanden in 8 ml absolutem Acetonitril (Rückstandsanalyse), gibt 0,25 g (0,85 mmol) Bis(diisopropylamino)(2-cyanoethoxy)phosphan zu und rührt das Reaktionsgemisch unter Schutzgasatmosphäre über Nacht bei Raumtemperatur. Anschließend wird mit Essigester verdünnt und 3 x mit je 40 ml Natriumchlorid/Natriumhydrogencarbonat-Lösung gewaschen. Die wäßrigen Phasen werden mit Essigester rückextrahiert, sämtliche organischen Phasen werden über Natriumsulfat getrocknet, abfiltriert und einrotiert.
Zur Reinigung trägt man das Rohprodukt auf eine Kieselgelsäule (2,5 x 21,0 cm) auf, eluiert mit Toluol (100 ml), Toluol/Essigester 4:1 (125 ml), 3:1 (400 ml), 2:1 (150 ml), 1:1 (100 ml) wobei man nach 350 ml Laufmittelverbrauch die erste Produktfraktion auffangen kann, die in der Folge alle vereinigt und einrotiert werden. Nach Koevaporieren des Rückstandes mit Essigester und Trocknen des farblosen Schaums im Hochvakuum, erhält man 0,34 g (0,25 mmol, 75 %) Guanosin-Phosphitamid.
DC (Kieselgel): R_{f} = 0,43 + 0,52 (Toluol/Essigester 1:1)

| Analyse: C₆₇H₇₃N₁₀O₁₇P (1321,35) | | | | | | |
|---|---|---|---|---|---|---|
| ber. | C | 60,90 | H | 5,56 | N | 10,60 |
| gef. | | 60,85 | | 5,74 | | 10 18 |

UV (Methanol): λₘₐₓ[nm] (lg ε): 203 (50,5); [214 (4,85)]; 236 (4,51); 268 (4,63)
¹H-NMR (250 MHz, CDCl₃, TMS, ppm):
8,14-7,96 (m, 7H, H-C(8), o-H zu NO₂(NPE, NPEOC und NPEE)); 7,52-7,08 (m, 15H, m-H zu NO₂(NPE, NPEOC und NPEE), m-H zu OCH₃, H(Phenyl)); 6,75 + 6,74 (2d, 4H, o-H zu OCH₃)); 6,13-5,98 (m, 1H, H-C(1')); 5,28-5,08 (m, 1H, CH(NPEE)); 4,91-4,69 (m, 3H, H-C(2'), α-CH₂(NPEOC)); 4,58-4,43 (m, 1H, H-C(3')); 4,41-4,19 (m, 3H, H-C(4'), α-CH₂(NPEE)); 4,08-3,53 (m, 2H, P-O-CH₂); 3,738 + 3,73 (2s, 6H, OCH₃); 3,68-3,46 (m, 4H, CH₂(5'), N-CH); 3,35-3,25 (m, 4H, α-CH₂(NPE), β-CH₂(NPEOC)); 3,08-2,98 (m, 2H, β-CH₂(NPE)); 2,75-2,58 (m, 4H, CH₂-CN, β-CH₂(NPEE)); 1,34-0,96 (m, 15H, CH₃(NPEE), C(CH₃)₂)
³¹P-NMR (161-70 MHz, CDCl₃, H₃PO₄, ppm)
151,58; 151,02; 150,82; 150,48

### 27. 5'-O-(4,4'-Dimethoxytriphenylmethyl)-2'-O-[1-(4-nitrophenylethoxy)ethyl]-3'-O-succinoyl-uridin

221,92 mg (0,3 mmol) 5'-O-(4,4'-Dimethoxytriphenylmethyl)-2'-O-[1-(4-nitrophenylethoxy)ethyl]-uridin werden 2 x mit je 5 ml absolutem Pyridin koevaporiert. Der Rückstand wird in 7 ml absolutem Dichlormethan gelöst, mit 48,86 mg (0,4 mmol) 4-Dimethylaminopyridin, 40,07 mg (0,4 mmol) Bernsteinsäureanhydrid versetzt und bei Raumtemperatur über Nacht gerührt. Anschließend verdünnt man die Reaktionslösung mit Dichlormethan, schüttelt 2 x mit je 25 ml gesättigter Natriumhydrogencarbonat-Lösung, 3 x mit je 25 ml 10 %-iger Zitronensäurelösung und 5 x mit je 30 ml Wasser aus. Mit Dichlormethan extrahiert man die wäßrigen Phasen zurück, sämtliche organischen Phasen werden über Natriumsulfat getrocknet, abfiltriert und einrotiert.
Das Rohprodukt wird zur Reinigung auf eine Kieselgelsäule (3,0 x 6,0 cm) aufgetragen, mit Dichlormethan (200 ml), Dichlormethan/Methanol 100:1 (200 ml), 90:1 (450 ml), 80:1 (800 ml), 70:1 (560 ml), 60:1 (300 ml), 50:1 (300 ml) eluiert und man erhält nach 1000 ml Laufmittelverbrauch die erste Produtkfraktion. Alle produkthaltigen Fraktionen werden vereinigt und einrotiert. Bis zur Schaumbildung wird der ölige Rest mehrere Male mit Essigester koevaporiert und anschließend bei 40°C im Hochvakuum getrocknet. Es können 0,184 g (0,219 mmol, 73 %) gewünschtes Produkt in Form eines leicht gelb gefärbten Schaumes isoliert werden.
DC (Kieselgel): R_{f} = 0,54 (Dichlormethan/Methanol 9:1)

| Analyse: C₄₄H₄₅N₃O₁₄ (839,86) | | | | | | |
|---|---|---|---|---|---|---|
| ber. | C | 62,92 | H | 5,40 | N | 5,00 |
| gef. | | 62,21 | | 5,22 | | 5,62 |

UV (Methanol): λₘₐₓ[nm] (lg ε): 202 (4,87); 233 (4,36); 266 (4,28)
¹H-NMR (250 MHz, d₆-DMSO, ppm):
11,49 (s, 1H, NH); 8,11-8,08 (m, 2H, o-H zu NO₂(NPEE)); 7,72-7,66 (m, 1H, H-C(6)); 7,45-7,20 (m, 11H, m-H zu NO₂(NPEE), H(Phenyl), m-H zu OCH₃)); 6,87 (d, 4H, o-H zu OCH₃)); 5,87-5,84 (m, 1H, H-C(1')); 5,48-5,42 (m, 1H, H-C(5)); 5,27-5,23 (m, 1H, H-C(3')); 4,82-4,73 (m, 1H, CH(NPEE)); 4,58-4,53 (m, 1H, H-C(2')); 4,14-4,12 (m, 1H, H-C(4')); 3,71 (s, 6H, OCH₃); 3,69-3,25 (m, 4H, α-CH₂(NPEE), CH₂(5')); 2,88-2,81 (m, 2H, β-CH₂(NPEE)); 2,50-2,48 (m, 4H, CH₂(Succinat)); 1,19 + 1,13 (2d, 3H, CH₃(NPEE))

### 28. 5'-O-(4,4'-Dimethoxytriphenyl)-N⁴-[2-(4-nitrophenyl)ethoxycarbonyl]-2'-O-[1-(4-nitrophenylethoxy)ethyl]-3'-O-succinoyl-cytidin

Man koevaporiert 279,6 mg (0,3 mmol) 5'-O-(4,4'-Dimethoxytriphenylmethyl)-N⁴-[2-(4-nitrophenyl)ethoxycarbonyl]-2'-O-[1-(4-nitrophenylethoxy)ethyl]-cytidin 2 x mit je 5 ml absolutem Pyridin, nimmt den Rückstand in 7 ml absolutem Dichlormethan auf, versetzt mit 48,86 mg (0,4 mmol) 4-Dimethylaminopyridin, 40,07 mg (0,4 mmol) Bernsteinsäureanhydrid und rührt die Reaktionslösung bei Raumtemperatur über Nacht. Anschließend verdünnt man mit Dichlormethan, wäscht nacheinander 2 x mit je 30 ml gesättigter Natriumhydrogencarbonat-Lösung, 3 x mit je 30 ml 10 %-iger Zitronensäurelösung, 1 x mit 30 ml gesättigter Natriumhydrogencarbonat-Lösung und 1 x mit 30 ml Wasser. Die wäßrigen Phasen werden mit Dichlormethan rückextrahiert, sämtliche organischen Phasen werden über Natriumsulfat getrocknet, abfiltiert und einrotiert.
Das Rohprodukt wird zur Reinigung auf eine Kieselgelsäule (3,0 x 8,0 cm) aufgetragen, mit Dichlormethan/Methanol 100:1 (300 ml), 90:1 (570 ml), 85:1 (250 ml), 80:1 (240 ml), 70:1 (280 ml), 60:1 (540 ml), 50:1 (600 ml) chromatographiert und man erhält nach 2000 ml Laufmittelverbrauch die erste Produktfraktion. Sämtliche Fraktionen die Produkt enthalten werden vereinigt und einrotiert. Der Rückstand wird, bis zur Schaumbildung mehrmals mit Essigester koevaporiert und anschließend bei 40°C im Hochvakuum getrocknet.
Man isoliert 0,288 g (0,28 mmol, 93 %) Produkt als leicht gelb gefärbten Schaum.
DC (Kieselgel): R_{f} = 0,63 (Dichlormethan/Methanol 9:1)

| Analyse: C₅₃H₅₅N₅O₁₈ x H₂O (1050,04) | | | | | | |
|---|---|---|---|---|---|---|
| ber. | C | 60,62 | H | 5,27 | N | 6,66 |
| gef. | | 60,89 | | 5,59 | | 6,19 |

UV (Methanol): λₘₐₓ[nm] (Ig ε): 202 (4,99); 236 (4,56); 274 (4,42)
¹H-NMR (250 MHz, d₆-DMSO, ppm):
10,90 (s, 1H, NH); 8,20-8,02 (m, 5H, H-C(6), o-H zu NO₂(NPEOC und NPEE)); 7,58-7,21 (m, 13H, m-H zu NO₂(NPEE und NPEOC), H(Phenyl), m-H zu OCH₃); 6,83-6,78 (m, 5H, H-C(5), o-H zu OCH₃); 5,90 (m, 1H, H-C(1')); 5,22 (m, 1H, H-C(3')); 4,97-4,73 (m, 1H, CH(NPEE)); 4,50-4,35 (m, 3H, α-CH₂(NPEOC), H-C(2')); 4,19 (m, 1H, H-C(4')); 3,72 (s, 6H, OCH₃); 3,61-3,34 (m, 4H, α-CH₂(NPEE), CH₂(5')); 3,07 (m, 2H, β-CH₂(NPEOC)); 2,82 (m, 2H, β-CH₂(NPEE)); 2,59-2,49 (m, 4H, CH₂(Succinat)); 1,20 + 1,09 (2d, 3H, CH₃(NPEE))

### 29. 5'-O-(4,4'-Dimethoxytriphenylmethyl)-N⁶-[2-(4-nitrophenyl)ethoxycarbonyl]-2'-O-[1-(4-nitrophenylethoxy)ethyl]-3'-O-succinoyl-adenosin

239,0 mg (0,25 mmol) 5'-O-(4,4'-Dimethoxytriphenylmethyl)-N⁶-[2-(4-nitrophenyl)ethoxycarbonyl]-2'-O-[1-(4-nitrophenylethoxy)ethyl-adenosin werden 2 x mit 5 ml absolutem Pyridin koevaporiert, der Rückstand wird in 5 ml absolutem Dichlormethan gelöst, mit 40,72 mg (0,33 mmol) 4-Dimethylaminopyridin, 33,35 mg (0,33 mmol) Bernsteinsäureanhydrid versetzt und bei Raumtemperatur über Nacht gerührt. Anschließend wird das Reaktionsgemisch mit Essigester verdünnt und nacheinander 3 x mit je 30 ml gesättigter Natriumhydrogencarbonat-Lösung, 3 x mit je 30 ml 10 %-iger Zitronensäurelösung, 1 x mit 30 ml gesättigter Natriumhydrogencarbonat-Lösung und 1 x mit 30 ml Wasser ausgeschüttelt. Die wäßrigen Phasen werden mit Essigester rückextrahiert, alle organischen Phasen werden über Natriumsulfat getrocknet, abfiltriert und einrotiert. Man löst das resultierende Öl in 3 ml Essigester, tropft in 70 ml n-Hexan ein, saugt den entstandenen Niederschlag ab und trocknet ihn bei 40°C im Hochvakuum über Paraffinschnitzeln. Man erhält 0,201 g (0,19 mmol, 76 %) gewünschtes Produkt in Form eines farblosen amorphen Feststoffs.
DC (Kieselgel): R_{f} = 0,62 (Dichlormethan/Methanol 9:1)

| Analyse: C₅₄H₅₅N₇O₁₇ x H₂O (1074,07) | | | | | | |
|---|---|---|---|---|---|---|
| ber. | C | 60,38 | H | 5,16 | N | 9,12 |
| gef. | | 59,91 | | 4,91 | | 9,14 |

UV (Methanol): λₘₐₓ[nm] (Ig ε): 202 (5,02); 236 (4,44); 267 (4,58); [275 (4,51)]
¹H-NMR (250 MHz, d₆-DMSO, ppm):
12,28 (s, 1H, OH-(Succinat)); 10,70 (s, 1H, NH); 8,62 (s, 1H, H-C(8)); 8,46 + 8,43 (2s, 1H, H-C(2)); 8,13 (d, 2H, o-H zu NO₂(NPEOC)); 8,03-7,97 (m, 2H, o-H zu NO₂(NPEE)); 7,60 (d, 2H, m-H zu NO₂(NPEOC)); 7,39-7,09 (m, 11 H, m-H zu NO₂(NPEE), H(Phenyl), m-H zu OCH₃)); 6,84-6,79 (m, 4H, o-H zu OCH₃)); 6,18-6,12 (m, 1H, H-C(1')); 5,42-5,38 (m, 2H, H-C(2'), H-C(3')); 4,78-4,66 (m, 1H, CH(NPEE)); 4,38 (t, 2H, α-CH₂(NPEOC)); 4,27 (m, 1H, H-C(4')); 3,70 (s, 6H, OCH₃); 3,35-3,22 (m, 3H, H-C(5'), α-CH₂(NPEE)); 3,12-3,02 (m, 3H, H-C(5"), β-CH₂(NPEOC)); 2,59-2,39 (m, 6H, β-CH₂(NPEE)), CH₂(Succinat)); 1,10 + 1,01 (2d, 3H, CH₃(NPEE))

### 30. 5'-O-(4,4'-Dimethoxytriphenylmethyl)-N²-[2-(4-nitrophenyl)ethoxycarbonyl]-O⁶-[-2-(4-nitrophenyl)ethyl]-2'-O-[1-(4-nitrophenylethoxy)ethyl]-3'-O-succinoyl-guanosin

Man koevaporiert 168,17 mg (0,15 mmol) 5'-O-(4,4'-Dimethoxytriphenylmethyl)-N²-[2-(4-nitrophenyl)ethoxycarbonyl]-O⁶-[-2-(4-nitrophenyl)ethyl]-2'-O-[1-(4-nitrophenylethoxy)ethyl]-guanosin 3 x mit je 5 ml absolutem Pyridin, nimmt den Rückstand in 25 ml absolutem Dichlormethan auf, versetzt mit 24,43 mg (0,2 mmol) 4-Dimethylaminopyridin, 20,01 mg (0,2 mmol) Bernsteinsäureanhydrid und rührt die Reaktionslösung bei Raumtemperatur über Nacht. Anschließend wird mit Essigester verdünnt und nacheinander 3 x mit je 20 ml 10 %-iger Zitronensäurelösung, 2 x mit je 20 ml gesättigter Natriumhydrogencarbonat-Lösung und 2 x mit je 20 ml Wasser ausgeschüttelt. Die wäßrigen Phasen extrahiert man mit Essigester zurück, alle organischen Phasen werden über Natriumsulfat getocknet, abfiltiert und einrotiert. Man löst den öligen Rückstand in 2 ml Essigester, tropft in 40 ml n-Hexan ein, saugt den erhaltenen Niederschlag ab und trocknet ihn bei 40°C im Hochvakuum über Paraffinschnitzeln. Vom gewünschten Produkt erhält man 0,116 g (0,095 mmol, 64 %) in Form eines farblosen amorphen Feststoffs.
DC (Kieselgel): R_{f} = 0,46 (Toluol/ Essigester/Methanol 5:4:1)

| Analyse: C₆₂H₆₀N₈O₁₉ (1221,21) | | | | | | |
|---|---|---|---|---|---|---|
| ber. | C | 60,97 | H | 4,95 | N | 9,17 |
| gef. | | 60,28 | | 4,96 | | 9,15 |

UV (Methanol): λₘₐₓ[nm] (Ig ε): 202 (5,07);[214 (4,88)]; 236 (4,48); 268 (4,64);
¹H-NMR (250 MHz, d₆-DMSO, ppm):
12,28 (s, 1H, HO-(Succinat)); 10,32 + 10,28 (2s, 1H, NH); 8,39 + 8,37 (2s, 1H, H-C(8)); 8,16-8,10 (m, 4H, o-H zu NO₂(NPE und NPEOC)); 8,01-7,96 (m, 2H, o-H zu NO₂(NPEE)); 7,63-7,57 (m, 4H, m-H zu NO₂(NPE und NPEOC)); 7,31-7,10 (m, 11H, m-H zu NO₂(NPEE, H(Phenyl), m-H zu OCH₃)); 6,78-6,70 (m, 4H, o-H zu OCH₃); 6,08-6,0 (m, 1H, H-C(1')); 5,48-5,30 (m, 2H, H-C(2'), H-C(3')); 4,78-4,60 (m, 3H, α-CH₂(NPEOC), CH(NPEE)); 4,37 (m, 2H, α-CH₂(NPE)):4,13 (m, 1H, H-C(4')); 3,68 (s, 6H, OCH₃); 3,60-3,05 (m, 8H, CH₂(5'), α-CH₂(NPEE), β-CH₂(NPEOC und NPE)); 2,55-2,49 (m, 6H, β-CH₂(NPEE), CH₂(Succinat)); 1,09 + 1,01 (2d, 3H, CH₃(NPEE))

### 31. Allgemeine Vorschrift zur Herstellung von 4-Alkoxycarbonyloxybenzyl-vinylether- bzw. 4-Acyloxybenzylvinylether-Derivaten

### 31.1 3-Fluor-4-hydroxy-benzylalkohol

22,8 g (0,2 Mol) 2-Fluorphenol werden in 60 ml 20 % KOH (0,214 Mol) aufgelöst und die gelbliche Lösung auf 60°C auf dem Ölbad erwärmt. Anschließend werden 30 ml einer 37 %igen Formaldehydlösung (0,370 mmol) mit H₂O auf 100 ml aufgefüllt und dann innerhalb 3 Stunden zugetropft. Die DC-Kontrolle zeigt das Entstehen zweier Hauptprodukte, wobei das höherlaufende dem gewünschten monoalkylierten Produkt entspricht. Die jetzt rötliche Lösung wird für weitere 3 Stunden bei 60°C gehalten, bevor man die abgekühlte Lösung auf 0,5 l Eiswasser/300 ml Essigsäureethylester (EE) gießt. Mit ca. 20 ml konz. HCl wird der pH-Wert auf 5-6 eingestellt. Nach dem Abtrennen der organischen Phase im Scheidetrichter wird diese zweimal mit je 300 ml Phosphatpuffer pH 6 und 300 ml ges. NaCl-Lösung gewaschen, die gesammelten wäßrigen Phasen mit zweimal 150 ml EE zurückextrahiert und erneut mit Phosphatpuffer/NaCl-Lösung gewaschen. Die vereinigten organischen Phasen werden über NaSO₄ getrocknet, filtriert und am Rotationsverdampfer zum Öl (ca. 23 g) eingeengt. Die weitere Aufarbeitung erfolgt chromatographisch. Dazu wird eine Säule (6 cm ⌀) mit 200 g Flash-Kieselgel beschickt, mit Toluol equilibriert und nach Auftragen des Rohproduktes folgender Gradient (Tol/EE in ml) gefahren: 200/0, 450/50, 400/100, 350/150, 300/200. Man fängt das Eluat in Fraktion zu je 100 ml auf, vereinigt die Produktfraktionen (ca. 500 ml) und engt diese am Rotationsverdampfer auf ca. 100 ml ein. Nach Ankratzen mit einem Glasstab wird die Lösung zur Kristallisation über Nacht im Kühlschrank aufbewahrt. Der farblose Kristallbrei wird abgesaugt, mit 2 x 20 ml kaltem Petrolether gewaschen und im Hochvakuum bei 250°C getrocknet.
Man erhält so 10,3 g (7,25 mmol, 36 %) 3-Fluor-4-hydroxy-benzylalkohol.
- DC (Kieselgel):: R_{f} = 0,52 (Tol/EE = 1/2);
- Schmelzpunkt:: 101 °C
UV (Methanol): λₘₐₓ [nm] (lg ε): 222 (3,98) 272 (3,22)
¹H-NMR (250 MHz, d⁶-DMSO, TMS, ppm):
9,80 (s, 1 H, Phenyl-OH); 7,05 (s, 1 H, H-(C2)); 6,95-6,80 (m, 2 H, H-(C6), H-(C-5)); 5,12 (s, 1 H, Benzyl-OH); 4,35 (d, 2 H, CH₂).

### 31.2 4-Methoxytrityl[2,5-dichloro-4-hydroxy-benzyl]ether

6 g (31,1 mmol) 2,5-Dichlor-4-hydroxy-benzylalkohol werden in 70 ml abs. Pyridin gelöst und dann 11,0 g (35,62 mmol) Monomethoxytrityl-chlorid dazugegeben. Die gelbliche Lösung wird mehrere Stunden bei Raumtemperatur gerührt. Die DC-Kontrolle sollte vollstndige Umsetzung anzeigen, bevor man die Reaktion mit 20 ml MeOH und Rühren für etwa 30 Minuten abstoppt. Die farblose Reaktionslösung wird am Rotationsverdampfer auf ca. 30 ml eingeengt, mit 300 ml EE verdünnt und dann dreimal mit je 300 ml ges. NaCl-Lösung gewaschen. Die vereinigten wäßrigen Phasen werden mit zweimal 200 ml EE zurückextrahiert und diese noch einmal mit 200 ml ges. NaCl-Lösung gewaschen. Die EE-Phasen werden über Na₂SO₄ getrocknet, filtriert und am Rotationsverdampfer zum Öl (ca. 17 g) eingeengt. Nach Koevaporieren mit zweimal 50 ml Toluol wird das Rohprodukt chromatographisch gereinigt. Das Eluat wird in Fraktionen zu je 75 ml aufgefangen, die Produktfraktionen vereinigt (ca. 300 ml) und diese am Rotationsverdampfer zum Öl eingeengt. Man koevaporiert zweimal mit 30 ml dest. MeOH, schäumt mit dest. CH₂Cl₂ auf und trocknet den Schaum, der allmählich ausharzt, im Hochvakuum bei 25°C. Die Ausbeute liegt mit 13,51 g (27,27 mmol) bei 88 % der Theorie.
- DC (Kieselgel):: R_{f} = 0,32 (Tol/EE = 10/1)
¹H-NMR (250 MHz, d⁶-DMSO, TMS, ppm):
10,65 (bs, 1 H, Phenyl-OH); 7,48-7,10 (m, 13 H, Phenyl-H, H-(C6)); 7,00-6,81 (m, 3 H, H-(C3), o-H zu -OCH₃); 4,05 (s, 2 H, CH₂); 3,68 (s, 3 H, -OCH₃).

### 31.3 3-Chlor-4-[2-(4-nitrophenyl)ethoxycarbonyloxy]-benzylalkohole

Der mit abs. Toluol koevaporierte 4-Methoxytrityl-(3-chlor-4-hydroxy)-benzylether (12,1 g = 28,08 mmol) wird in 50 ml abs. CH₃CN aufgenommen und mit 9,35 g (30,0 mmol) 2-(p-Nitrophenyl)ethoxycarbonyl-1-methylimidazoliumchlorid versetzt. Der Suspension fügt man anschließend 700 mg DMAP hinzu und rührt für 30 Minuten bei Raumtemperatur, wobei sich der Niederschlag innerhalb weniger Minuten auflöst. Am Rotationsverdampfer wird die Reaktionslösung auf etwa die Hälfte eingeengt, mit 300 ml EE verdünnt, mit je 200 ml ges. NaHCO₃-/NaCl-Lösung gewaschen, die gesammelten wäßrigen Phasen mit zweimal 100 ml EE zurückextrahiert und erneut mit NaHCO₃-/NaCl-Lösung gewaschen. Die vereinigten organischen Phasen werden über NaSO₄ getrocknet, filtriert und am Rotationsverdampfer zum gelblichen Öl (ca. 24 g) eingeengt. Das Rohprodukt ist für die nachfolgende saure Etherspaltung ausreichend rein. Das Öl wird mit 50 ml CHCl₃/MeOH (4/1) aufgenommen und mit 1,0 g (3,17 mmol) p-Toluolsulfonsäure versetzt bei Raumtemperatur für 2 bis 3 Stunden gerührt. Die Reaktionslösung wird mit 200 ml CHCl₃ verdünnt und auf kalte ges. NaHCO₃-Lösung gegossen. Die organische Phase wird nach dem Abtrennen im Scheidetrichter erneut mit 200 ml NaHCO₃-Lösung und 200 ml ges. NaCl-Lösung gewaschen, die wäßrigen Phasen mit je 50 ml CHCl₃ zurückextrahiert und die vereinigten org. Phasen über NaSO₄ getrocknet, filtriert und am Rotationsverdampfer zum Öl (ca. 25 g) eingeengt. Die weitere Reinigung erfolgt chromatographisch. Dazu wird eine Säule (6 cm ⌀) mit 250 g Flash-Kieselgel beschickt, mit Toluol equilibriert und nach Auftragen des Rohproduktes folgender Gradient (Tol/EE in ml) gefahren: 300/0, 450/50, 400/100, 350/150, 300/200. Man fängt das Eluat in Fraktionen zu je 50 ml auf, vereinigt die Produktfraktionen (ca. 300 ml) und engt diese am Rotationsverdampfer zum Öl ein. Nach Koevaporieren mit MeOH wird das Harz mit CH₂Cl₂ aufgeschäumt und im Hochvakuum bei Raumtemeratur getrocknet.
Man erhält so 7,41 g (21,07 mmol, 74 %) eines farblosen Harzes.
- DC (Kieselgel):: R_{f} = 0,24 (Tol/EE = 3/1)

### 31.4. 2,5-Dichlor-4-pivaloyloxy-benzylalkohol

Der mit abs. Toluol koevaporierte 4-Methoxytrityl-(2,5-dichlor-4-hydroxy)-benzylether (12,0 g = 24,22 mmol) wird in 150 ml abs. CH₃CN aufgenommen und mit 15 ml = 13,77 g (73,93 mmol) Pivalinsäureanhydrid (p.a.) versetzt. Anschließend fügt man 780 mg DMAP hinzu und rührt für 30 Minuten bei Raumtemperatur. Nach ca. 30 Minuten ist die Reaktion beendet. Am Rotationsverdampfer wird die Reaktionslösung auf etwa 50 ml eingeengt, mit 200 ml EE verdünnt, mit je 200 ml ges. NaHCO₃-/NaCl-Lösung gewaschen, die gesammelten wäßrigen Phasen mit zweimal 100 ml EE zurückextrahiert und erneut mit NaHCO₃-/NaCl-Lösung gewaschen. Die vereinigten organischen
Phasen werden über NaSO₄ getrocknet, filtriert und am Rotationsverdampfer zum gelblichen Öl eingeengt. Analog der Vorschrift nach 31.3 (Abspaltung der Methoxytritylschutzgruppe) erhält man so 6,01 g (21,68 mmol) eines farblosen Harzes.
- DC (Kieselgel):: R_{f} = 0,29 (Tol/EE = 3/1)
¹H-NMR (250 MHz, CDCl₃, TMS, ppm):
7,58 (s, 1 H, H-(C3)); 7,12 (s, 1 H, H-(C5)); 4,70 (s, 2 H, -CH₂); 1,38 (s, 9 H, -CH₃).

### 31.5 2-Chlor-4-[2-(4-nitrophenyl)ethoxycarbonyloxy]-benzyl-vinylether

In 120 ml Ethylvinylether werden 7,48 g (20,12 mmol) 2-Chlor-4-[2-(4-nitrophenyl)-ethoxycarbonyloxy]-benzylalkohol gelöst, mit 680 mg Quecksilber-(II)-bistrifluoracetat versetzt und für 6 Stunden bei Raumtemperatur gerührt. Durch Verdünnen mit 300 ml EE und Gießen auf 200 ml ges. NaHCO₃-Lösung wird die Reaktion abgebrochen. Man wiederholt das Waschen der org. Phase mit 100 ml ges. NaHCO₃-Lösung, und 100 ml ges. NaCl-Lösung. Die gesammelten wäßrigen Phasen werden mit zweimal 100 ml EE zurückextrahiert. Die vereinigten organischen Phasen werden über NaSO₄ getrocknet, filtriert und am Rotationsverdampfer auf etwa 15 ml eingeengt. Die weitere Reinigung erfolgt chromatographisch. Dazu wird eine Säule (4 cm ⌀) mit 150 g Flash-Kieselgel beschickt, mit Toluol equilibriert und nach Auftragen des Rohproduktes mit 500 ml Toluol eluiert. Man fängt das Eluat in Fraktionen zu je 50 ml auf, vereinigt die Produktfraktionen (ca. 250 ml) und engt diese am Rotationsverdampfer zum Öl ein. Nach Koevaporieren mit je 20 ml dest. MeOH wird das zähflüssige Öl im Hochvakuum bei 25°C getrocknet. Die Ausbeute liegt mit 6,78 g (17,95 mmol) bei 82 % der Theorie.
- DC (Kieselgel):: R_{f} = 0,78 (Tol/EE = 10/1)
¹H-NMR (250 MHz, d⁶-DMSO, TMS, ppm):
8,18 (m, 2 H, o-H zu -NO₂); 7,50 (d, 1 H, H-(C6)); 7,42 (d, 2 H, o-H zu -NO₂), 7,20 (d, 1 H, H-(C3)); 7,05 (dd, 1 H, H-(C5)); 6,53 (dd, 1 H, Vinyl-H); 4,78 (s, 2 H, -CH₂); 4,48 (t, 2 H, α-CH₂ (NPEE)); 4,30 (dd, 1 H, trans-Vinyl-H); 4,12 (dd, 1 H, cis-Vinyl-H); 3,18 (t, 2 H, β-CH₂ (NPEE)).

Analog werden die folgenden Verbindungen hergestellt:

### 31.1.a 3-Chlor-4-hydroxy-benzylalkohol

- Ausbeute:: 23 %
- DC (Kieselgel):: R_{f} = 0,52 (Tol/EE = 1/2)
- Schmelzpunkt:: 125°C
¹H-NMR (250 MHz, d⁶-DMSO, TMS, ppm):
10,05 (bs, 1 H, Phenyl-OH); 7,28 (s, 1 H, H-(C2)); 7,08 (d, 1 H, H-(C6)); 6,9 (d, 1 H, H-(C5)); 5,12 (bs, 1 H, Benzyl-OH); 4,35 (s, 2 H, CH₂).

### 31.1.b 2-Chlor-4-hydroxy-benzylalkohol

- Ausbeute:: 28 %
- DC (Kieselgel):: R_{f} = 0,52 (Tol/EE = 1/2)
- Schmelzpunkt:: 127°C
¹H-NMR (250 MHz, d⁶-DMSO, TMS, ppm):
9,72 (s, 1 H, Phenyl-OH); 7,29 (s, 1 H, H-(C3)); 6,83-6,65 (m, 2 H, H-(C3), H-(C5)); 5,13 (t, 1 H, Benzyl-OH); 4,45 (d, 2 H, CH₂).

### 31.1.c 2,5-Dichlor-4-hydroxy-benzylalkohol

- Ausbeute:: 33 %
- DC (Kieselgel):: R_{f} = 0,52 (Tol/EE = 1/2)
- Schmelzpunkt:: 144°C
¹H-NMR (250 MHz, d⁶-DMSO, TMS, ppm):
10,52 (s, 1 H, Phenyl-OH); 7,40 (s, 1 H, H-(C6)); 6,95 (s, 1 H, H-(C3)); 5,31 (t, 1 H, Benzyl-OH); 4,44 (d, 2 H, CH₂).

### 31.2.a 4-Methoxytrityl-(2-fluor-4-hydroxy)-benzylether

- Ausbeute:: 95 %
- DC (Kieselgel):: R_{f} = 0,38 (Tol/EE = 10/1)

### 31.2.b 4-Methoxytrityl-(3-chlor-4-hydroxy)-benzylether

- Ausbeute:: 89 % (Schaum/Harz)
- DC (Kieselgel):: R_{f} = 0,35 (Tol/EE = 10/1)

### 31.2.c 4-Methoxytrityl-(2-chlor-4-hydroxy)-benzylether

- Ausbeute:: 93 % (Schaum/Harz)
- DC (Kieselgel):: R_{f} = 0,52 (Tol/EE = 1/2)

### 31.2.d 4-Methoxytrityl-(4-hydroxy)-benzylether

- Ausbeute:: 93 % (Schaum/Harz)
- DC (Kieselgel):: R_{f} = 0,52 (Tol/EE = 1/2)
¹H-NMR (250 MHz, CDCl₃, TMS, ppm);
7,50-7,05 (m, 14 H, Phenyl-H, m-H zu Phenyl-OH); 6,83-6,65 (m, 4 H, o-H zu Phenyl-OH, o-H zu -OCH₃); 4,8-4,7 (bs, 1 H, Benzyl-OH); 4,03 (s, 2 H, CH₂); 3,73 (s, 3 H), -OCH₃).

### 31.3.a 3-Fluor-4-[2-(4-nitrophenyl)ethoxycarbonyloxy]-benzylalkohol

- Ausbeute:: 78 %
- DC (Kieselgel):: R_{f} = 0,22 (Tol/EE = 3/1)
- Schmelzpunkt:: 105°C

### 31.3.b 2-Chlor-4-[2-(4-nitrophenyl)ethoxycarbonyloxy]-benzylalkohol

- Ausbeute:: 76 %
- DC (Kieselgel):: R_{f} = 0,23 (Tol/EE = 3/1)
¹H-NMR (250 MHz, CDCl₃, TMS, ppm):
8,18 (m, 2 H, o-H zu -NO₂); 7,48 (d, 1 H, H-(C6)); 7,40 (m, 2 H, m zu NO₂); 7,14 (d, 1 H, H-(C3)); 7,05 (d, 1 H, H-(C5)); 4,72 (s, 2 H, -CH₂); 4,46 (t, 2 H, α-CH₂ (NPEE)); 3,13 (t, 2 H, β-CH₂ (NPEE)); 1,88 (bs, 1 H, Benzyl-OH ?).

### 31.3.c 2,5-Dichlor-4-[2-(4-nitrophenyl)ethoxycarbonyloxy]-benzylalkohol

- Ausbeute:: 62 %
- DC (Kieselgel):: R_{f} = 0,26 (Tol/EE = 3/1)

| | | | | |
|---|---|---|---|---|
| Analyse: C₁₆H₁₃NO₆Cl₂ (351,74): | ber. | C 49,76 | H 3,39 | N 3,63 |
| | gef. | C 50,00 | H 3,58 | N 3,60 |

¹H-NMR (250 MHz, d⁶-DMSO, TMS, ppm):
8,18 (m, 2 H, o-H zu -NO₂); 7,65-7,52 (m, 4 H, m-H zu -NO₂, H-(C3), H-(C6)); 5,63 (t, 1 H, Benzyl-OH); 4,58-446 (m, 4 H, Benzyl-CH₂, α-CH₂ (NPEE)); 3,15 (t, 2 H, β-CH₂ (NPEE)).

### 31.3.d 4-[2-(4-Nitrophenyl)ethoxycarbonyloxy]-benzylalkohol

- Ausbeute:: 67 % (Harz)
- DC (Kieselgel):: R_{f} = 0,52 (Tol/EE = 1/2)

| | | | | |
|---|---|---|---|---|
| Analyse: C₁₆H₁₅NO₆ (351,74): | ber. | C 60,57 | H 4,76 | N 4,41 |
| | gef. | C 60,93 | H 4,81 | N 4,59 |

UV (Methanol): λₘₐₓ [nm] (Ig E): 4,02 (268)
¹H-NMR (250 MHz, d⁶-DMSO, TMS, ppm):
8,20 (d, 2 H, o-H zu -NO₂); 7,68 (d, 1 H, m-H zu OCO); 7,30 (d, 2 H, m zu NO₂); 7,05 (d, 2 H, m-H zu OCO); 5,2 (t, 1 H, Benzyl-OH); 4,50-4,25 (m, 4 H, -CH₂, α-CH₂ (NPEE)); 3,13 (t, 2 H, p-CH₂ (NPEE)).

### 31.5.a 3-Chlor-4-[2-(4-nitrophenyl)ethoxycarbonyloxy]-benzyl-vinylether

- Ausbeute:: 75 % (Öl)
- DC (Kieselgel):: R_{f} = 0,70 (Tol/EE = 10/1)

| | | | | |
|---|---|---|---|---|
| Analyse: C₁₈H₁₆NO₆Cl (377,38) | ber. | C 12,33 | H 3,12 | H 2,34 |
| | gef. | C 12,43 | H 3,23 | H 4,34 |

¹H-NMR (250 MHz, d⁶-DMSO, TMS, ppm):
8,15 (m, 2 H, o-H zu -NO₂); 7,45-7,73 (m, 3 H, m zu -NO₂, H-(C2)); 7,29-72,0 (dd, 1 H, H-(C6)); 7,13 (d, 1 H, H-(C5)); 4,70 (s, 2 H, -CH₂); 4,50 (t, 2 H, α-CH₂ (NPEE)); 4,27 (dd, 1 H, trans-Vinyl-H); 4,10 (dd, 1 H, cis-Vinyl-H); 3,13 (t, 2 H, β-CH₂ (NPEE).

### 31.5.b 3-Fluor-4-[2-(4-nitrophenyl)ethoxycarbonyloxy]-benzyl-vinylether

- Ausbeute:: 87 % (Öl)
- DC (Kieselgel):: R_{f} = 0,72 (Tol/EE = 10/1)

| | | | | |
|---|---|---|---|---|
| Analyse: C₁₈H₁₆NO₆F (361,33) | ber. | C 12,33 | H 3,12 | N 2,34 |
| | gef. | C 12,43 | H 3,23 | N 4,34 |

¹H-NMR (250 MHz, d⁶-DMSO, TMS, ppm):
8,18 (d, 2 H, o-H zu -NO₂); 7,40 (m, 2 H, m zu -NO₂); 7,21-7,06 (m, 3 H, H-(C2), H-(C5), H-(C6)); 6,52 (dd, 1 H, Vinyl-H); 4,72 (s, 2 H, -CH₂); 4,50 (t, 2 H, α-CH₂ (NPEE); 4,28 (dd, 1 H, trans-Vinyl-H); 4,09 (dd, 1 H, cis-Vinyl-H); 3,13 (t, 2 H, β-CH₂ (NPEE).

### 31.5.c 2,5-Dichlor-4-[2-(4-nitrophenyl)ethoxycarbonyloxy]-benzyl-vinylether

- Ausbeute:: 84 %
- DC (Kieselgel):: R_{f} = 0,78 (Tol/EE = 10/1)
- Schmelzpunkt:: 48°C

| | | | | |
|---|---|---|---|---|
| Analyse: C₁₈H₁₅NO₆Cl₂ (412,23): | ber. | C 52,45 | H 3,67 | H 3,40 |
| | gef. | C 52,07 | H 3,73 | N 3,83 |

¹H-NMR (250 MHz, CDCl₃, TMS, ppm):
8,18 (d, 2 H, o-H zu NO₂); 7,57 (s, 1 H, H-(C6)); 7,41 (d, 2 H, m-H zu -NO₂); 7,21 (s, 1 H, H-(C6)); 6,53 (s, 1 H, (CH(Cl₂BnNPEOC); 4,78 (s, 2 H, Benzyl-CH₂); 4,53 (t, 2 H, α-CH₂ (NPEE)); 4,37/4,29 (d, 1 H, trans-Vinyl-H); 4,13 (dd, 1 H, cis-Vinyl-H); 3,17 (t, 2 H, β-CH₂ (NPEE)).

### 31.5.d 4-[2-(4-nitrophenyl)ethoxycarbonyloxy]-benzyl-vinylether

- Ausbeute:: 89 %
- DC (Kieselgel):: R_{f} = 0,88 (Tol/EE = 3/1)
- Schmelzpunkt:: 64°C

| | | | | |
|---|---|---|---|---|
| Analyse: C₁₈H₁₇NO₆ (343,33): | ber. | C 62,97 | H 4,99 | N 4,08 |
| | gef. | C 62,92 | H 5,00 | N 4,04 |

UV (Methanol): λₘₐₓ [nm] (Ig ε): 268 (4,01)
¹H-NMR (250 MHz, d⁶-DMSO, TMS, ppm):
8,18 (d, 2 H, o-H zu NO₂); 7,47-7,31 (m, 4 H, m-H zu -NO₂, m-H zu -OCO); 7,12 (m, 2 H, o-H zu -OCO); 6,52 (q, 1 H, CH (BnNPEOC); 4,73 (s, 2 H, Benzyl-CH₂); 4,48 (t, 2 H, α-CH₂ (NPEE)); 4,31/4,28 (d, 1 H, trans-Vinyl-H); 4,08 (dd, 1 H, cis-Vinyl-H); 3,15 (t, 2 H, β-CH₂ (NPEE)).

### 31.5.e 2,5-Dichlor-4-pivaloyloxy-benzyl-vinylether

- Ausbeute:: 58 % (Öl)
- DC (Kieselgel):: R_{f} = 0,81 (Tol/EE = 20/1)

| | | | | |
|---|---|---|---|---|
| Analyse: C₁₄H₁₆O₃Cl₂ (303,19): | ber. | C 12,33 | H 3,12 | N 2,34 |
| | gef. | C 12,43 | H 3,23 | N 4,34 |

¹H-NMR (250 MHz, CDCl₃, TMS, ppm):
7,53 (s, 1 H, H-(C6)); 7,15 (s, 1 H, H-(C3)); 6,53 (q, CH(Cl₂BnOEE); 4,78 (s, 2 H, Benzyl-CH₂); 4,36/4,28 (d, 1 H, trans-Vinyl-H-); 4,13 (dd, 1 H, cis-Vinyl-H); 1,38 (s, 9 H, -CH₃).

### 32.1 2'-O-1-{3-Fluor-4-[2-[4-nitrophenyl)-ethoxycarbonyloxy]-benzyloxy}-ethyl-3',5'-tetraisopropyl-disiloxan-1,3-diyl-uridin

In jeweils 30 ml absolutem Toluol werden 3,50 g (7,19 mmol) 3',5'-geschütztes Uridin gelöst und am Rotationsverdampfer zweimal koevaporiert. Dann nimmt man den Schaum in 80 ml abs. Toluol auf, fügt 3,20 g (8,86 mmol) des Vinylethers zu, der in 64 ml abs. Toluol gelöst ist und versetzt die farblose Lösung mit 10 ml (100 mg = 0,52 mmol) einer Stammlösung p-TsOH*H₂O in abs. Dioxan (1,0 g/100 ml). Nachdem über Nacht bei Raumtemperatur gerührt wurde (DC-Kontrolle !), wird die klare Reaktionslösung mit EE auf 300 ml verdünnt und mit zweimal 100 ml ges. NaHCO₃-Lösung und 100 ml ges. NaCI-Lösung ausgeschüttelt. Die wäßrigen Phasen werden mit 100 ml EE zurückextrahiert, die vereinigten organischen Phasen über NaSO₄ getrocknet, filtriert und einrotiert. Die weitere Reinigung erfolgt chromatographisch über eine Säule mit 4 cm Durchmesser, die mit 120 g Flash-Kieselgel beschickt ist und mit Toluol equilibriert wird (Laufmittelgradient (Tol/EE/MeOH in ml):
200/0, 200/20, 250/50, 10/100 (P1 +P2); Fraktionen mit jeweils 50 ml). Die Produktfraktionen werden einrotiert und mehrmals mit MeOH/CH₂Cl₂ koevaporiert.
Man erhält so 4,67 g (5,51 mmol, 77 %) farblosen Schaum, der aus beiden Diastereomeren (P1 + P2) besteht.
- DC (Kieselgel):: R_{f} = 0,60/0,65 (Tol/EE = 1/1)

| | | | | |
|---|---|---|---|---|
| Analyse: C₃₉H₅₄N₃O₁₃Si₂F (848,04) | ber. | C 55,24 | H 6,42 | N 4,96 |
| | gef. | C 55,06 | H 6,48 | N 5,07 |

UV (Methanol): λₘₐₓ [nm] (Ig ε): 265 (4,33)
¹H-NMR (250 MHz, CDCl₃, TMS, ppm):
8,93/8,73 (bs, 1H, -NH); 8,19 (m, 2H, o-H zu NO₂); 7,93/7,84 (d, 1H, H-(C6)); 7,18-6,87 (m, 3H, H-(C2), H-(C5), H-(C6)); 5,78/5,68 (s,1H, H-(C1')); 5,68-5,59 (m, 1H, H-(C5)), 5,14 (m, 1H, CH(2FPNPE); 4,78-4,46 (m, 4H, Benzyl-CH₂, α-CH₂ (NPEE)); 4,29-3,41 (m, 5H, H-(C2'), H-(C3'), H-(C4'), H-(C5'), H-(-C5"); 3,14 (t, 2H, β-CH₂ (NPEE)); 1,49 (t,2H, CH₃(2FPNPE);

### 32.1.a 2'-O-1-{3-Chlor-4-[2-(4-nitrophenyl)ethoxycarbonyloxy]-benzoyloxy}-ethyl-3',5'-tetraisopropyl-disiloxan-1,3-diyl-uridin

- Ausbeute:: 86 %
- DC (Kieselgel):: R_{f} = 0,64/0,68 (Tol/EE = 1/1)

| | | | | |
|---|---|---|---|---|
| Analyse: C₃₉H₅₄N₃O₁₃Si₂Cl (864,50) | ber. | C 54,19 | H 6,30 | N 4,86 |
| | gef. | C 54,14 | H 6,39 | N 4,88 |

UV (Methanol): λₘₐₓ [nm] (Ig ε): 264 (4,28)

### 32.1.b 2'-O-1-{2-Chlor-4-[2-(4-nitrophenyl)ethoxycarbonyloxy]-benzoyloxy}-ethyl-3',5'-tetraisopropyl-disiloxan-1,3-diyl-uridin

- Ausbeute:: 79 %
- DC (Kieselgel):: R_{f} = 0,65/0,69 (Tol/EE = 1/1)

| | | | | |
|---|---|---|---|---|
| Analyse: C₃₉H₅₄N₃O₁₃Si₂Cl (864,50) | ber. | C 54,19 | H 6,30 | N 4,86 |
| | gef. | C 54,13 | H 6,44 | N 4,82 |

UV (Methanol): λₘₐₓ [nm] (lg ε): 265 (4,29)

### 32.1.c 2'-O-1-{2,5-Dichlor-4-[2-(4-nitrophenyl)ethoxycarbonyloxy]-benzoyloxy}-ethyl-3',5'-tetraisopropyl-disiloxan-1,3-diyl-uridin

- Ausbeute:: 76 % (Schaum)
- DC (Kieselgel):: R_{f} = 0,66/0,69 (Tol/EE = 1/1)

| | | | | |
|---|---|---|---|---|
| Analyse: C₃₉H₅₄N₃O₁₃Si₂Cl (898,94) | ber. | C 52,11 | H 5,94 | N 4,67 |
| | gef. | C 52,06 | H 5,05 | N 4,57 |

### 32.1.d 2'-O-1-{4-[2-(4-Nitrophenyl)ethoxycarbonyloxy]-benzyloxy}-ethyl-3',5'-tetraisopropyl-disiloxan-1,3-diyl-uridin

- Ausbeute:: 44 % (Schaum)
- DC (Kieselgel):: R_{f} = 0,82/0,84 (Tol/EE = 1/3)

| | | | | |
|---|---|---|---|---|
| Analyse: C₃₉H₅₅N₃O₁₃Si₂ (830,05) | ber. | C 56,43 | H 6,68 | N 5,06 |
| | gef. | C 56,32 | H 6,71 | N 5,04 |

UV (Methanol): λₘₐₓ [nm] (Ig ε): 264 (4,30)

### 32.1.e 2'-O-1-{2,5-Dichlor-4-pivaloyloxybenzyloxy)-ethyl-3',5'-tetraisopropyl-disiloxan-1,3-diyl-uridin (Schaum)

- DC (Kieselgel):: R_{f} = 0,72/0,79 (Tol/EE = 1/1)

| | | | | |
|---|---|---|---|---|
| Analyse: C₃₅H₅₄N₂O₁₀Si₂Cl₂ (789,90) | ber. | C 53,22 | H 6,89 | N 3,55 |
| | gef. | C 53,87 | H 7,09 | N 3,61 |

### 32.2 2'-O-1-{3-Fluor-4-[2-(4-nitrophenyl)ethoxycarbonyloxy]-benzyloxy}-ethyl-uridin

4,33 g (5,11 mmol) 2'-O-1-{3-Fluor-4-[2-(4-nitrophenyl)ethoxycarbonyloxy]-benzoyloxy}-ethyl-3',5'-tetraisopropyldisiloxan-1,3-diyl-uridin werden in 80 ml einer Stammlösung TBAFx3H₂O (50 mg/ml)/AcOH (50 mg/ml) in Dioxan (abs.) aufgenommen und bei Raumtemperatur über Nacht gerührt. Die Lösung wird am Rotationsverdampfer (45°C) bis auf ein Restvolumen von ca. 20 ml eingeengt und das Rohprodukt auf eine Säule (d = 5 cm) aufträgt. Die Säule ist mit 150 g Flash-Kieselgel beschickt und mit Toluol equilibriert (Laufmittelgradient (Tol/EE/MeOH in ml): 200/0, 200/20, 250/50, 200/100, 150/150, 150/150/15, 15/150/30, 150/150/30m P1 + P2); Fraktionen mit jeweils 50 ml). Die Produktfraktionen werden einrotiert und mehrmals mit MeOH/CH₂Cl₂ koevaporiert.
Man erhält so 2,75 g (4,54 mmol, 89 %) farblosen Schaum, der aus beiden Diastereomeren (P1 + P2) besteht.
- DC (Kieselgel):: R_{f} = 0,45/0,47 (Tol/EE/MeOH = 5/4/1)

| | | | | |
|---|---|---|---|---|
| Analyse: C₂₇H₂₈N₃O₁₂Si₂F (605,53) | ber. | C 53,56 | H 4,66 | N 6,94 |
| | gef. | C 52,86 | H 4,83 | N 6,91 |
| x 0,5 H₂O | ber. | C 52,77 | H 4,76 | H 6,84 |

UV (Methanol): λₘₐₓ[nm] (Ig ε): 264 (4,30)
¹H-NMR (250 MHz, d⁶-DMSO, TMS, ppm):
11,28 (bs, 1H, -NH); 8,18 (m, 2H, o-H zu NO₂); 7,95/7,80 (m, 1H, H-(C6)); 7,63-7,09 (m, 5H,m-H zu NO₂, H-(C2"'), H-(C5"'), H-(C6"'); 5,91 (m, 1H, H-(C1')); 5,53 (m, 1H, H-(C5)); 5,29-5,09 (m, 2H, HO-(C3'), HO(C5'); 4,95 (m, 1H, CH(2FPNPE); 4,70-4,36 (m, 4H, Benzyl-CH₂, α-CH₂ (NPEE)); 4,26 (t, 1H, H-(C2'), 4,06 (m, 1H, H-(C3'); 3,87 (m, 1H, H(C4'); 3,78-3,48 (m, 2H, H-(C5'), H-(-C5"); 3,12 (t, 2H, β-CH₂ (NPEE)); 1,46/1,38 (d, 3H, CH₃(2FPNPE).

### 32.2.a 2'-O-1-{3-Chlor-4-[2-(4-nitrophenyl)ethoxycarbonyloxy]-benzyloxy}-ethyl-uridin

- Ausbeute:: 97 % (Schaum)
- DC (Kieselgel):: R_{f} = 0,64/0,68 (Tol/EE/MeOH = 5/4/1)

| | | | | |
|---|---|---|---|---|
| Analyse: C₂₇H₂₈N₃O₁₂Cl (621,88) | ber. | C 52,14 | H 4,54 | N 6,76 |
| | gef. | C 51,77 | H 4,56 | N 6,53 |

UV (Methanol): λₘₐₓ [nm] (lg ε): 265 (4,27)

### 32.2.b 2'-O-1-{2-Chlor-4-[2-(4-nitrophenyl)ethoxycarbonyloxy]-benzyloxy)-ethyl-uridin

- Ausbeute:: 82 %
- DC (Kieselgel):: R_{f} = 0,61/0,63 (Tol/EE/MeOH = 5/4/1)

| | | | | |
|---|---|---|---|---|
| Analyse: C₂₇H₂₈N₃O₁₂Cl (621,88) | ber. | C 52,14 | H 4,54 | N 6,76 |
| | gef. | C 51,91 | H 4,70 | N 6,76 |

UV (Methanol): λₘₐₓ [nm] (Ig ε): 264 (4,25)

### 32.2.c 2'-O-1-{2,5-Dichlor-4-[2-(4-nitrophenyl)ethoxycarbonyloxy]-benzyloxy}-ethyl-uridin

- Ausbeute:: 69 % (Schaum)
- DC (Kieselgel):: R_{f} = 0,66/0,69 (Tol/EE = 1/1)

| | | | | |
|---|---|---|---|---|
| Analyse: C₂₇H₂₇N₃O₁₂Cl₂ (656,43) | ber. | C 49,40 | H 4,15 | N 6,40 |
| | gef. | C 48,99 | H 4,31 | N 6,29 |

### 32.2.d 2'-O-1-{4-[2-(4-Nitrophenyl)ethoxycarbonyloxy]-benzyloxy}-ethyl-uridin

- Ausbeute:: 80 % gelbliche Kristalle
- DC (Kieselgel):: R_{f} = 0,65/0,66 (Tol/EE/MeOH = 5/4/1)

| | | | | |
|---|---|---|---|---|
| Analyse: C₂₇H₂₉N₃O₁₂ (587,54) | ber. | C 55,20 | H 4,97 | N 7,15 |
| | gef. | C 54,91 | H 5,03 | N 7,13 |

UV (Methanol): λₘₐₓ [nm] (Ig ε): 264 (4,30)

### 32.2.e 2'-O-1-(2,5-Dichlor-4-pivaloyloxybenzyloxy)-ethyl-uridin (Schaum)

- DC (Kieselgel):: R_{f} = 0,72/0,79 (Tol/EE = 1/1)

| | | | | |
|---|---|---|---|---|
| Analyse: C₂₃H₂₈N₂O₉Cl₂ (547,39) | ber. | C 50,47 | H 5,16 | N 5,12 |
| | gef. | C 50,31 | H 5,42 | N 5,11 |

UV (Methanol): λₘₐₓ [nm] (Ig ε): 261 (3,99)

### 32.3. 2'-O-1-{3-Fluor-4-[2-(4-nitrophenyl)ethoxycarbonyloxy]-benzyloxy}-ethyl-5'-DMTR-uridin

2,44 g (4,03 mmol) vorgetocknetes 2'-O-1-{3-Fluor-4-[2-(4-nitrophenyl)-ethoxycarbonyloxy]-benzyloxy}-ethyl-uridin werden zweimal mit je 10 ml abs. Toluol am Rotationsverdampfer koevaporiert, dann in 20 ml abs. Pyridin aufgenommen und anschließend mit 2,05 g (6,06 mmol) DMTR-Cl versetzt. Die REaktionslösung läßt man über Nacht bei Raumtemperatur rühren und bricht die Reaktion dadurch ab, indem man 10 ml MeOH zusetzt und für 20 Minuten weiterrührt. Danach wird die Lösung im Vakuum zum Öl einrotiert mit 250 ml EE aufgenommen, mit 150 ml ges. NaHCO₃-Lösung und zweimal 150 ml ges. NaCl-Lösung ausgeschüttelt. Die wäßrige Phase wird mit 150 ml EE zurückextrahiert, die vereinigten org. Phasen mit Na₂SO₄ getrocknet, filtriert und zum Öl eingeengt. Die weitere Reinigung erfolgt chromatographisch über eine Säule mit 4 cm Durchmesser, die mit 130 g Flash-Kieselgel beschickt ist und mit Toluol equilibriert wird (Laufmittelgradient (Tol/EE in ml): 300/0, 300/30, 300/50, 250/50, 210/70, 150/50, 150/100, 150/150, 100/200, Fraktionen mit jeweils 100 ml). Die Produktfraktionen werden einrotiert und mehrmals mit MeOH/CH₂Cl₂ koevaporiert. Man erhält so 3,76 g (3,98 mmol, 98 %) chromatographisch reinen farblosen Schaum, der aus beiden Diastereomeren (P1 + P2) besteht.
- DC (Kieselgel):: R_{f} = 0,57/0,59 (Tol/EE/MeOH = 5/4/1)
¹H-NMR (250 MHz, CDCl₃, TMS, ppm):
8,5 (bs, 1H, -NH); 8,18 (m, 2H, o-H zu -NO₂); 7,96/7,87 (d, 1H, H-(C6)); 7,42-7,00 (m, 14H, Phenyl-H, m-H zu -OCH₃, m-H zu -NO₂, H-(C2"'), H-(C'5"'), H-(C6"'); 6,88-6,79 (m, 4H, o-H zu -OCH₃); 6,04/5,95 (d. 1H, H-(C'); 5,27 (m, 1H, H-(C5)); 5,14 (m, 1H, CH(2FPNPE), 4,78-4,29 (m, Benzyl-CH₂, β-CH₂ (NPEE), H-(C2'), H-(C3'); 4,07 (m, 1H, H'-(C4')); 3,78 (s, 6H, -OCH₃); 3,51 (m, 2H, H-(C5'), H-(C5"); 2,74/2,58 (d, 1H, HO-(C3'); 1,46/1,40 (d, 3H, CH₃ (2FPNPE))

### 32.4 2'-O-1-{3-Fluor-4-[2-(4-nitrophenyl)ethoxycarbonyloxy]-benzyloxy}ethyl-5'-O-DMTR-uridin-3'-O-phosphorigsäure-β-cyanoethylester-N,N-diisopropylamid

600 mg (0,66 mmol) trockenes 2'-0-1-{3-Fluor-4-[2-(4-nitrophenyl)ethoxycarbonyloxy]benzyloxy}-ethyl-5'-DMTR-uridin wird in einen 50 ml Kolben gefüllt, über Nacht im Hochvakuum getrocknet und dann mit 20 ml abs. CH₃CN aufgenommen. Dazu gibt man 6 ml (2,7 mmol) einer Stammlösung Phosphitylierungsreagenz (0,45 mmol/ml abs. CH₃CN) und 6 ml einer Stammlösung Tetrazol (10 mg/ml) und rührt für weitere 12h bei Raumtemperatur. Durch Gießen auf 300 ml NaHCO₃/EE (1/1)-Lösung wird die Reaktion abgebrochen. Man trennt die Phasen im Scheidetrichter, schüttelt erneut mit 150 ml EE aus und wäscht die vereinigten organischen Phasen zweimal mit je 100 ml ges. NaCI-Lösung. Nach dem Trocknen über Na₂SO₄ und Filtrieren wird am Rotationsverdampfer zum Öl einrotiert. Die weitere Reinigung erfolgt chromatographisch. Dazu wird eine Säule mit 2 cm Durchmesser mit 20 g Flash-Kieselgel beschickt und mit Toluol konditioniert (Laufmittelgradient (Tol/EE in ml): 100/20, 210/70; Fraktionen zu je 10 ml). Die Produktfraktionen werden einrotiert und mit je 10 ml CH₂Cl₂/MeOH aufgeschäumt. Getrocknet wird bei Raumtemperatur im Hochvakuum.
Man erhält so 650 mg (0,587 mmol, 89 %) farblosen Schaum.
- DC (Kieselgel): R_{f} =: 0,65/0,57 (Tol/EE/MeOH = 5/4/1)

| | | | | |
|---|---|---|---|---|
| Analyse C₅₇H₆₄PN₃O₁₅F (1109,13): | ber | C 61,73 | H 5,82 | N 6,31 |
| | gef. | C 61,56 | H 5,82 | N 5,67 |

UV (Methanol): λₘₐₓ(nm) (lgε): 264 (4,30)
¹H-NMR (250 MHz, CDCl₃, TMS, ppm):
8,18 (m, 2H, o-H zu -NO₂); 8,09-7,84 (bs/m, 2H, -NH, H-(C6)); 7,46-7,00 (m, 14H, Phenyl-H, m-H zu -OCH₃, m-H zu -NO₂, H-(C2"'), H-(C5"'); H-(C"'6)); 6,87-6,76 (m, 4H, o-H zu -OCH₃);, 6,08/5,96 (m, 1H, H-(C1')); 5,27 (m, 1H, H-(C%)); 5,14 (m, 1H, CH(2FPNPE), 4,77-4,42 (m, Benzyl-CH₂, α-CH₂ (NPEE), H-(C2'), H-(C3'), 4,29-4,13 (m, 1H, H'-(C4')); 3,97-3,33 (m, 12H, -OCH₃, POCH₂, N-CH, H-(C5'), H-(C5")); 3,15 (t, β-CH₂ (NPEE)); 2,63-2,33 (m, 2H, CH₂-CN; 1,50/0,98 (m, 15H, CH₃(2FPNPE), CH(CH₃)₂);
³¹P-NMR (400 MHz, CDCl₃, H₃PO₄, ppm):
151,18; 150,87; 150,61; 150,36

### 33. Oligoribonucleotidsynthesen mit einheitlichen Sequenzen und intakter 2'-O-NPE-Schutzgruppe

### 33.1 Verwendung von Nucleosid-Phosphitamiden mit dem Cyanoethyl-Rest als Phosphatschutzgruppe

Ein trägergebundenes Uridin (Beispiel 27) bildet das 3'-Ende des zu synthetisierenden Oligonucleotids, d.h. nach Abspaltung der DMTR-Gruppe kann das erste Uridin-Phosphitamid (Beispiel 14) ankondensiert werden.

Die Bestimmung der schrittweisen Kondensationsausbeuten erfolgt mittels Absorptionsmessung des Dimethoxytritylkations bei 498 nm.
Die Nucleosid-Phosphitamide werden als 0,1 M Lösungen in absolutem Acetonitril bereitgestellt.
5'-HO-(UUU UUU UUU UUU)*-OH
* = 2'-0-[1-(4-Nitrophenylethoxy)ethyl] (2'-O-NPEE) geschützt

Die Kondensationszeit bei der Synthese der 12-meren Uridins beträgt 1200 Sekunden.
Nachdem die letzte 5'-O-DMTR-Gruppe abgespalten wurde, konnte die durchschnittliche Kondensationsausbeute mit 100 % berechnet werden.
Nach der Abspaltung des Oligomeren vom Träger erhält man ungefähr 46 OD₂₆₅ an 12-meren Uridin.
Die berechnete Produktgesamtausbeute liegt bei 100 %.

### 33.2 5'-HO-(AAA AAA AAA A)' *-OH (* = 2'-O-NPEE-geschützt)

Die Synthese des 10-meren Adenosins mit intakter 2'-O-NPEE-Gruppe (Verwendung von Beispiel 21) beginnt mit einem über das 3'-O-Succinat an das Trägermaterial gebundenen Adenosins (Beispiel 29). Die Kondensationszeit beträgt 1200 Sekunden.
Die mittlere Kondensationsausbeute nach 8 Kondensationen beträgt 100 %. Das 10-mere Adenosinribonucleotid wird mit intakter 5'-O-DMTR-Gruppe von Hand vom Träger abgespalten. Man erhält (photometrisch bestimmt) 24 OD₂₆₀ (Trityl on). Das erhaltene Trityl on-Rohprodukt wird mittels OPC (Oligonucleotide Purification Cartridges) gereinigt. Man erhält das gewünschte Oligomer, von eventuellen Fehlsequenzen gereinigt, in seiner Trityl off-Form (nach Abspaltung der DMTR-Gruppe mit 2 %-iger Trifluoressigsäure).

Zur OPC-Reinigung setzt man 18,7 OD₂₆₀ ein und erhält 10 OD₂₆₀ gereinigtes Ribonucleotid, was einer Ausbeute von 54 % entspricht.

### 33.3 5'-HO-(CCC CCC CCC C)*-OH (* = 2'-O-NPEE-geschützt)

### Aus Beispiel 17 und 28.

Die Kondensationszeit für das 10-mere Cytidinribonucleotid beträgt 1200Sekunden. Da man das Ribonucleotid in seiner Trityl on-Form nach Abspaltung vom Träger erhält, können nur 8 Kondensationen zur Bestimmung der mittleren Kondensationsausbeute herangezogen werden. Sie beträgt 91 %. Die berechnete Produktausbeute liegt bei 74 % bezogen auf die erste Kondensation.
Nach der ammoniakalischen Trägerabspaltung erhält man 17,5 OD₂₇₀ Rohprodukt. Das Rohprodukt wird einer OPC-Reinigung unterzogen. Man erhält es anschließend in seiner Trityl off-Form.

### 33.4 5'-HO-(GGG GGG GGG G)*-OH (* = 2-O-NPEE geschützt)

### Aus Beispiel 30 und 26.

Bei dieser Synthese wird die Kondensationszeit ab der fünften Kondensation auf 700 Sekunden reduziert.
Die mittlere Kondensationsausbeute beträgt 93 %.

Ausgehend von der ersten Kondensation erhält man eine Produktausbeute von 52 %.
Man erhält 20 - 23 OD₂₇₁ an Trityl on-Ribonucleotid nach der Trägerabspaltung.
Das Rohprodukt wird mittels OPC gereinigt. Das Trityl off-Oligomer wird in 60 % Ausbeute (12,8 OD₂₇₁) erhalten.

### 34. Verwendung von Nucleosid-Phosphitamiden mit dem NPE-Rest als Phosphatschutzgruppe

Bei allen nachfolgend beschriebenen Synthesen werden Nucleosid-Phosphitamide mit der NPE-Phosphatschutzgruppe verwendet.

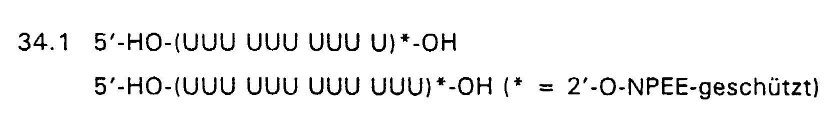

### Verwendung von Beispiel 27 und 18.

Bei beiden oben gezeigten Sequenzen werden Kondensationszeiten von 1200 Sekunden verwendet.
Die mittleren Kondensationsausbeuten liegen beim 10-meren Uridin bei 98 % beim 12-meren Uridin bei 99 %.
Die berechneten Produktausbeuten liegen bei 83 % bzw. 88 %.
Man erhält beide Oligomere nach der Trägerabspaltung in der Trityl off-Form. Beim 10-meren Uridin beträgt die Rohausbeute 31,6 OD₂₆₅, beim 12-meren 33,8 OD₂₆₅.

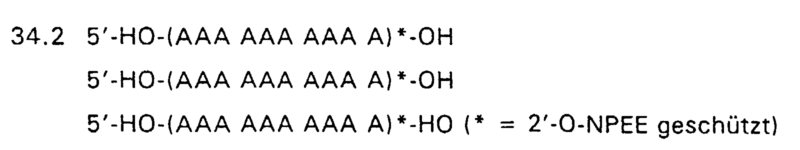

Es wurden drei 10-mere Adenosinribonucleotide mit drei verschiedenen Chargen an Adenosin-Phosphitamid synthetisiert. Verwendung von Beispiel 29 und 22. Die mittleren Kondensationsausbeuten liegen bei 95 % (Verbindung 2) bzw. 98 % (Verbindungen 1 + 3).

Die Kondensationszeit beträgt jeweils 1200 Sekunden.
Die letzte Sequenz wurde mittels OPC gereinigt.

### 34.3 5'-HO-(CCC CCC CCC C)*-OH (* = 2'-O-NPEE-geschützt)

### Verwendung von Beispiel 28 und 18.

Die durchschnittliche Kondensationsausbeute nach 8 Kondensationen beträgt 89 %. Das Rohprodukt erhält man in seiner Trityl on-Form nach ammoniakalischer Trägerabspaltung. Man erhält 18,1 OD₂₇₃ die einer OPC-Reinigung unterzogen werden. Das gereinigte Trityl off-Cytidinribonucleotid erhält man in 77 % Ausbeute (14,0 OD₂₇₃).

### 35. Polyacrylamid-Gelelektrophorese (PAGE) der 2'-O-NPEE geschützten RNA-Sequenzen

Das analytische Polyacrylamidgel (PAGE) wird unter denaturierenden Bedingungen durchgeführt.
Zunächst werden die Gelelektrophoreseplatten mit 20 %igem Trimethylsilylchlorid in Dichlormethan silyliert.
Zur Darstellung der 20 %-igen PAGE werden 0,21 mmol Harnstoff, 1 ml Wasser, 3 ml Gelelektrophorese-Puffer und 15 ml 40 %-ige Acrylamidlösung im warmen Wasserbad gelöst und anschließend im Eisbad abgekühlt. Nach Zugabe von 1 ml 1,6-%iger Ammoniumpersulfatlösung und 20 ml Tetramethylethylendiamin (TEMED) gießt man die Lösung zur Polymerisation zwischen die Glasplatten. Als Elektrolyt dient Gelelektrophorese-Puffer in 10-facher Verdünnung.

Das Gelelektrophorese-Puffer stellt man her, indem man 0,89 mol Tris(hydroxymethyl)aminomethan, 0,89 mol Borsäure und 23 mmol Na₂EDTA·2 H₂O in einem Liter Wasser löst und pH 8 einstellt.

Nach dem Auftragen der Proben in die dafür vorgesehenen Kammern, wird das Gel drei Stunden bei 300 Volt entwickelt. Die Oligonucleotidbanden werden nach der Stains all-Methode angefärbt und sichtbar gemacht.

Aufgrund der Tatsache, daß die HPLC-Chromatogramme der 2'-O-NPEE-geschützten Oligoribonucleotide (RP-18-Säule) keine eindeutige Aussage bezüglich ihrer Reinheit zulassen, soll hier an dieser Stelle die PAGE der einzelnen Ribonucleotide diskutiert werden.

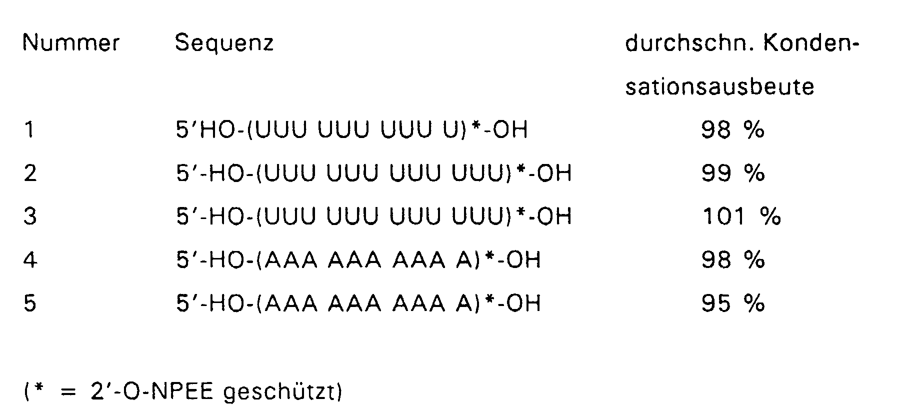

Die Sequenzen 1, 2 und 5 wurden mit Nucleosid-Phosphitamiden synthetisiert, die den NPE-Rest als Phosphatschutzgruppe tragen. 3 und 4 mit solchen, die als Phosphatschutz die Cyanoethyl-Gruppe besitzen. Wie bereits aus den durchschnittlichen Kondensationsausbeuten von Sequenz 1 bis 4 zu erwarten ist, sollten sehr wenig bzw. keine Fehlsequenzen auftreten. Dies wird durch die PAGE bestätigt. Man erhält jeweils eine Bande für die Sequenzen 1 bis 4.
Ebenso bei Sequenz 5, trotz der etwas schlechteren Kondensationsausbeute von 95 %.
Bei den Sequenzen 1 bis 5 handelt es sich um Rohprodukte (Trityl off) nach der Trägerabspaltung. Es wurden bei 1 bis 5 keine weiteren Reinigungsschritte unternommen.

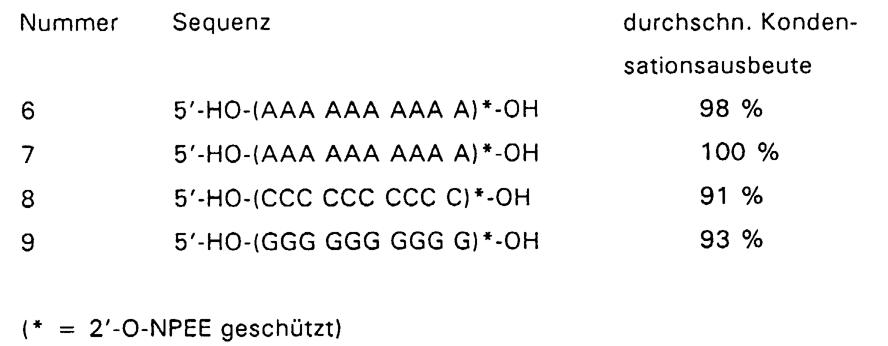

Die Sequenz 6 wurde mit einem Phosphitamid synthetisiert, das den NPE-Rest als Phosphatschutzgruppe trägt. Sequenzen 7 bis 9 wurden mit Phosphitamiden dargestellt, welche die Cyanoethyl-Gruppe als Phosphatschutz aufweisen. 6 bis 9 wurden nach der ammoniakalischen Trägerabspaltung mittels OPC gereinigt. Man erhält sie in ihrer Trityl off-Form.

Auch hier wiederum zeigt die PAGE eine außerordentlich hohe Reinheit der synthetisierten Oligoribonucleotide 6 bis 9. In allen Fällen erhält man ausschließlich eine Bande, d.h. 6 bis 9 sind frei von Fehlsequenzen.

5′-HO-(CUC CGG UUC GAU UCC GGA CUC GUC CAC CAU)*-OH
5′-HO-(UUG GCG UGG AGA GGU CUC CGG UUC GAU UCC GGA CUC GUC
CAC CAG)*-OH (* = 2′-O-NPEE geschützt)

Verwendung von Beispiel 27, 30, 14, 21, 17, 26.

Die Kondensationszeit beträgt bei beiden Sequenzen 700 Sekunden. Man erhält jeweils mittlere Kondensationsausbeuten von 99 %. Bei beiden Synthesen erhält man Produktausbeuten von 45 %.

Das 30-mere Oligomer wird nach der Trägerabspaltung in seiner Trityl off-Form in 95 OD₂₆₄ erhalten, das 45-mer als Trityl on-Verbindung in 85 - 93 OD₂₆₃.

Das 45-mere Oligonucleotid (17,0 OD₂₆₃) wird in einer OPC-Reinigung unterzogen. Man erhält das gereinigte Produkt in Trityl off-Form in 8,6 OD₂₆₃ (51 % Ausbeute).

### 37. Abspaltung der 2'-O-NPEE-Schutzgruppe von den synthetisierten RNA-Sequenzen

1. Pro 1,0 OD an Oligonucleotid werden 100 µl an 4 %ige pTsOH (Methylenchlorid/Methanol 4:1) zugegeben.
   Nach einer Reaktionszeit von zwei Stunden wird die jeweilige Probe mit 0,84 M Ammoniak/Methanol-Lösung neutralisiert und am Speed vac einrotiert.
2. In einem ersten Reinigungsschritt werden die freien RNA-Fragmente mit Natriumacetat aus Ethanol als Na⁺-Salze ausgefällt.
   Pro 1,0 OD Oligoribonucleotid gibt man 10 bis 20 µl 0,1 M Natriumacetatlösung pH 7 zu. Mit einem 4 bis 5-fachen Volumen an Ethanol fällt man die RNA über Nacht bei -40°C aus.
3. Die resultierenden Na⁺-Salze der RNA werden mittels RP-18-Chromatographie gereinigt.
   Die Produktpeaks werden aufgefangen und am Speed vac einrotiert.

Die nach diesem Abspaltungsvorgang resultierenden freien Oligoribonucleotide werden nun mittels HPLC (RP-18) und PAGE auf ihre Reinheit überprüft.

Untersuchungen der freien Oligoribonucleotide.

Die analytischen Untersuchungen der völlig entschützten Oligoribonucleotide werden an reversed-phase-Material (RP-18) durchgeführt.
Die mobile Phase besteht aus einem Acetonitril/Puffersystem (1:1) und 0,1 M Triethylammoniumacetat-Puffer pH 7.
Die RP-18-Säule wird mit einem Lineargradienten von 2,5 % bis 50 % Acetonitril innerhalb von 52 Minuten eluiert.
Das Puffersystem wird mit sterilem Wasser bereitet.
- Experimentelle Konstanten:: Merck-Hitatchi-I-6200 Intelligent Pump 100 RP-18, 5 µm,
l = 125 mm, d = 4 mm.

### Mobile Phase:

| Zeit (min) | % A | % B |
|---|---|---|
| 0 | 5 | 95 |
| 2 | 5 | 95 |
| 32 | 40 | 60 |
| 52 | 100 | 0 |
| 55 | 100 | 0 |
| 60 | 5 | 95 |

Folgende Sequenzen wurden entschützt:

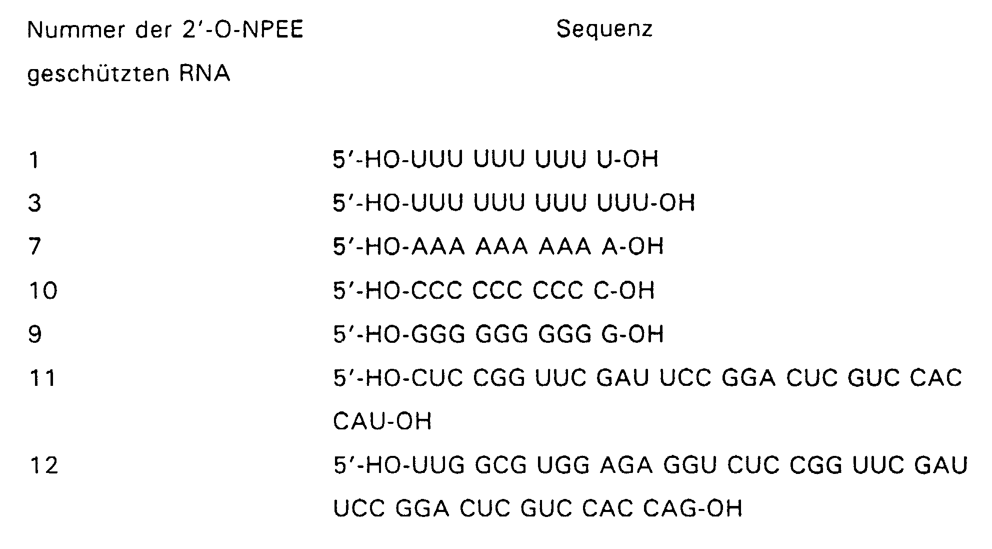

Die HPLC-Chromatogramme zeigen in allen Fällen einen einheitlichen Peak, was zu der Schlußfolgerung Anlaß gibt, daß die gereinigten, völlig entschützten RNA-Fragmente eine hohe Reinheit aufweisen.

### 38. Stabilitätsprüfung

Zur Stabilitätsprüfung wurden folgende Uridin-Derivate in 0,05N HCI/MeOH (1:2), 30 mmolare Lösung, 20°C, pH 2.0 (Tabelle 1) bzw. in 80 % Essigsäure, 30 mmolare Lösung, 20°C (Tabelle 2) inkubiert und dei Halbwertzeit für die Entschützung bestimmt:

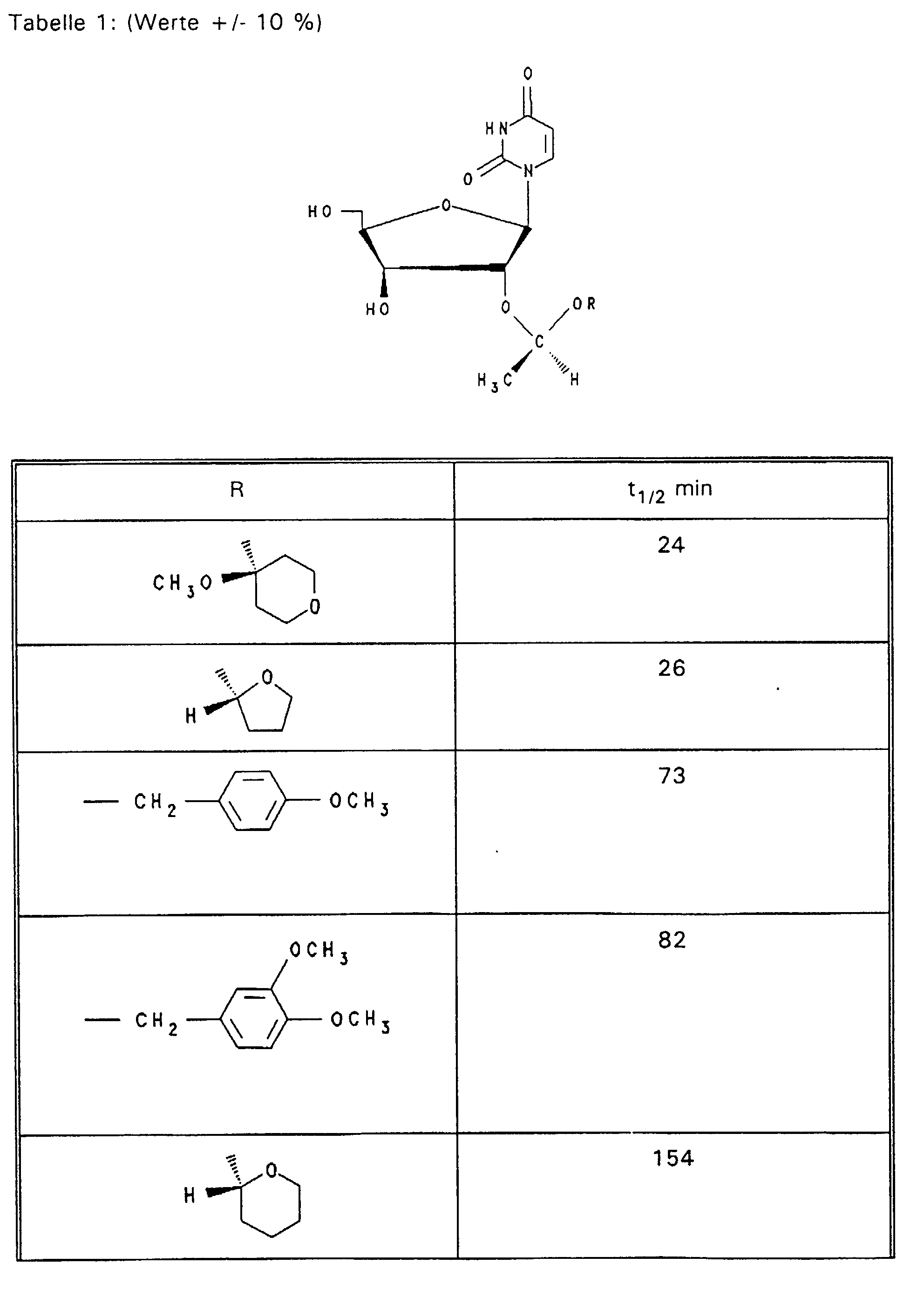

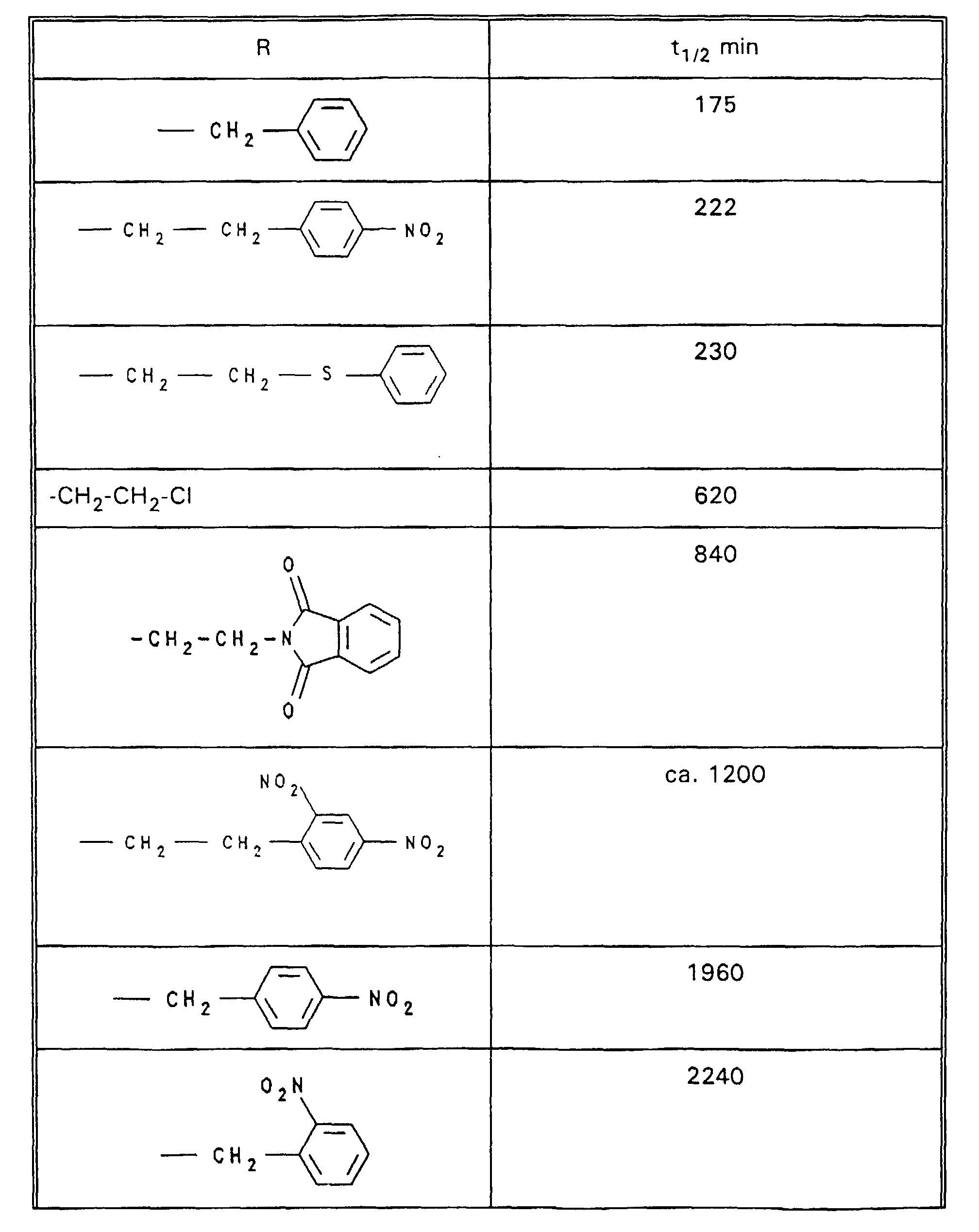

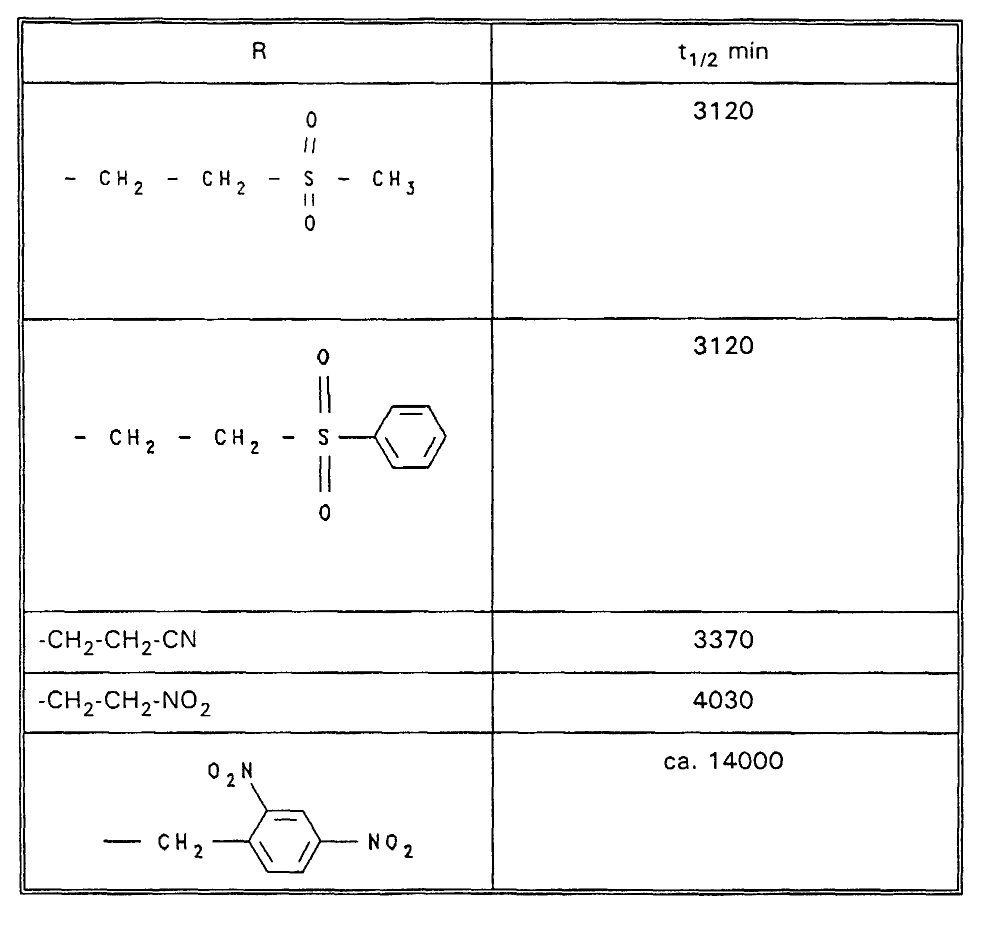

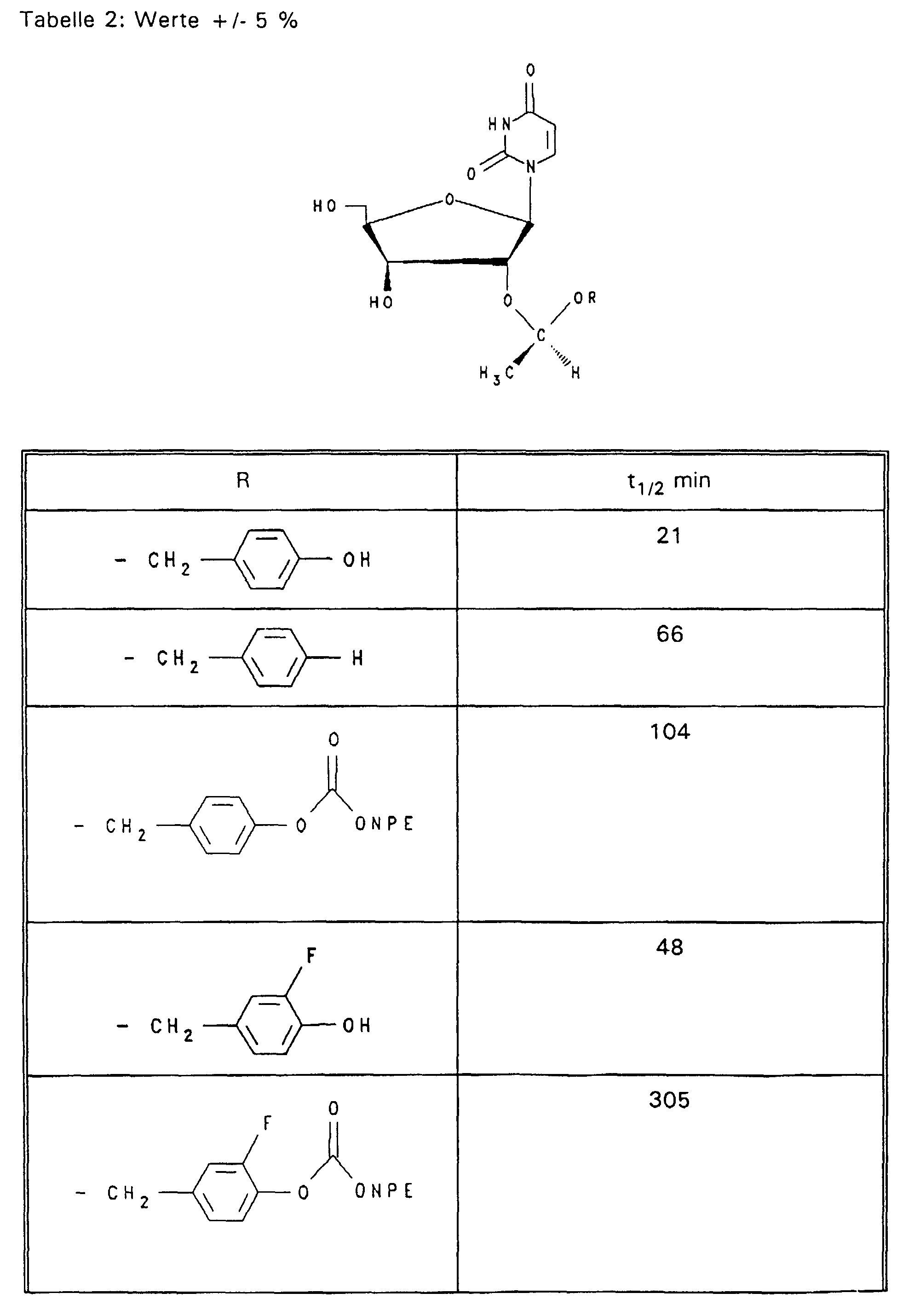

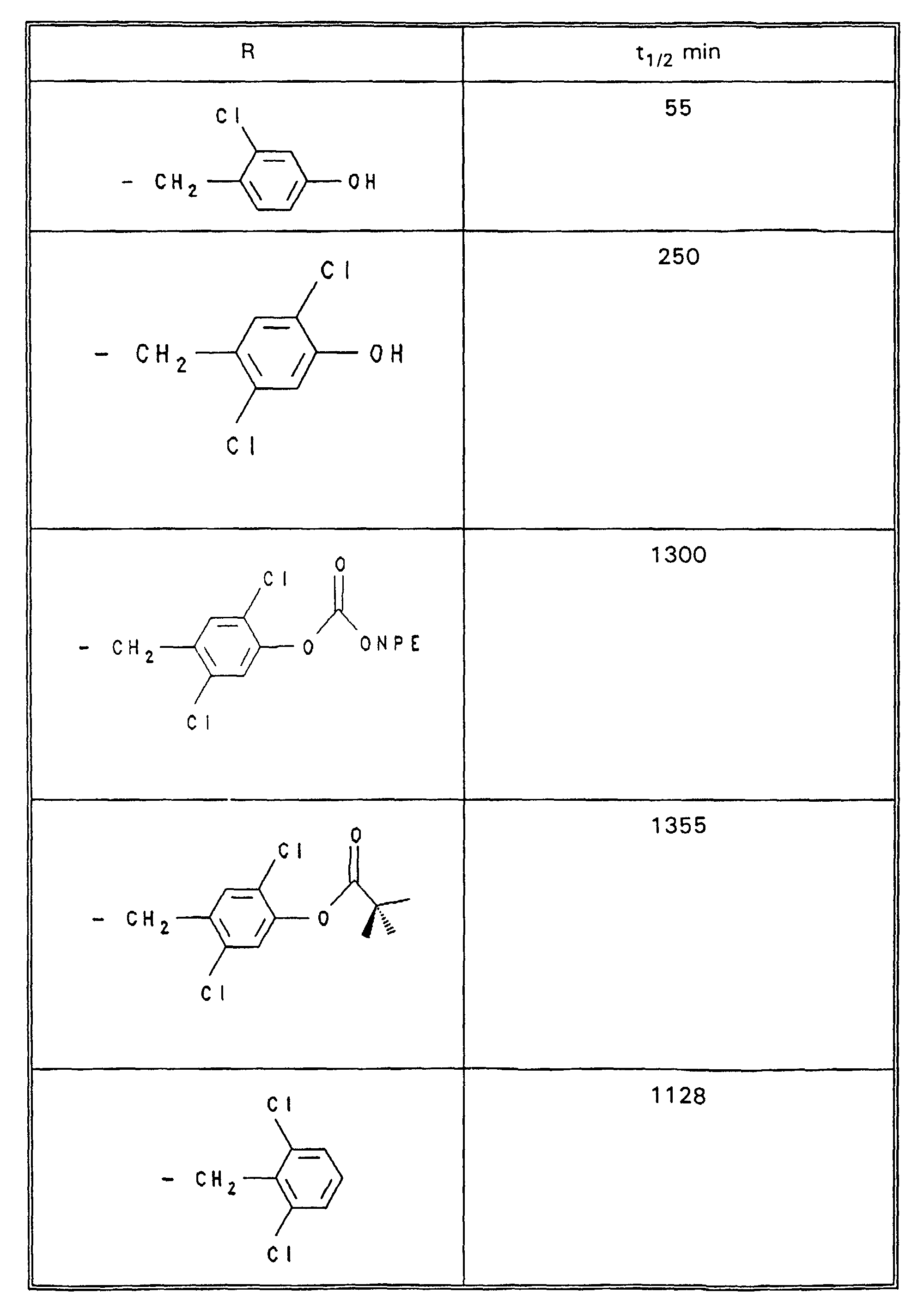

### Abkürzungen:

- 4-DMAP: 4-Dimethylaminpyridin
- DMTR: Dimethoxytrityl
- NPE: Para-Nitrophenylethoxy
- NPEE: 1 -(para-Nitrophenylethoxy)ethyl
- NPEOC: Para-Nitrophenylethoxycarbonyl
- TBDMS: Tert.-butyldimethylsilyl

## Patentansprüche

1. Verfahren zur Herstellung einer Verbindung der Formel I 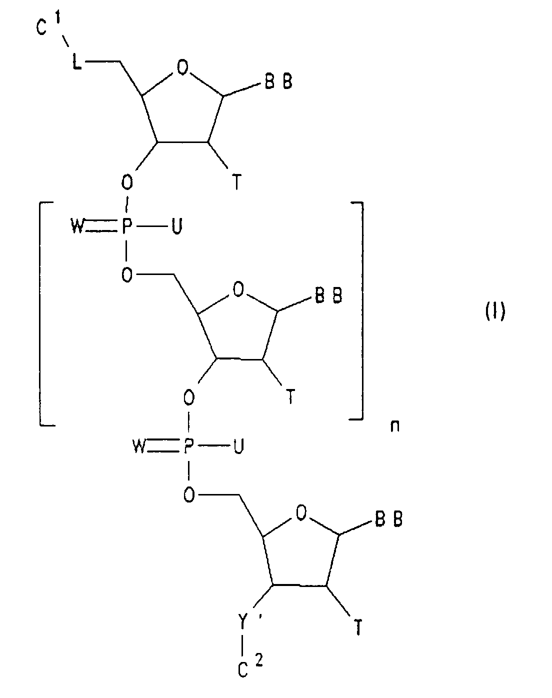 worin
n eine Zahl von 1-150 ist;
L Oxy, Sulfandiyl oder Imino bedeutet;
BB unabhängig voneinander 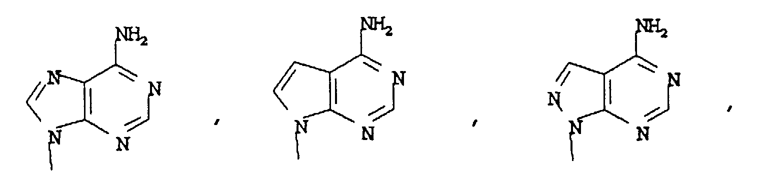 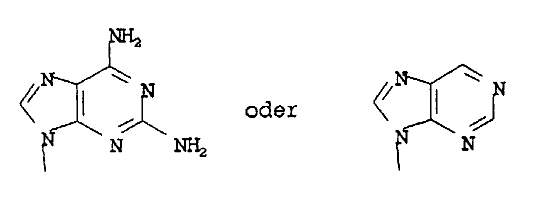 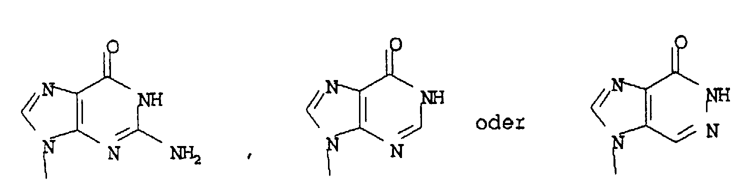 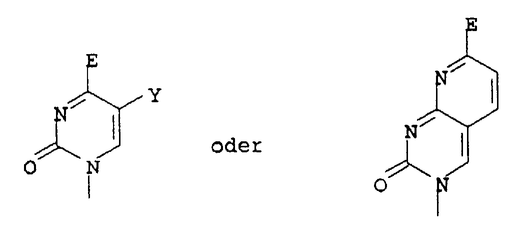 ist, und
E für OH oder NH₂ und
Y für Wasserstoff, C₁-C₄-Alkyl, Fluor, Chlor, Brom oder C₂-C₆-Alkenyl, C₂-C₆-Alkinyl stehen; oder
BB kann neben den natürlichen Nucleosid-Basen auch für andere modifizierte Nucleosid-Basen, wie 5-(Hydroxymethyl)uridin, 5-Aminouridin, Pseudouridin, Dihydrouridin, Inosin, 8-Aza-7-deazaadenosin, Tubercidin, Nebularin, Xanthosin, 2-Aminoadenosin-, Etheno-Adenosin, 7-DeazaGuanosin, O4-Methylthymidin, N6-Methyladenosin, O6-Methylguanosin oder Pyridopyrimidin-Nucleoside stehen;
W unabhängig voneinander Sauerstoff oder Schwefel bedeutet;
T unabhängig voneinander für Wasserstoff, -OCH₃, -O-CH₂CH₃, -O-CH₂-CH = CH₂ oder OH, mindestens jedoch einmal für OH steht;
Y' für Oxy, Sulfandiyl, Imino, (CH₂)ₖ oder N(CH₂)ₖ steht, wobei k eine ganze Zahl von 1 bis 18 bedeutet;
U für Hydroxy, Mercapto, SeH, C₁-C₁₈-Alkoxy, C₁-C₁₈-Alkyl, C₆-C₂₀-Aryl, C₆-C₁₄-Aryl-C₁-C₈-alkyl, NHR¹⁷, NR¹⁷R¹⁸ oder einen Rest der Formel (OCH₂CH₂)_{c}O(CH₂)_{c'}CH₂R²⁰ steht, wobei
R¹⁷ C₁-C₁₈-Alkyl, C₆-C₂₀-Aryl, C₆-C₁₄-Aryl-C₁-C₈-alkyl, -(CH₂)_{d}-[NH(CH₂₋)_{d}]_{d'}-NR¹⁹R¹⁹, worin
d eine ganze Zahl von 2 bis 6 und
d' eine ganze Zahl von 0 bis 6 sind, und
R¹⁸ C₁-C₁₈-Alkyl, C₆-C₂₀-Aryl, C₆-C₁₄-Aryl-C₁-C₈-alkyl steht oder im Falle von NR¹⁷R¹⁸ zusammen mit R¹⁷ und dem sie tragenden Stickstoffatom einen 5-6-gliedrigen heterocyclischen Ring bedeutet, der zusätzlich ein weiteres Heteroatom aus der Reihe O, S, N enthalten kann;
R¹⁹ unabhängig voneinander Wasserstoff, oder C₁-C₆-Alkyl oder C₁-C₄-Alkoxy-C₁-C₆-alkyl ist;
c eine ganze Zahl von 1 bis 100;
c' eine ganze Zahl von 0 bis 18;
R²⁰ Wasserstoff oder eine funktionelle Gruppe wie Hydroxy, Amino, NHR¹⁷, COOH, CONH₂, COOR²¹, oder Fluor, Chlor oder Brom, bedeutet, mit R²¹ C₁-C₄-Alkyl, sind;
C¹ und C² gleich oder verschieden sind und für Wasserstoff, C₁-C₁₈-Alkyl, C₂-C₁₈-Alkenyl, C₂-C₁₈-Alkinyl, C₁-C₁₈-Alkylcarbonyl, C₂-C₁₈-Alkenylcarbonyl, C₂-C₁₈-Alkinylcarbonyl, C₆-C₂₀-Aryl, C₆-C₁₄-Aryl-C₁-C₈-alkyl, einen Rest der Formel II stehen, 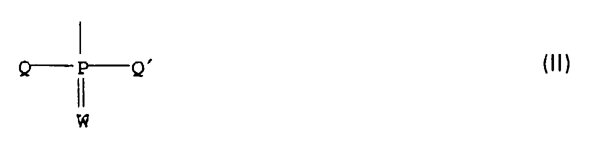 wobei
W wie oben definiert ist;
Q und Q' unabhängig voneinander für Hydroxy, Mercapto, SeH, C₁-C₂₂-Alkoxy, -O-(CH₂)_{b}-NR¹⁵R¹⁶ stehen, mit
b 1 bis 6, und
R¹⁵ und R¹⁶ unabhängig voneinander Wasserstoff, C₁-C₁₈-Alkyl, C₆-C₂₀-Aryl, C₆-C₁₄-Aryl-C₁-C₈-alkyl, C₆-C₁₄-Aryl-C₁-C₈-alkoxy, wobei Aryl auch Heteroaryl bedeutet und Aryl gegebenenfalls durch 1, 2, oder 3 gleiche oder verschiedene Reste aus der Reihe Carboxy, Amino, Nitro, C₁-C₄-Alkylamino, C₁-C₆-Alkoxy, Hydroxy, Fluor, Chlor, Brom und Cyano substituiert ist, C₁-C₁₈-Alkylmercapto, NHR¹⁷, NR¹⁷R¹⁸, wobei R¹⁷ und R¹⁸ wie oben definiert sind, oder zusammen mit dem sie tragenden Stickstoffatom einen 3-6 gliedrigen Ring bilden, oder
Q und Q' stehen für einen Rest der Formel III 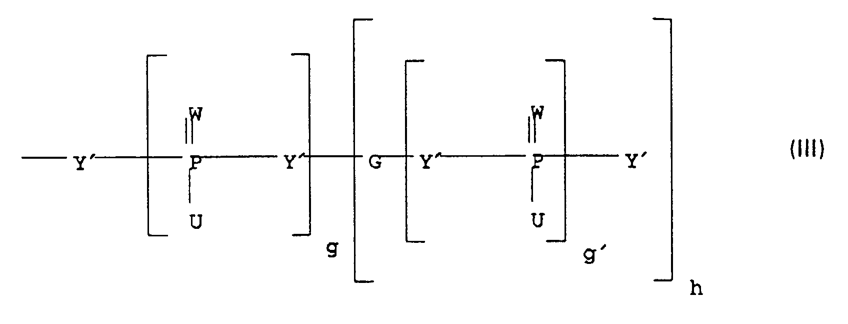 mit
g und g' = 0 oder 1,
h = 0 bis 10,
G C₂-C₁₂-Alkylen, C₆-C₁₄-Aryl-di-C₁-C₈-alkylen, C₆-C₁₈-Arylen, gegebenenfalls durch Fluor, Chlor, Brom, Amino, Hydroxy, C₁-C₁₈-Alkyl, C₁-C₁₈-Alkoxy, C₁-C₁₈-Alkoxycarbonyl, C₆-C₁₄-Aryl, C₆-C₁₄-Aryl-C₁-C₁₈-alkyl, oder C₆-C₁₄-Aryl-C₁-C₈-alkoxy ein- bis dreifach substituiert sein kann, einer Gruppe der Formel (CH₂CH₂N')ᵢCH₂CH₂ oder (CH₂N')ᵢCH₂, worin
i eine ganze Zahl von 1 bis 11, ist und
N' für Oxy, Sulfandivl, Imino oder Methylen steht und
W, U und Y' sind wie oben definiert; oder
Q und Q' bedeuten eine Gruppe, die die intrazelluläre Aufnahme begünstigt oder als Markierung einer DNA-Sonde dient oder bei der Hybridisierung des Oligonucleotidanalogons an die Target-Nucleinsäure diese unter Quervernetzung oder Spaltung angreift, wobei in Formel I die Nucleinsäuresequenz auch ein- oder mehrfach durch Linker der Formel III unterbrochen sein kann und Konjugate nach bekannten Verfahren auch über die Nucleinbasen oder über das Phosphodiester- oder Phosphothiodiester-Backbone gebildet werden können;
dadurch gekennzeichnet, daß man
a) eine Verbindung der Formel IV 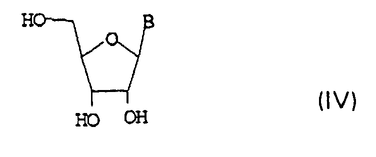 worin
B steht für 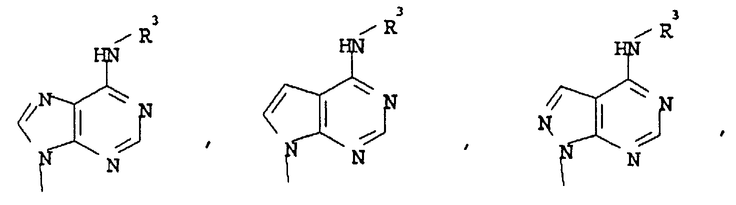 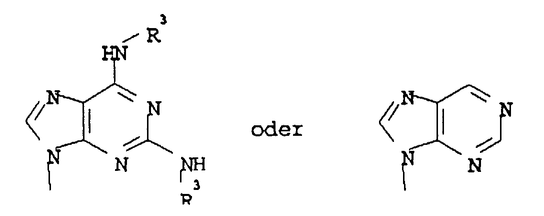 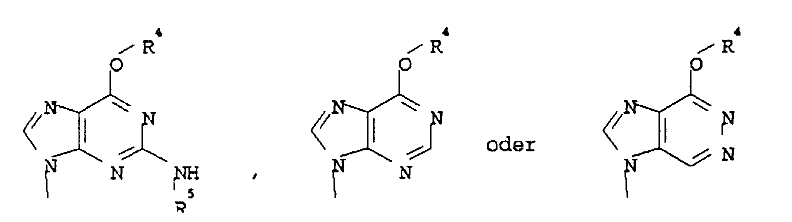 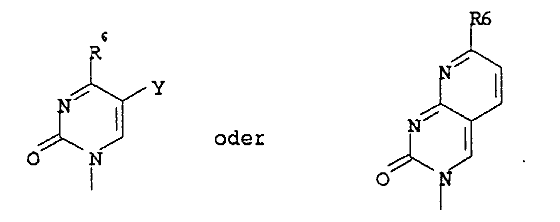 worin
R³ jeweils unabhängig voneinander eine Gruppe der Formel 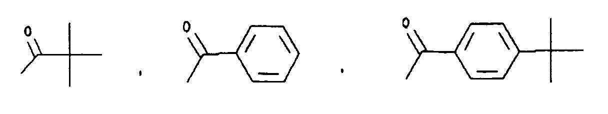 oder 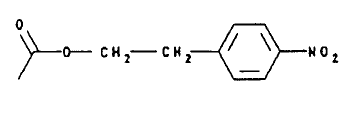 bedeutet;
R⁴ für Wasserstoff oder 2-(p-Nitrophenylethyl) steht;
R⁵ 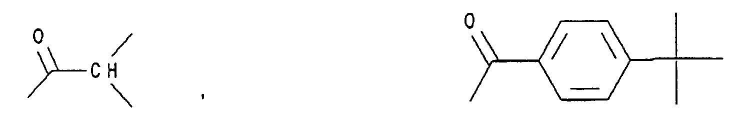 oder 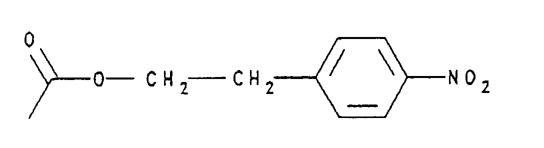
R⁶ OH, NH₂, 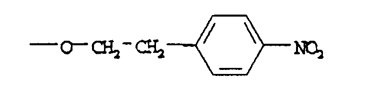 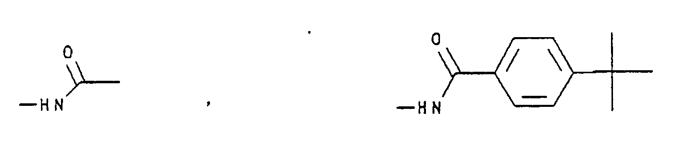 oder 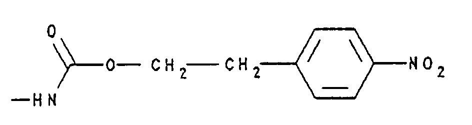 bedeuten;
Y wie oben definiert ist; oder
B kann neben den natürlichen Nucleosid-Basen auch für andere modifizierte Nucleosid-Basen, deren Amino- bzw. Hydroxygruppen durch geeignete, bekannte Schutzgruppen, wie die para-Nitrophenylethyloxycarbonyl-Gruppe, die Benzoyl-Gruppe und die para-(t-Butyl)benzoylgruppe für die Hydroxygruppe und die Benzoyl-, para-(t-Butyl)benzoyl-, para-Nitrophenylethyloxycarbonyl-, Isobutyryl-, para(tert-Butyl)phenylacetyl-Gruppe für die Aminogruppe, geschützt werden stehen,
in 3' und 5'-Position nach bekannten Verfahren schützt;
b) anschließend die geschützte Verbindung mit dem Vinylether der Formel V umsetzt, 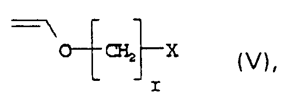 worin
r 1 oder 2 bedeutet und
X für C₆-C₁₂-Aryl steht, wobei Aryl ein- oder mehrfach mit Hydroxy, Mercapto, Nitro, Fluor, Chlor, Brom, C₁-C₆-Alkyl, C₁-C₆-Alkoxy, C₁-C₆-Alkylmercapto, C₂-C₆-Alkenyl, C₃-C₆-Alkinyl, C(O)OH, C(O)NH₂, C(O)O-C₁-C₁₈-Alkyl, C(O)O-C₆-C₁₂-Aryl, C(O)-C₁-C₁₈-Alkyl, C(O)-C₆-C₁₂-Aryl, O-C(O)NH₂, O-C(O)O-C₁-C₁₈-Alkyl, O-C(O)O-C₆-C₁₂-Aryl, O-C(O)-C₁-C₁₈-Alkyl, O-C(O)-C₆-C₁₂-Aryl, O-C(O)-[CH₂]ᵣ-X¹ oder O-C(O)O-[CH₂]ᵣ-X¹ substituiert sein kann, mit X¹ = C₆-C₁₂-Aryl, das gegebenenfalls ein- bis dreifach durch Amino, Hydroxy, Nitro, C₁-C₆-Alkyl, C₁-C₆-Alkoxy, C₁-C₆-Alkoxycarbonyl, Fluor, Chlor oder Brom substituiert sein kann;
für r = 1 steht X weiterhin für Phenyl oder C₁-C₄-Alkoxy-phenyl;
für r = 2 steht X weiterhin für CN, S-Phenyl, SO₂-Phenyl, N-Phthalimid oder NO₂;
in Analogie zu bekannten Verfahren umsetzt und die anschließende Aufarbeitung ebenfalls nach bekannten Verfahren durchführt;
c) die 5'- und 3'-Schutzgruppen, ebenfalls in Analogie zu bekannten Verfahren wieder abspaltet;
d) die 5'-Schutzgruppe mit
R² = Dimethoxytrityl, Monomethoxytrityl, Pixyl oder Trityl,
nach bekannten Verfahren eingefügt, wobei man eine Verbindung der Formel VI erhält 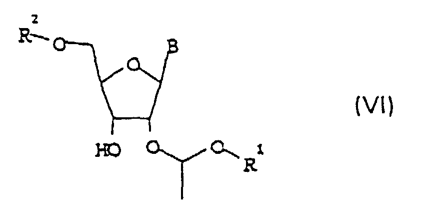 worin
R¹ für -[CH₂]ᵣ-X steht, wobei r und X wie oben definiert sind;
R² für Dimethoxytrityl, Monomethoxytrityl, Pixyl oder Trityl steht; und
B wie oben definiert ist;
e) die Verbindung der Formel VI mit einer Verbindung der Formel VII umsetzt 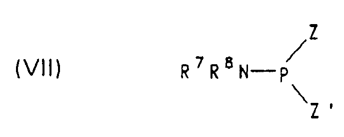 worin
Z' für OR⁹ oder C₁-C₁₈-Alkyl, C₁-C₁₈-Alkoxy, C₆-C₂₀-Aryl, C₆-C₁₄-Aryl-C₁-C₈-Alkyl steht;
R⁷ und R⁸ gleich oder verschieden sind und C₁-C₈-Alkyl oder C₅-C₁₂-Cycloalkyl, Benzyl oder Phenyl oder zusammen mit dem Stickstoffatom, an das sie gebunden sind, einen gesättigten oder ungesättigten heterocyclischen Ring, mit gegebenenfalls weiteren Heteroatomen und Substituenten bedeuten; und
R⁹ für eine Gruppe der Formel 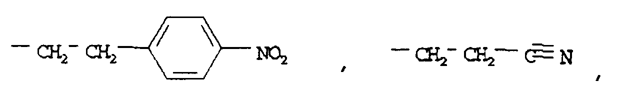 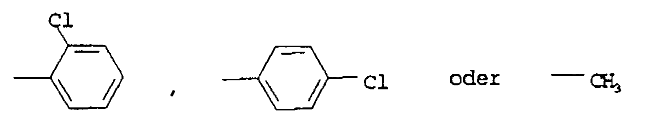 oder eine Benzylgruppe, welche nicht oder ein- bis vierfach ringsubstituiert ist, wobei der oder die Substituenten unabhängig voneinander Fluor, Chlor, Brom, eine C₁-C₄-Alkyl-, Nitro-, Methoxy-, oder Carboxylgruppe ist, steht;
Z steht für Chlor oder Brom oder einen Rest der Formel NR⁷R⁸, wobei R⁷ und R⁸ wie oben definiert sind;
in Gegenwart einer Base mit einem C₁-C₄-Trialkylamin, oder, wenn Z ein Rest der Formel NR⁷R⁸ ist, dann in Gegenwart einer Verbindung der Formel [HNR¹²R¹³R¹⁴]⁽⁺⁾ A⁽⁻⁾, wobei R¹², R¹³, R¹⁴ gleich oder verschieden voneinander sind und eine C₁-C₄-Alkylgruppe und A = Fluor, Chlor, Brom bedeuten, oder Tetrazol zu einer Verbindung der Formel VIII umsetzt 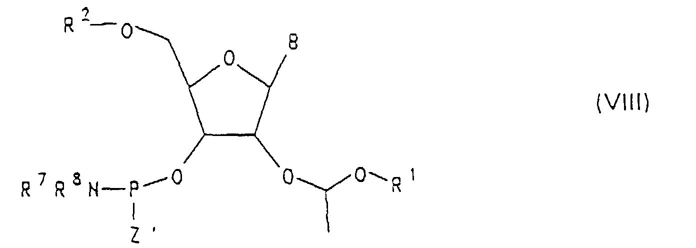
worin R¹, R², R⁷, R⁸, Z' und B wie oben definiert sind;
f) die nach d) erhaltene Verbindung VI nach bekannten Verfahren mit 1 bis 10 Equivalenten eines Linkers, wie Bernsteinsäureanhydrid, in einem geeigneten organischen Lösungsmittel, gegebenenfalls nach Zugabe eines Katalysators, zu einer Verbindung der Formel IX 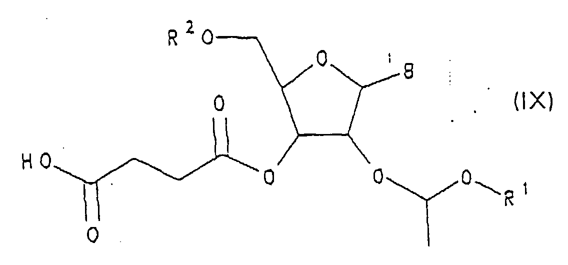 wobei R¹, R² und B wie oben definiert sind, umsetzt und anschließend nach bekannten Verfahren aufarbeitet, wobei der Bernsteinsäure-Rest in 3'-Position als Linker zu dem in der Synthese eingesetzten Polymerträger dient und alternativ zum Bernsteinsäurelinker auch andere Linker verwendet werden können;
g) die Verbindung der Formel X 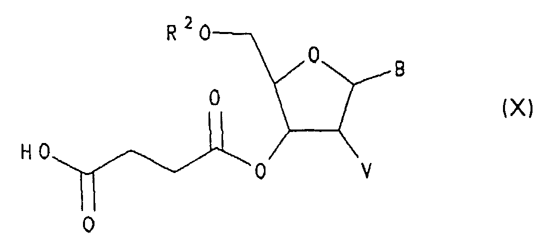 worin
R² und B wie oben definiert sind;
V für Wasserstoff, O-C₁-C₁₈-Alkyl, O-C₁-C₁₈-Alkenyl, O-C₁-C₁₈-Alkinyl oder -O-CH(CH₃)-OR¹, worin R¹ wie oben definiert ist, steht,
nach bekannten Verfahren auf den festen Träger koppelt;
h) die 5'-Schutzgruppe nach bekannten Verfahren abspaltet;
i) die erhaltene Verbindung mit einer Verbindung der Formel XI 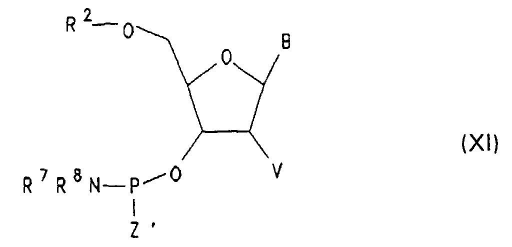 umsetzt, in der R², R⁷, R⁸, V, Z' und B wie oben definiert sind und
j) die erhaltene Verbindung nach bekannten Verfahren oxidiert;
k) die Reaktionsschritte h-j bis zur gewünschten Kettenlänge wiederholt;
l) das Oligonucleotid nach bekannten Verfahren vom Träger abspaltet und die Schutzgruppen am Phosphat und Nucleobasen ebenfalls nach bekannten Verfahren abspaltet, wobei man die Verbindung der Formel XII erhält 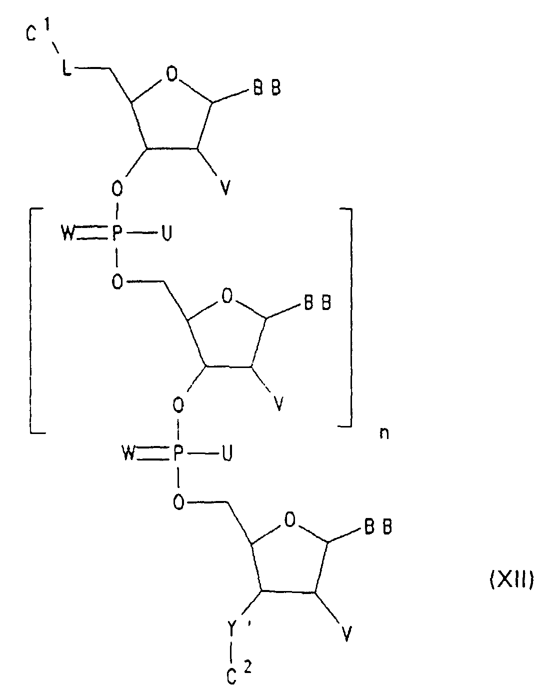 worin n, L, BB, W, V, Y', U, C¹ und C² wie oben definiert sind;
m) die Verbindung der Formel XII im sauren Bereich mit einer 1-30 % Lösung von p-Toluolsulfonsäure in einem geeigneten organischen Lösungsmittel, während 0,5 - 10h inkubiert, anschließend neutralisiert und nach dem Abdampfen des Lösungsmittels und nach Reinigung die Verbindung der Formel I erhält.

2. Verbindung der Formel VI 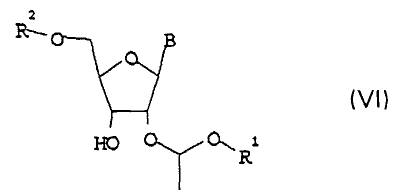 worin B, R¹ und R² wie in Anspruch 1 definiert sind.

3. Verbindung der Formel VI gemäß Anspruch 2, dadurch gekennzeichnet, daß
R¹ für -[CH₂]ᵣ-X steht, wobei für r =1, X für C₆-C₁₂-Aryl steht, wobei Aryl ein- bis dreifach mit Fluor, Chlor, Brom, C₁-C₄-Alkyl, C₁-C₄-Alkoxy, O-C(O)-[CH₂]ᵣ-X¹ oder O-C(O)O-[CH₂]ᵣ-X¹substituiert sein kann, mit X¹ = C₆-C₁₂-Aryl, das gegebenenfalls ein- bis dreifach durch C₁-C₄-Alkyl, C₁-C₄-Alkoxy, Fluor, Chlor oder Brom substituiert sein kann, für r = 2, X bevorzugt für 4-Nitrophenyl steht, und
R² für Dimethoxytrityl steht.

4. Verbindung der Formel VIII 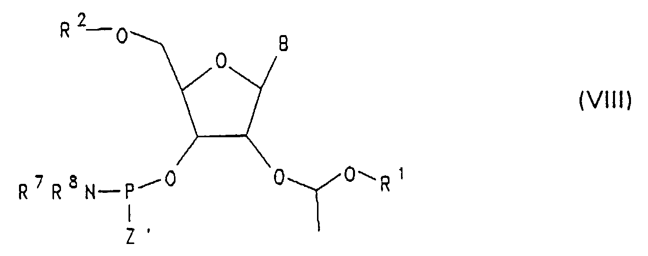 worin B, Z', R¹, R², R⁷ und R⁸ wie in Anspruch 1 definiert sind.

5. Verbindung der Formel VIII gemäß Anspruch 4, dadurch gekennzeichnet, daß
R¹ für -[CH₂]ᵣ-X steht und für r =1, X für C₆-C₁₂-Aryl steht, wobei Aryl ein- bis dreifach mit Fluor, Chlor, Brom, C₁-C₄-Alkyl, C₁-C₄-Alkoxy, O-C(O)-[CH₂]ᵣ-X¹ oder O-C(O)O-[CH₂]ᵣ-X¹substituiert sein kann, mit X¹ = C₆-C₁₂-Aryl, das gegebenenfalls ein- bis dreifach durch C₁-C₄-Alkyl, C₁-C₄-Alkoxy, Fluor, Chlor oder Brom substituiert sein kann; für r = 2, X bevorzugt für 4-Nitrophenyl steht;
R² für Dimethoxytrityl steht;
Z' für OR⁹ steht;
R⁷ und R⁸ gleich oder verschieden sind und Isopropyl, C₅-C₈-Cycloalkyl, Benzyl oder Phenyl, oder zusammen mit dem Stickstoffatom, an das sie gebunden sind, einen gesättigten oder ungesättigten heterocyclischen Ring der gegebenenfalls weiteren Heteroatomen, wie Morpholin, und Substituenten , wie OC(O)O-C₁-C₄-Alkyl-Ester, enthalten kann, bedeuten; und
Y für Wasserstoff, CH₃ oder 1-Propinyl steht.

6. Verbindung der Formeln VI und VIII gemäß den Ansprüchen 2 - 5, dadurch gekennzeichnet, daß
B steht für

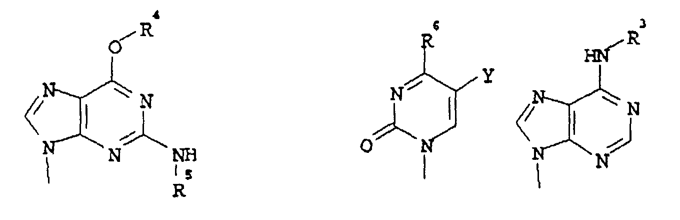

7. Verbindung der Formel XII 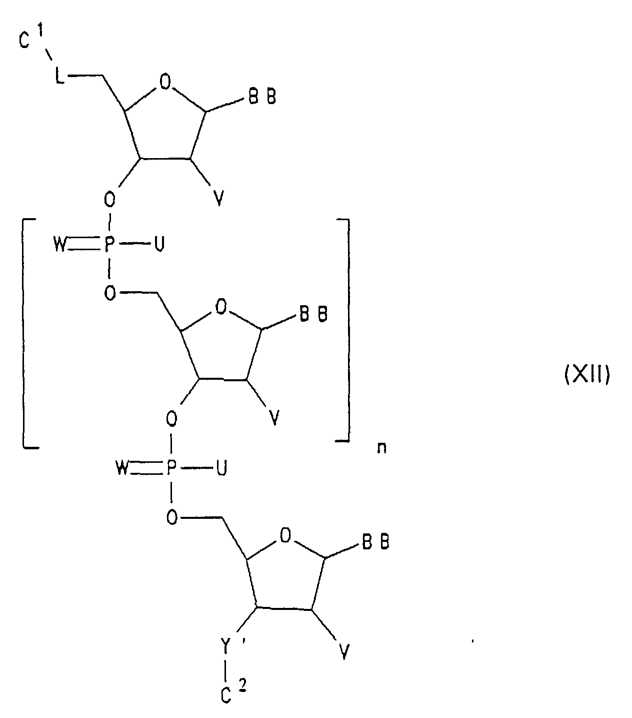 worin
V unabhängig voneinander für Wasserstoff, -O-C₁-C₁₈-Alkyl, -O-C₁-C₁₈-Alkenyl, -O-C₁-C₁₈-Alkinyl, oder -O-CH(CH₃)-OR¹, worin R¹ wie in Anspruch 1 definiert ist, steht, mindestens jedoch einmal für -O-CH(CH₃)-OR¹;
Y' für Oxy, Sulfandiyl, Imino, (CH₂)ₖ oder N(CH₂)ₖ steht, wobei k eine ganze Zahl von 1 bis 18 bedeutet;
und BB, C¹, C², L, V, W, U, Y' und n wie in Anspruch 1 definiert sind.

## Claims

1. A process for the preparation of a compound of the formula I 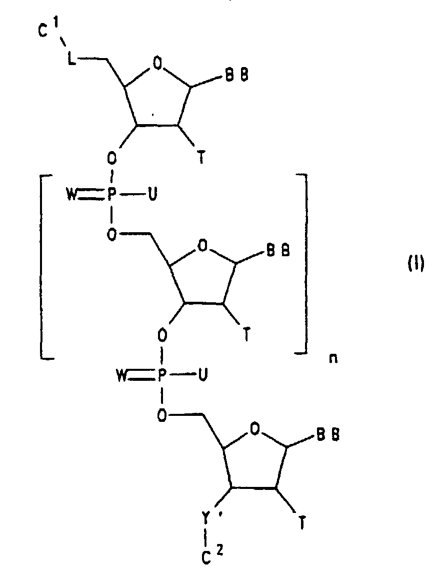 in which
n is a number from 1-150;
L is oxy, sulfanediyl or imino;
BB is, independently of one another, 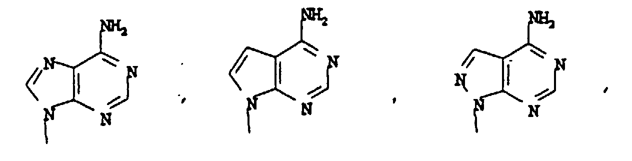 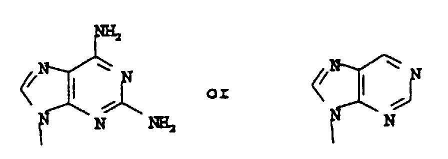 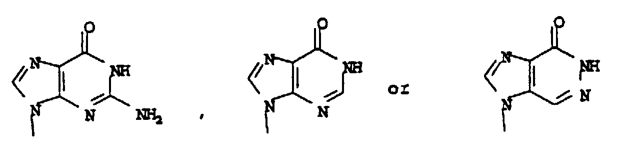 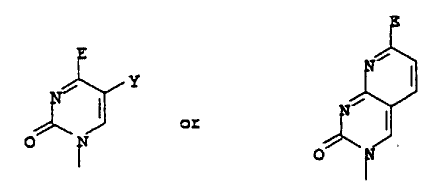 and
E is OH or NH₂ and
Y is hydrogen, C₁-C₄-alkyl, fluorine, chlorine, bromine or C₂-C₆-alkenyl or C₂-C₆-alkynyl; or
BB can, besides the natural nucleoside bases, also be other modified nucleoside bases such as 5-(hydroxymethyl)uridine, 5-aminouridine, pseudouridine, dihydrouridine, inosine, 8-aza-7-deazaadenosine, tubercidin, nebularine, xanthosine, 2-aminoadenosine, ethenoadenosine, 7-deazaguanosine, O4-methylthymidine, N6-methyladenosine, O6-methylguanosine or pyrido-pyrimidine nucleosides;
W is, independently of one another, oxygen or sulfur;
T is, independently of one another, hydrogen, -OCH₃, -O-CH₂CH₃, -O-CH₂-CH=CH₂ or OH, but is OH in at least one case;
Y' is oxy, sulfanediyl, imino, (CH₂)ₖ or N(CH₂)ₖ where k is an integer from 1 to 18;
U is hydroxyl, mercapto, SeH, C₁-C₁₈-alkoxy, C₁-C₁₈-alkyl, C₆-C₂₀-aryl, C₆-C₁₄-aryl-C₁-C₈-alkyl, NHR¹⁷, NR¹⁷R¹⁸ or a radical of the formula (OCH₂CH₂)_{c}O(CH₂)_{c'}CH₂R²⁰, where
R¹⁷ is C₁-C₁₈-alkyl, C₆-C₂₀-aryl, C₆-C₁₄-aryl-C₁-C₈-alkyl, -(CH₂)d-[NH(CH₂₋)_{d}]_{d}'-NR¹⁹R¹⁹, in which
d is an integer from 2 to 6 and
d' is an integer from 0 to 6, and
R¹⁸ is C₁-C₁₈-alkyl, C₆-C₂₀-aryl, C₆-C₁₄-aryl-C₁-C₈-alkyl or, in the case of NR¹⁷R¹⁸, together with R¹⁷ and the nitrogen atom carrying them a 5-6-membered heterocyclic ring which can additionally contain another heteroatom from the series consisting of O, S and N;
R¹⁹ is, independently of one another, hydrogen or C₁-C₆-alkyl or C₁-C₄-alkoxy-C₁-C₆-alkyl;
c is an integer from 1 to 100;
c' is an integer from 0 to 18;
R²⁰ is hydrogen or a functional group such as hydroxyl, amino, NHR¹⁷, COOH, CONH₂, COOR²¹ or fluorine, chlorine or bromine, with R²¹ being C₁-C₄-alkyl;
C¹ and C² are identical or different and are hydrogen, C₁-C₁₈-alkyl, C₂-C₁₈-alkenyl, C₂-C₁₈-alkynyl, C₁-C₁₈-alkylcarbonyl, C₂-C₁₈-alkenylcarbonyl, C₂-C₁₈-alkynylcarbonyl, C₆-C₂₀-aryl, C₆-C₁₄-aryl-C₁-C₈-alkyl, or a radical of the formula II 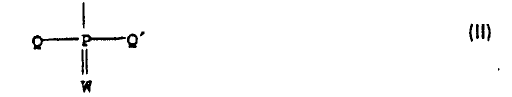 where
W is as defined above;
Q and Q' are, independently of one another, hydroxyl, mercapto, SeH, C₁-C₂₂-alkoxy, -O-(CH₂)_{b}-NR¹⁵R¹⁶ with
b being 1 to 6 and
R¹⁵ and R¹⁶ being, independently of one another, hydrogen, C₁-C₁₈-alkyl, C₆-C₂₀-aryl, C₆-C₁₄-aryl-C₁-C₈-alkyl, C₆-C₁₄-aryl-C₁-C₈-alkoxy, where aryl is also heteroaryl, and aryl is optionally substituted by 1, 2 or 3 identical or different radicals from the series consisting of carboxyl, amino, nitro, C₁-C₄-alkylamino, C₁-C₆-alkoxy, hydroxyl, fluorine, chlorine, bromine and cyano, or is C₁-C₁₈-alkylmercapto, NHR¹⁷, NR¹⁷R¹⁸ where R¹⁷ and R¹⁸ are as defined above, or together with the nitrogen atom carrying them a 3-6-membered ring or
Q and Q' are a radical of the formula III 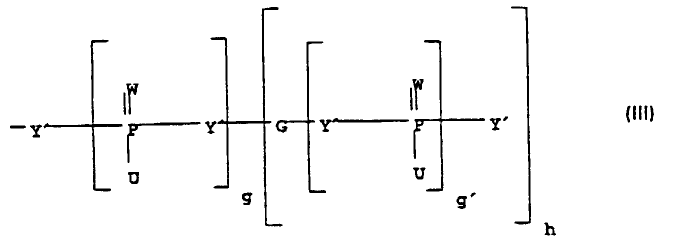 with
g and g' = 0 or 1,
h = 0 to 10,
G is C₂-C₁₂-alkylene, C₆-C₁₄-aryl-di-C₁-C₈-alkylene, C₆-C₁₈-arylene, which can optionally be substituted one to three times by fluorine, chlorine, bromine, amino, hydroxyl, C₁-C₁₈-alkyl, C₁-C₁₈-alkoxy, C₁-C₁₈-alkoxy-carbonyl, C₆-C₁₄-aryl, C₆-C₁₄-aryl-C₁-C₁₈-alkyl or C₆-C₁₄-aryl-C₁-C₈-alkoxy, or is a group of the formula (CH₂CH₂N')ᵢCH₂CH₂ or (CH₂N')ᵢCH₂ in which i is an integer from 1 to 11, and N' is oxy, sulfanediyl, imino or methylene and
W, U and Y' are as defined above; or
Q and Q' are a group which favors intracellular uptake or acts as label of a DNA probe or attacks the target nucleic acid on hybridization with the oligonucleotide analog, with crosslinking or cleavage, where the nucleic acid sequence in formula I can also be interrupted one or more times by linkers of the formula III, and conjugates can be formed by known processes also via the nucleic bases or via the phosphodiester or phosphothiodiester backbone;
which comprises
a) protecting in the 3' and 5' position a compound of the formula IV 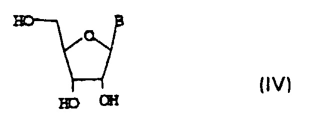 in which
B is 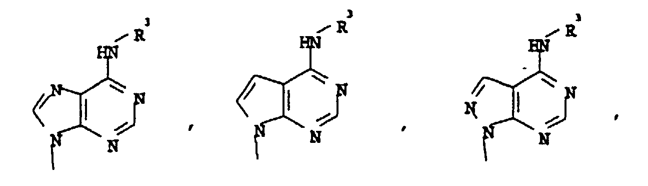 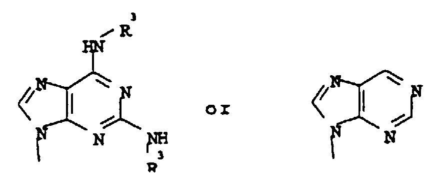 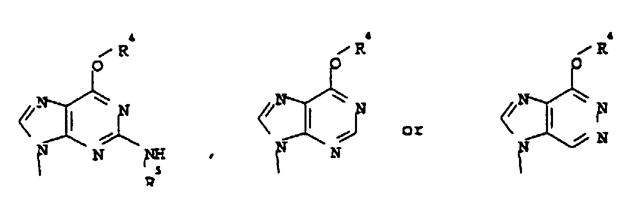 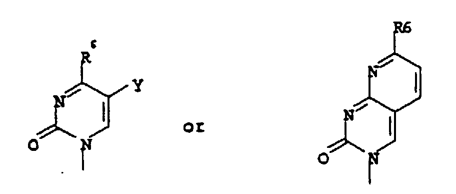 in which
R³ is, in each case, independently of one another, a group of the formula 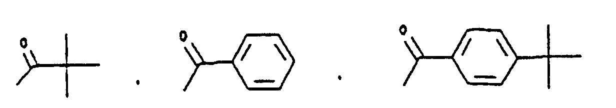 or 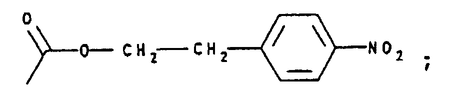
R⁴ is hydrogen or 2-(p-nitrophenyl)ethyl;
R⁵ is 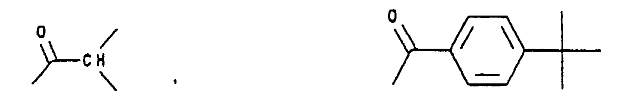 or 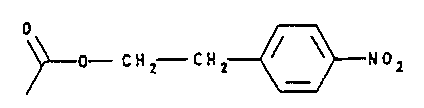
R⁶ is OH, NH₂, 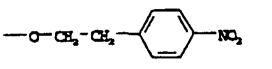 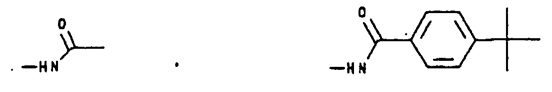 or 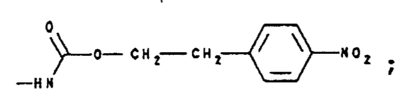
Y is as defined above; or
B can, besides the natural nucleoside bases, also be other modified nucleoside bases whose amino or hydroxyl groups are protected by suitable known protective groups such as the para-nitrophenylethyloxycarbonyl group, the benzoyl group and the para(t-butyl)benzoyl group for the hydroxyl group and the benzoyl, para-(t-butyl)benzoyl, para-nitrophenylethyloxycarbonyl, isobutyryl, para-(tert-butyl)phenylacetyl group for the amino group,
by known processes;
b) subsequently reacting the protected compound with the vinyl ether of the formula V 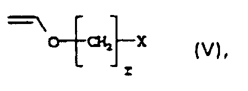 in which
r is 1 or 2, and
X is C₆-C₁₂-aryl where aryl can be substituted one or more times by hydroxyl, mercapto, nitro, fluorine, chlorine, bromine, C₁-C₆-alkyl, C₁-C₆-alkoxy, C₁-C₆-alkylmercapto, C₂-C₆-alkenyl, C₃-C₆-alkynyl, C(O)OH, C(O)NH₂, C(O)O-C₁-C₁₈-alkyl, C(O)O-C₆-C₁₂-aryl, C(O)-C₁-C₁₈-alkyl, C(O)-C₆-C₁₂-aryl, O-C(O)NH₂, O-C(O)O-C₁-C₁₈-alkyl, O-C(O)O-C₆-C₁₂-aryl, O-C(O)-C₁-C₁₈-alkyl, O-C(O)-C₆-C₁₂-aryl, O-C(O)-[CH₂]ᵣ-X¹ or O-C(O)O-[CH₂]ᵣ-X¹, with X¹ = C₆-C₁₂-aryl which can optionally be substituted one to three times by amino, hydroxyl, nitro, C₁-C₆-alkyl, C₁-C₆-alkoxy, C₁-C₆-alkoxycarbonyl, fluorine, chlorine or bromine;
for r = 1, X is also phenyl or C₁-C₄-alkoxyphenyl; for r = 2, X is also CN, S-phenyl, SO₂-phenyl, N-phthalimide or NO₂;
in analogy to known processes, and carrying out the subsequent working up likewise by known processes;
c) eliminating the 5' and 3' protected groups again, likewise in analogy to known processes;
d) introducing the 5' protective group with R² = dimethoxytrityl, monomethoxytrityl, pixyl or trityl,
by known processes, resulting in a compound of the formula VI 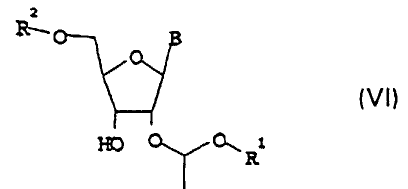 in which
R¹ is -[CH₂]ᵣ-X where r and X are as defined above;
R² is dimethoxytrityl, monomethoxytrityl, pixyl, or trityl; and
B is as defined above;
e) reacting the compound of the formula VI with a compound of the formula VII 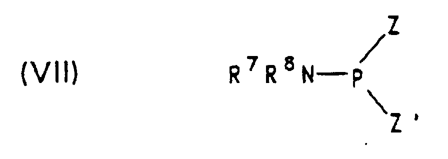 in which
Z' is OR⁹ or C₁-C₁₈-alkyl, C₁-C₁₈-alkoxy, C₆-C₂₀-aryl, C₆-C₁₄-aryl-C₁-C₈-alkyl;
R⁷ and R⁸ are identical or different and are C₁-C₈-alkyl or C₅-C₁₂-cycloalkyl, benzyl or phenyl, or together with the nitrogen atom to which they are bonded are a saturated or unsaturated heterocyclic ring optionally with further heteroatoms and substituents; and
R⁹ is a group of the formula 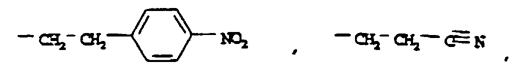 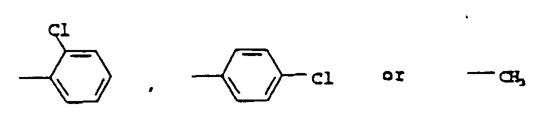 or a benzyl group, which is not substituted or is ring-substituted one to four times, where the substituent or substituents is or are, independently of one another, fluorine, chlorine, bromine, a C₁-C₄-alkyl, nitro, methoxy or carboxyl group;
Z is chlorine or bromine or a radical of the formula NR⁷R⁸ where R⁷ and R⁸ are as defined above;
in the presence of a base with a C₁-C₄-trialkyl-amine, or, if Z is a radical of the formula NR⁷R⁸, then in the presence of a compound of the formula [HNR¹²R¹³R¹⁴]⁽⁺⁾A⁽⁻⁾ where R¹², R¹³, R¹⁴ are identical or different and are a C₁-C₄-alkyl group and A = fluorine, chlorine, bromine, or tetrazole, to give a compound of the formula VIII 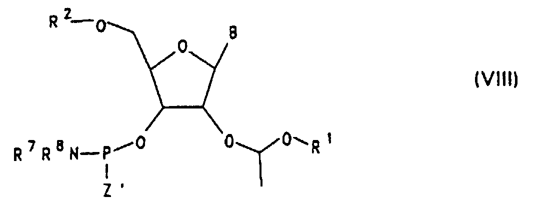 in which R¹, R², R⁷, R⁸, Z' and B are as defined above;
f) reacting the compound VI obtained according to d) by known processes with 1 to 10 equivalents of a linker such as succinic anhydride in a suitable organic solvent, where appropriate after addition of a catalyst, to give a compound of the formula IX 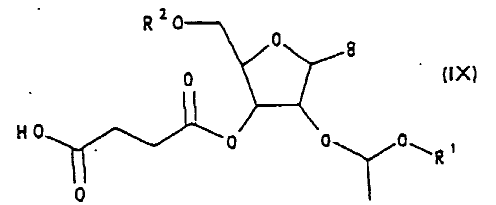 where R¹, R² and B are as defined above, and subsequently working up by known processes, where the succinic acid residue in the 3' position acts as linker to the polymeric support used in the synthesis, and it is also possible, as alternative to the succinic acid linker, to use other linkers;
g) coupling the compound of the formula X 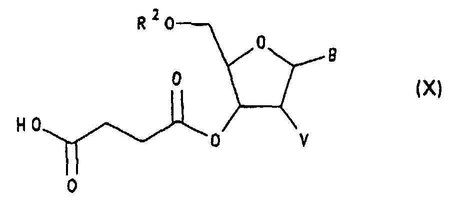 in which
R² and B are as defined above;
V is hydrogen, O-C₁-C₁₈-alkyl, O-C₁-C₁₈-alkenyl, O-C₁-C₁₈-alkynyl or -O-CH(CH₃)-OR¹ in which R¹ is as defined above,
by known processes, to the solid support;
h) eliminating the 5' protective group by known processes;
i) reacting the resulting compound with a compound of the formula XI 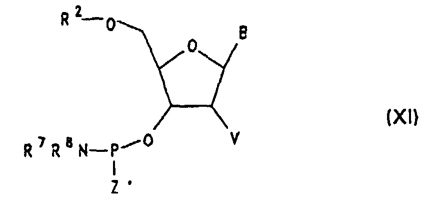 in which R², R⁷, R⁸, V, Z' and B are as defined above and
j) oxidizing the resulting compound by known processes;
k) repeating reaction steps h-j to the required chain length;
1) eliminating the oligonucleotide by known processes from the support, and eliminating the protective groups on the phosphate and nucleic bases likewise by known processes, resulting in the compound of the formula XII 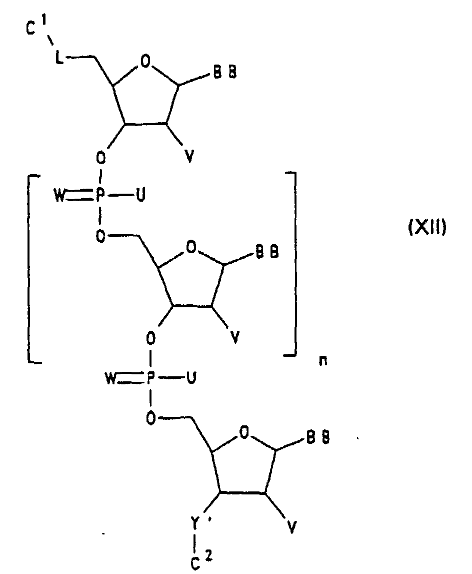 in which n, L, BB, W, V, Y', U, C¹ and C² are as defined above;
m) incubating the compound of the formula XII in the acid range with a 1-30% solution of p-toluenesulfonic acid in a suitable organic solvent for 0.5-10 h, subsequently neutralizing, and, after evaporation of the solvent and after purification, obtaining the compound of the formula I.

2. A compound of the formula VI 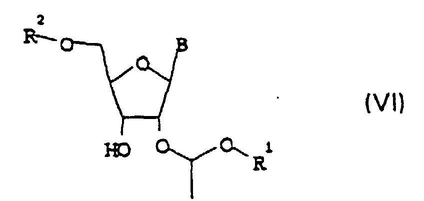 in which B, R¹ and R² are as defined in claim 1.

3. A compound of the formula VI as claimed in claim 2, wherein
R¹ is -[CH₂]ᵣ-X. where for r = 1, X is C₆-C₁₂-aryl where aryl can be substituted once to three times by fluorine, chlorine, bromine, C₁-C₄-alkyl, C₁-C₄-alkoxy, O-C(O)-[CH₂]ᵣ-X¹ or O-C(O)O-[CH₂]ᵣ-X¹, with X¹ = C₆-C₁₂-aryl which can optionally be substituted once to three times by C₁-C₄-alkyl, C₁-C₄-alkoxy, fluorine, chlorine or bromine, for r = 2, X is preferably 4-nitrophenyl, and
R² is dimethoxytrityl.

4. A compound of the formula VIII
in which B, Z', R¹, R², R⁷ and R⁸ are as defined in claim 1.

5. A compound of the formula VIII as claimed in claim 4, wherein
R¹ is -[CH₂]ᵣ-X, and for r = 1, X is C₆-C₁₂-aryl where aryl can be substituted once to three times by fluorine, chlorine, bromine, C₁-C₄-alkyl, C₁-C₄-alkoxy, O-C(O)-(CH₂]ᵣ-X¹ or O-C(O)O-[CH₂]ᵣ-X¹, with X¹ = C₆-C₁₂-aryl which can optionally be substituted once to three times by C₁-C₄-alkyl, C₁-C₄-alkoxy, fluorine, chlorine or bromine; for r = 2, X is preferably 4-nitrophenyl;
R² is dimethoxytrityl;
Z' is OR⁹ ;
R⁷ and R⁸ are identical or different and are isopropyl, C₅-C₈-cycloalkyl, benzyl or phenyl, or together with the nitrogen atom to which they are bonded are a saturated or unsaturated heterocyclic ring which can optionally contain further hetero atoms, such as morpholine, and substituents, such as OC(O)O-C₁-C₄-alkyl esters; and
Y is hydrogen, CH₃ or 1-propynyl.

6. A compound of the formulae VI and VIII as claimed in claims 2-5, wherein
B is

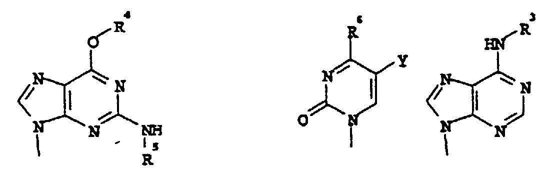

7. A compound of the formula XII 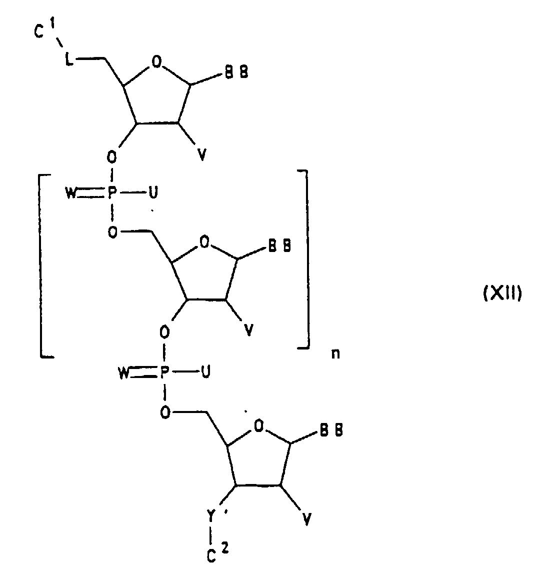 in which
V is, independently of one another, hydrogen, -O-C₁-C₁₈-alkyl, -O-C₁-C₁₈-alkenyl, -O-C₁-C₁₈-alkynyl or -O-CH(CH₃)-OR¹ in which R¹ is as defined in claim 1, but is -O-CH(CH₃)-OR¹ in at least one case;
Y' is oxy, sulfanediyl, imino, (CH₂)ₖ or N(CH₂)ₖ where k is an integer from 1 to 18;
and BB, C¹, C², L, V, W, U, Y' and n are as defined in claim 1.

## Revendications

1. Procédé pour la préparation d'un composé de formule I 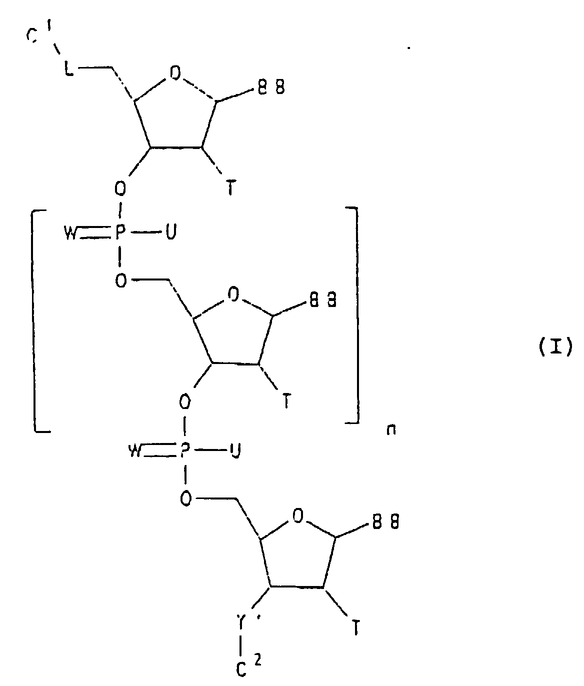 dans laquelle
n est un nombre entier allant de 1 à 150;
L représente le groupe oxy, sulfanediyle ou imino;
BB représente, indépendamment dans chaque cas, 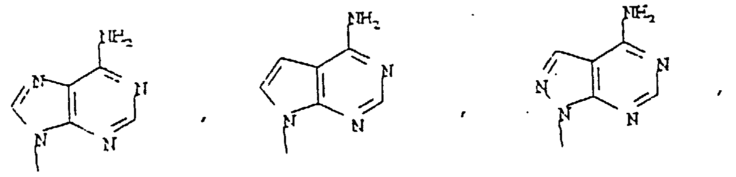 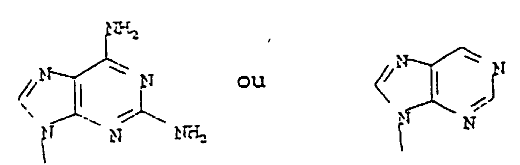 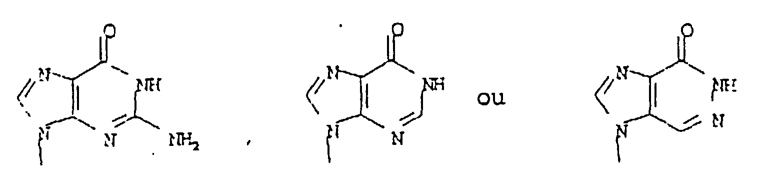 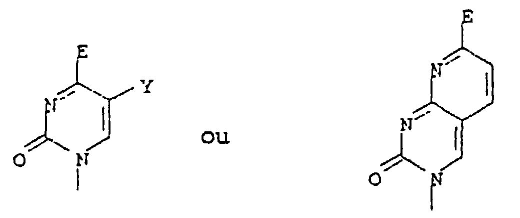 et
E représente OH ou NH₂, et
Y représente un atome d'hydrogène, de fluor, chlore, brome, ou un groupe alkyle en C₁-C₄, alcényle en C₂-C₆, alcynyle en C₂-C₆; ou
BB, outre les bases nucléosidiques naturelles, peut également représenter d'autres bases nucléosidiques modifiées, telles que la 5-(hydroxyméthyl)uridine, la 5-amino-uridine, la pseudo-uridine, la dihydro-uridine, l'inosine, la 8-aza-7-déazaadénosine, la tubercidine, la nébularine, la xanthosine, la 2-aminoadénosine, l'éthénoadénosine, la 7-déazaguanosine, l'O4-méthylthymidine, la N6-méthyladénosine, l'O6-méthylguanosine ou des nucléosides à pyridopyrimidine;
W représente, indépendamment dans chaque cas, un atome d'oxygène ou de soufre;
T représente, indépendamment dans chaque cas, un atome d'hydrogène ou le groupe -OCH₃, -O-CH₂CH₃, -O-CH₂-CH=CH₂ ou OH, mais au moins en un cas OH;
Y' représente un groupe oxy, sulfanediyle, imino, (CH₂)ₖ ou N(CH₂)ₖ, k étant un nombre entier allant de 1 à 18;
U représente un groupe hydroxy, mercapto, SeH, alcoxy en C₁-C₁₈ alkyle en C₁-C₁₈, aryle en C₆-C₂₀, aryl-(C₆-C₁₄)-alkyle(C₁-C₈), NHR¹⁷, NR¹⁷R¹⁸ ou un radical de formule (OCH₂CH₂)_{c}O(CH₂)_{c}.CH₂R²⁰;
R¹⁷ représentant un groupe alkyle en C₁-C₁₈, aryle en C₆-C₂₀, aryl(C₆-C₁₄)-alkyle(C₁-C₈), -(CH₂)_{d}-[NH(CH₂)_{d}]_{d'}-NR¹⁹R¹⁹,
d représentant un nombre entier allant de 2 à 6 et d' représentant un nombre entier allant de 0 à 6, et
R¹⁸ représentant un groupe alkyle en C₁-C₁₈, aryle en C₆-C₂₀, aryl(C₆-C₁₄)-alkyle(C₁-C₈) ou, dans le cas de NR¹⁷R¹⁸, formant, conjointement avec R¹⁷ et l'atome d'azote qui les porte, un cycle hétérocyclique à 5-6 chaînons qui peut contenir en outre un autre hétéroatome choisi parmi O, S, N;
R¹⁹ représentant, indépendamment l'un de l'autre, un atome d'hydrogène ou un groupe alkyle en C₁-C₆ ou alcoxy(C₁-C₄)-alkyle(C₁-C₆);
c étant un nombre entier allant de 1 à 100;
c' étant un nombre entier allant de 0 à 18;
R²⁰ représentant un atome d'hydrogène ou un groupe fonctionnel tel qu'un groupe hydroxy, amino, NHR¹⁷, COOH, CONH₂, COOR²¹ ou un atome de fluor, chlore ou brome, R²¹ représentant un groupe alkyle en C₁-C₄;
C¹ et C² sont identiques ou différents et représentent un atome d'hydrogène ou un groupe alkyle en C₁-C₁₈, alcényle en C₂-C₁₈, alcynyle en C₂-C₁₈, alkyl(C₁-C₁₈)carbonyle, alcényl(C₂-C₁₈)carbonyle, alcynyl(C₂-C₁₈)carbonyle, aryle en C₆-C₂₀; aryl(C₆-C₁₄)-alkyle(C₁-C₈), un radical de formule II 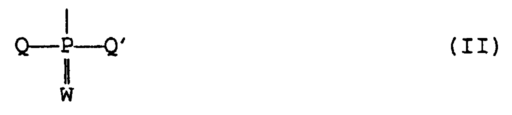 dans lequel
W est tel que défini plus haut;
Q et Q' représentent, indépendamment l'un de l'autre, un groupe hydroxy, mercapto, SeH, alcoxy en C₁-C₂₂, -O-(CH₂)_{b}-NR¹⁵R¹⁶,
b allant de 1 à 6 et
R¹⁵ et R¹⁶ représentant, indépendamment l'un de l'autre, un atome d'hydrogène ou un groupe alkyle en C₁-C₁₈, un groupe aryle en C₆-C₂₀, aryl(C₆-C₁₄-alkyle(C₁-C₈), aryl(C₆-C₁₄)-alcoxy(C₁-C₈), le groupe aryle représentant également un groupe hétéroaryle et le groupe aryle étant éventuellement substitué par un, deux ou trois radicaux, identiques ou différents, choisis dans l'ensemble constitué par les groupes carboxy, amino, nitro, alkyl(C₁-C₄) amino, alcoxy en C₁-C₆, hydroxy, cyano et les atomes de fluor, chlore et brome, un groupe alkyl(C₁-C₁₈)mercapto, NHR¹⁷, NR¹⁷R¹⁸, R¹⁷ et R¹⁸ étant tels que définis plus haut, ou formant ensemble, avec l'atome d'azote qui les porte, un cycle à 3-6 chaînons; ou
Q et Q' représentent un radical de formule III 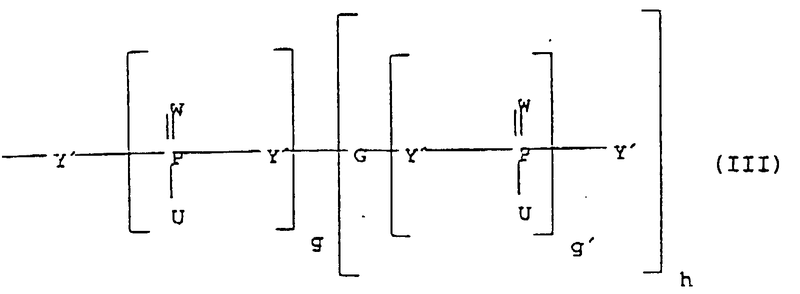 dans laquelle
g et g' = 0 ou 1,
h = 0 à 10,
G représente un groupe alkylène en C₂-C₁₂, aryl-(C₆-C₁₄)-dialkylène(C₁-C₈), arylène en C₆-C₁₈ qui peut éventuellement être une à trois fois substitué par un ou des atomes de fluor, chlore ou brome ou groupes amino, hydroxy, alkyle en C₁-C₁₈, alcoxy en C₁-C₁₈, alcoxy(C₁-C₁₈)-carbonyle, aryle en C₆-C₁₄, aryl(C₆-C₁₄)-alkyle(C₁-C₁₈) ou aryl(C₆-C₁₄)-alcoxy-(C₁-C₈), ou un groupe de formule (CH₂CH₂N')ᵢCH₂CH₂ ou (CH₂N')ᵢCH₂, où
i est un nombre entier allant de 1 à 11; et
N' représente un groupe oxy, sulfanediyle, imino ou méthylène, et
W, U et Y' sont tels que définis plus haut; ou
Q et Q' représentent un groupe qui favorise la capture intracellulaire ou sert de marquage d'une sonde d'ADN ou, dans l'hybridation de l'analogue d'oligonucléotide avec l'acide nucléique cible, attaque ce dernier avec réticulation ou coupure, dans la formule I la séquence d'acide nucléique pouvant également être une ou plusieurs fois interrompue par des segments de liaison de formule III, et des conjugués pouvant également être formés, selon des procédés connus, par l'intermédiaire des bases nucléiques ou par l'intermédiaire du squelette phosphodiester ou phosphothiodiester;
caractérisé en ce que
a) on protège en positions 3' et 5', selon des procédés connus, un composé de formule IV 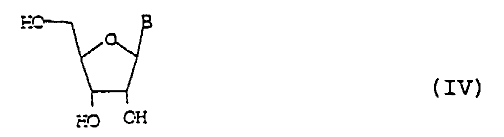 dans laquelle
B représente 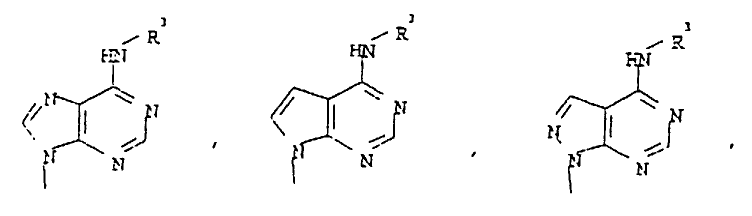 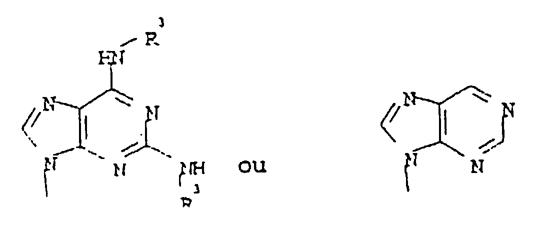 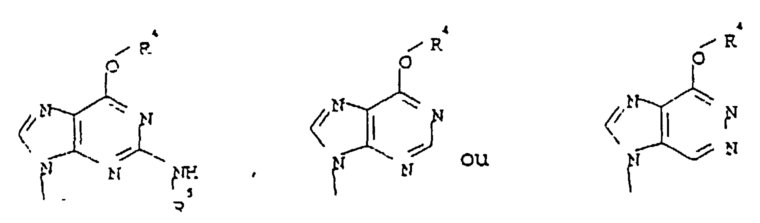 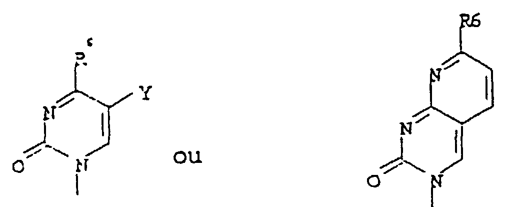 où
R³ représentent chacun, indépendamment les uns des autres, un groupe de formule 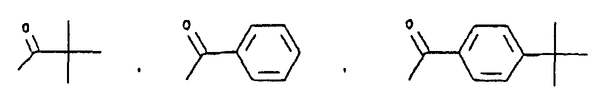 ou 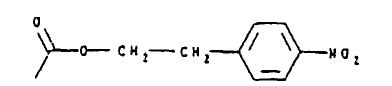
R⁴ représente un atome d'hydrogène ou le groupe 2-(p-nitrophényl)éthyle;
R⁵ représente 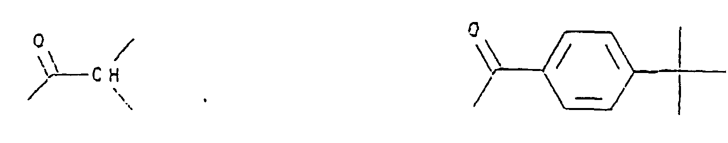 ou 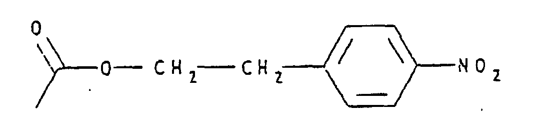
R⁶ représente OH, NH₂, 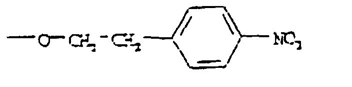 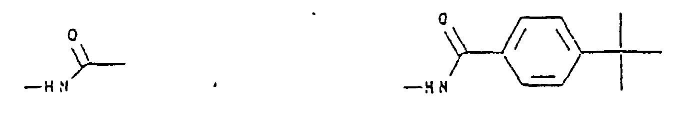 ou 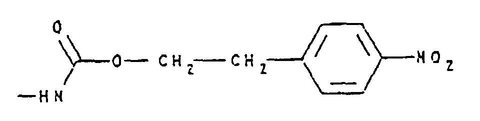
Y est tel que défini plus haut; ou
B peut, en plus des bases nucléosidiques naturelles, représenter également d'autres bases nucléosidiques modifiées, dont les groupes amino ou hydroxy sont protégés par des groupes protecteurs connus appropriés, comme le groupe p-nitrophényléthyloxycarbonyle, le groupe benzoyle et le groupe p-(tert-butyl)benzoyle pour le groupe hydroxy, et le groupe benzoyle, p-(tert-butyl)benzoyle, p-nitrophényléthyloxycarbonyle, isobutyryle, p-(tert-butyl)phénylacétyle pour le groupe amino;
b) on fait ensuite réagir le composé protégé avec l'éther vinylique de formule V 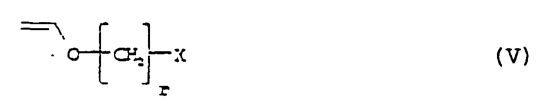 dans laquelle
r représente 1 ou 2 et
X représente un groupe aryle en C₆-C₁₂, le groupe aryle pouvant être substitué une ou plusieurs fois par un ou des atomes de fluor, chlore, brome ou groupes hydroxy, mercapto, nitro, alkyle en C₁-C₆, alcoxy en C₁-C₆, alkyl(C₁-C₆)mercapto, alcényle en C₂-C₆, alcynyle en C₃-C₆, C(O)OH, C(O)NH₂,C(O)O-alkyle(C₁-C₁₈), C(O)O-aryle(C₆-C₁₂), C(O)-alkyle(C₁-C₁₈), C(O)-aryle(C₆-C₁₂), O-C(O)NH₂, O-C(O)O-alkyle(C₁-C₁₈), O-C(O)O-aryle-(C₆-C₁₂), O-C(O)-alkyle(C₁-C₁₈), O-C(O)-aryle(C₆-C₁₂), O-C(O)-(CH₂)ᵣ-X¹ ou O-C(O)O-(CH₂)ᵣ-X¹, X¹ représentant un groupe aryle en C₆-C₁₂ qui peut éventuellement être une à trois fois substitué par un ou des groupes amino, hydroxy, nitro, alkyle en C₁-C₆, alcoxy en C₁-C₆, alcoxy(C₁-C₆)carbonyle ou atomes de fluor, chlore ou brome;
pour r = 1, X représente en outre le groupe phényle ou un groupe alcoxy(C₁-C₄)phényle;
pour r = 2, X représente en outre le groupe CN, S-phényle, SO₂-phényle, N-phtalimido ou NO₂;
par analogie avec des procédés connus, et on effectue le traitement final subséquent, également selon des procédés connus;
c) on élimine de nouveau les groupes protecteurs en 5' et 3', également par analogie avec des procédés connus; d) on introduire selon des procédés connus le groupe protecteur en 5' dans lequel R² représente le groupe diméthoxytrityle, monométhoxytrityle, pixyle ou trityle, pour obtenir un composé de formule VI 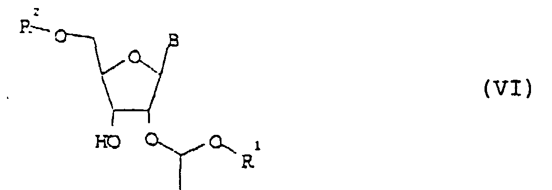 dans laquelle
R¹ représente un groupe -(CH₂)ᵣ-X, dans lequel r et X sont tels que définis plus haut,
R² représente le groupe diméthoxytrityle, monométhoxytrityle, pixyle ou trityle;
B est tel que défini plus haut;
e) on fait réagir le composé de formule VI avec un composé de formule VII 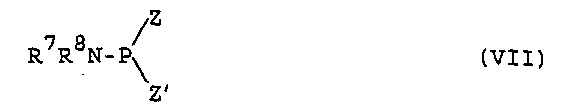 dans laquelle
Z' représente un groupe OR⁹ ou alkyle en C₁-C₁₈, alcoxy en C₁-C₁₈, aryle en C₆-C₂₀, aryl(C₆-C₁₄)-alkyle(C₁-C₈);
R⁷ et R⁸ sont identiques ou différents et représentent un groupe alkyle en C₁-C₈ ou un groupe cycloalkyle en C₅-C₁₂, benzyle ou phényle, ou forment ensemble, avec l'atome d'azote auquel ils sont fixés, un cycle hétérocyclique saturé ou insaturé, comportant éventuellement d'autres hétéroatomes et des substituants; et
R⁹ représente un groupe de formule 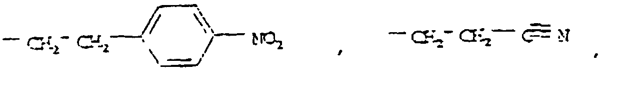 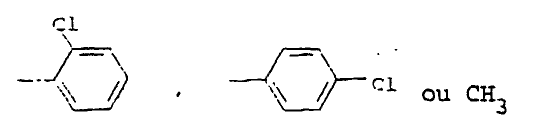 ou un groupe benzyle qui n'est pas substitué sur le noyau ou est substitué une à quatre fois sur le noyau, le ou les substituants étant, indépendamment les uns des autres, un atome de fluor, chlore ou brome ou un groupe alkyle en C₁-C₄, nitro, méthoxy ou carboxy;
Z représente un atome de chlore ou de brome ou un radical de formule NR⁷R⁸, R⁷ et R⁸ étant tels que définis plus haut;
en présence d'une base avec une trialkyl(C₁-C₄)amine, ou, lorsque Z est un radical de formule NR⁷R⁸, alors en présence d'un composé de formule (HNR¹²R¹³R¹⁴)⁽⁺⁾A⁽⁻⁾, R¹², R¹³, R¹⁴ étant identiques ou différents les uns des autres et représentant un groupe alkyle en C₁-C₄, et A représentant un atome de fluor, chlore ou brome, ou en présence de tétrazole, pour l'obtention d'un composé de formule VIII 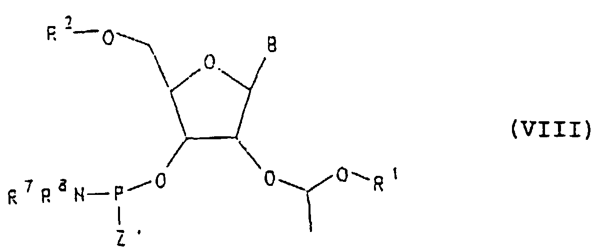 dans laquelle R¹, R², R⁷, R⁸, Z' et B sont tels que définis plus haut;
f) on fait réagir le composé VI obtenu selon d), conformément à des procédés connus, avec 1 à 10 équivalents d'un composé de liaison, tel que l'anhydride succinique, dans un solvant organique approprié, éventuellement après addition d'un catalyseur, pour aboutir à un composé de formule IX 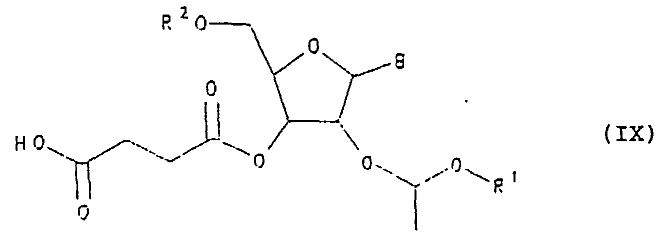 dans laquelle R¹, R² et B sont tels que définis plus haut, et on achève ensuite de le traiter selon des procédés connus, le reste d'acide succinique en position 3' servant de groupe de liaison au support polymère utilisé dans la synthèse, et en pouvant également utiliser, au lieu du groupe de liaison acide succinique, d'autres groupes de liaison;
g) on attache au support solide le composé de formule X 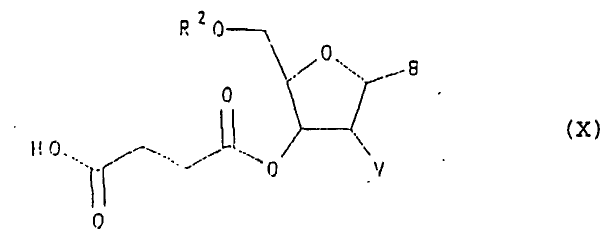 dans laquelle
R² et B sont tels que définis plus haut;
V représente un atome d'hydrogène ou un groupe O-alkyle-(C₁-C₁₈), O-alcényle(C₁-C₁₈), O-alcynyle(C₁-C₁₈) ou -O-CH(CH₃)OR¹, R¹ étant tel que défini plus haut;
h) on élimine le groupe protecteur en 5' selon des procédés connus;
i) on fait réagir le composé obtenu avec un composé de formule XI 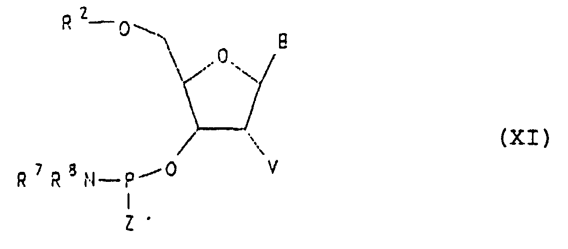 dans laquelle R², R⁷, R⁸, V, Z' et B sont tels que définis plus haut; et
j) on oxyde le composé obtenu selon des procédés connus;
k) on répète les étapes de réaction h-j jusqu'à la longueur de chaîne désirée;
1) on sépare l'oligonucléotide du support selon des procédés connus et on élimine les groupes protecteurs sur le phosphate et les bases nucléiques, également selon des procédés connus, pour obtenir le composé de formule XII 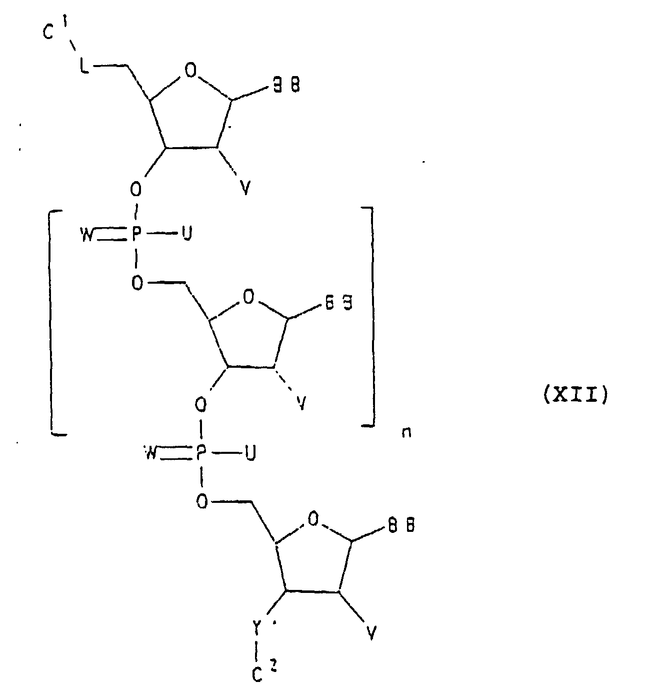 dans laquelle n, L, BB, W, V, Y', U, C¹ et C² sont tels que définis plus haut;
m) on met à incuber le composé de formule XII pendant 0,5-10 heures, dans le domaine acide, avec une solution d'acide p-toluènesulfonique à 1-30 %, dans un solvant organique approprié, ensuite on le neutralise, et, après élimination du solvant par évaporation et après purification, on obtient le composé de formule I.

2. Composé de formule VI 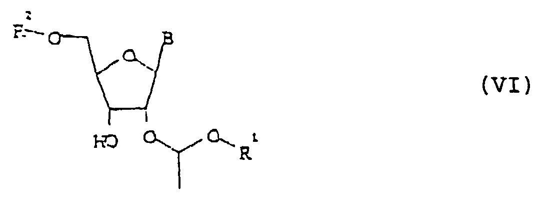 dans lequel B, R¹ et R² sont tels que définis dans la revendication 1.

3. Composé de formule VI selon la revendication 2, caractérisé en ce que
R¹ représente un groupe -(CH₂)ᵣ-X, dans lequel
pour r = 1, X représente un groupe aryle en C₆-C₁₂, le groupe aryle pouvant être substitué une ou trois fois par un ou des atomes de fluor, chlore ou brome ou groupes alkyle en C₁-C₄, alcoxy en C₁-C₄, O-C(O)-(CH₂)ᵣ-X¹ ou O-C(O)O-(CH₂)ᵣ-X¹ , où X¹ représente un groupe aryle en C₆-C₁₂ qui peut éventuellement être une à trois fois substitué par un ou des atomes de fluor, chlore ou brome ou groupes alkyle en C₁-C₄ ou alcoxy en C₁-C₄;
pour r = 2, X représente de préférence le groupe 4-nitrophényle; et
R² représente le groupe diméthoxytrityle.

4. Composé de formule VIII 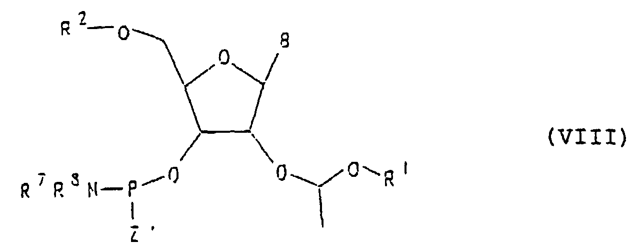 dans lequel B, Z', R¹, R², R⁷ et R⁸ sont tels que définis dans la revendication 1.

5. Composé de formule VIII selon la revendication 4, caractérisé en ce que
R¹ représente un groupe -(CH₂)ᵣ-X et
pour r = 1, X représente un groupe aryle en C₆-C₁₂, le groupe aryle pouvant être substitué une à trois fois par un ou des atomes de fluor, chlore, brome ou groupes alkyle en C₁-C₄, alcoxy en C₁-C₄, O-C(O)-(CH₂)ᵣ-X¹ ou O-C(O)O-(CH₂)ᵣ-X¹, X¹ représentant un groupe aryle en C₆-C₁₂ qui peut éventuellement être une à trois fois substitué par un ou des atomes de fluor, chlore ou brome ou groupes alkyle en C₁-C₄, ou alcoxy en C₁-C₄;
pour r = 2, X représente de préférence le groupe 4-nitrophényle;
R² représente le groupe diméthoxytrityle;
Z' représente un groupe OR⁹ ;
R⁷ et R⁸ sont identiques ou différents et représentent un groupe isopropyle, cycloalkyle en C₅-C₈, benzyle ou phényle, ou forment ensemble, avec l'atome d'azote auquel ils sont fixés, un cycle hétérocyclique saturé ou insaturé qui peut éventuellement contenir d'autres hétéroatomes, comme le cycle morpholine, et des substituants, tels qu'un ester OC(O)O-alkyle(C₁-C₄), et
Y représente un atome d'hydrogène ou le groupe CH₃ ou 1-propynyle.

6. Composé de formules VI et VIII selon les revendications 2 à 5, caractérisé en ce que
B représente

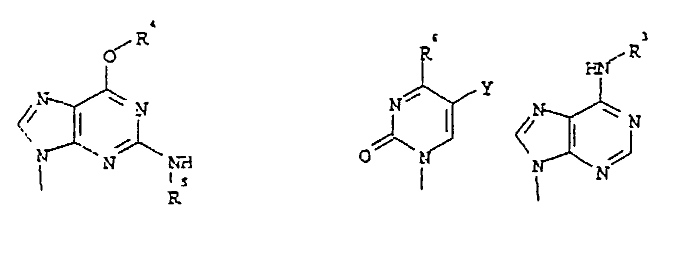

7. Composé de formule XII 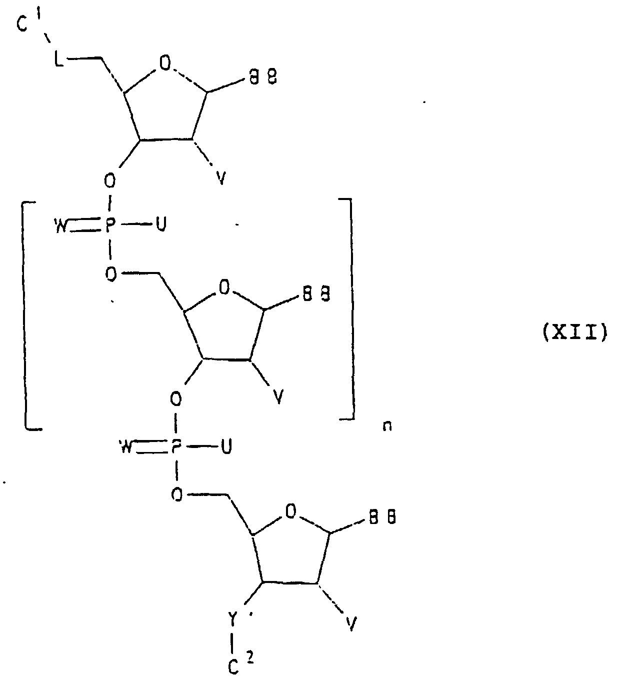 dans laquelle
V représente, indépendamment dans chaque cas, un atome d'hydrogène ou un groupe O-alkyle-(C₁-C₁₈), O-alcényle-(C₁-C₁₈), O-alcynyle(C₁-C₁₈) ou -O-CH(CH₃)OR¹, R¹ étant tel que défini dans la revendication 1, mais au moins en un cas -O-CH(CH₃)-OR¹;
Y' représente un groupe oxy, sulfanediyle, imino, (CH₂)k ou N(CH₂)ₖ, k étant un nombre entier allant de 1 à 18;
et
BB, C¹, C², L, V, W, U, Y' et n sont tels que définis dans la revendication 1.
